# FASCICULE DE BREVET EUROPEEN

(11) **EP 0 946 546 B1**
(45) Date de publication et mention de la délivrance du brevet: **25.06.2003**
(21) Numéro de dépôt: 97947107.5
(22) Date de dépôt: 20.11.1997
(51) Int. Cl.: C07D 401/12, C07D 401/14, C07D 417/14, C07D 409/14, C07D 405/14, C07D 413/14, A61K 31/445, C07D 211/38

(54) **DERIVES DE LA PYRIDIN-2-YL-METHYLAMINE, LEUR PROCEDE DE PREPARATION ET LEUR APPLICATION COMME MEDICAMENTS**
PIRIDIN-2-YL-METHYLAMIN-DERIVATE, VERFAHREN ZU DEREN HERSTELLUNG UND IHRE VERWENDUNG ALS ARZNEIMITTEL
PYRIDIN-2-YL-METHYLAMINE DERIVATIVES, METHOD OF PREPARING AND APPLICATION AS MEDICINE

(30) Priorité: 21.11.1996 FR 9614217
(43) Date de publication de la demande: 06.10.1999
(73) Titulaire: PIERRE FABRE MEDICAMENT, 92100 Boulogne-Billancourt (FR)
(72) Inventeur: VACHER, Bernard, F-81100 Castres (FR); BONNAUD, Bernard, F-81090 Lagarrigue (FR); KOEK, Wouter, F-81290 Viviers-les-Montagnes (FR)
(74) Mandataire: Ahner, Francis
(86) Numéro de dépôt international: FR9702097
(87) Numéro de publication internationale: WO98022459

(56) Documents cités:
- EP-A- 0 538 080
- EP-A- 0 661 266

## Description

La sérotonine (5-hydroxytryptamine, 5-HT) est un neurotransmetteur et un neuromodulateur du système nerveux central qui exerce ses fonctions physiologiques multiples par l'interaction avec des récepteurs 5-HT spécifiques. Ces récepteurs 5-HT ont été regroupés en plusieurs classes principales. Parmi ces classes principales, la classe 5-HT₁ comprend des récepteurs caractérisés par une affinité élevée pour la sérotonine. La classe 5-HT₁ est elle-même divisée en sous-classe de récepteurs dont les caractéristiques pharmacologiques et les distributions régionales dans le système nerveux central sont distinctes.

Des études cliniques de composés ayant une activité agoniste pour le sous-type 5-HT_{1A} ont démontré que les agonistes 5-HT_{1A} étaient efficaces dans le traitement de l'anxiété (J. Clin. Psychiatry 1987, 48 (12 suppl.), 3-6; Int. Clin. Psychopharmacol. 1993, 8, 173-6), de la dépression (J. Clin. Psychopharmacol. 1990, 10 (3 Suppl.), 67-76; J. Clin. Psychiatry 1991, 52, 217-20), des troubles obsessionnnels-compulsifs (Am. J. Psychiatry 1991, 148, 127-9), des crises de panique (J. Clin. Psychopharmacol. 1993, 13, 145-9), des troubles du sommeil (Psychopharmacol, 1995, 117, 186-92) et de l'abus d'alcool (J. Clin. Psychopharmacol. 1989, 9, 379-80).

Des études chez l'animal ont démontré que les agonistes 5-HT_{1A} possèdent des propriétés analgésiques (Eur. J. Pharmacol. 1996, 295, 181-8), anti-agressive (Neurosci. Biobehav. Rev. 1994, 18, 325-38) et antiémétiques (Pharmacol. Biochem. Behav. 1989, 33, 627-31; Pharmacol. Biochem. Behav. 1995, 52, 571-5). Les composés ayant une activité agoniste 5-HT_{1A} ont également été rapportés comme pouvant être utiles dans le traitement des troubles du comportement sexuel (Behavioural. pharmacol. 1995, 6, 276-82), pour la régularisation de la prise alimentaire (Int. Clin. Psychopharmacol. 1994, 9, 7-17) et pour la régulation de la sécrétion gastrique (J. Pharmacol. Exp. Ther. 1995, 272, 832-7). L'action antihypertensive des agonistes 5-HT_{1A}, via un mécanisme central, est reconnue (Trends Pharm. Sci. 1990, 11, 95-6; Fundam Clin. Pharmacol. 1993, 7, 499-511) de plus, les agonistes 5-HT_{1A} ont montrés des propriétés neuroprotectrices dans des modèles d'ischémie locale et globale chez les rongeurs (Brain Research 1993, 630, 10-20, Arch. Int. Pharmacodyn. Ther. 1995, 329, 347-59).

Des études in vitro tendent aussi à impliquer les récepteurs 5-HT_{1A} dans la stimulation de la prolifération des lymphocytes (INPHARMA®, 26 Aug. 1995, 10; Life Sciences, 57, 2197-203).

Compte tenu du potentiel thérapeutique important des composés dotés d'une activité agoniste pour les récepteurs du sous-type 5-HT_{1A}, la découverte de structures nouvelles possédant des propriétés agoniste 5-HT_{1A} est fortement souhaitable. La demanderesse a découvert que plusieurs composés dérivés de pyridin-2-yl-méthylamine ont une activité agoniste vis à vis du récepteur central 5-HT_{1A}.

La présente invention concerne donc des composés nouveaux répondant à la formule générale (I) 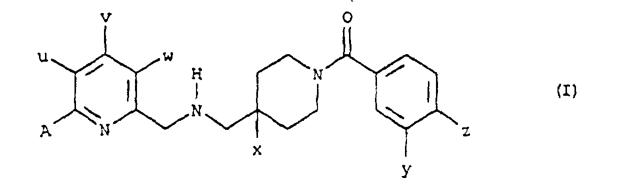 dans laquelle:
- **u**: représente un atome d'hydrogène ou un radical méthyl avec la réserve que lorsque u est un radical méthyl alors **v** et **w** représentent un atome d'hydrogène;
- **v**: représente un atome d'hydrogène ou un atome de chlore ou un radical méthyl avec la réserve que lorsque **v** représente un atome de chlore ou un radical méthyl alors **u** et **w** représentent un atome d'hydrogène;
- **w**: représente un atome d'hydrogène ou un atome de fluor ou un radical méthyl avec la réserve que lorsque **w** représente un atome de fluor ou un radical méthyl alors **u** et **v** représentent un atome d'hydrogène;
- **x**: représente un atome d'hydrogène ou un atome de fluor;
- **y**: représente un atome de chlore ou un radical méthyl;
- **z**: représente un atome d'hydrogène ou un atome de fluor ou un atome de chlore ou un radical méthyl;
- **A**: représente:
- un atome d'hydrogène ou un atome de fluor ou un atome de chlore;
- un radical alkyl en C₁-C₅, i.e. un reste d'hydrocarbure aliphatique saturé à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthylpropyl, 2-méthyl-butyl ou 3-méthyl-butyl, 1-éthyl-propyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (-CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons choisi parmi
   furan-2-yl, (O.CH:CH.CH:C-) ou
   furan-3-yl, (CH:CH.O.CH:C-) ou
   1H-pyrrol-2-yl, (NH.CH:CH.CH:C-) ou
   1H-pyrrol-3-yl, (CH:CH.NH.CH:C-) ou
   1-méthyl-pyrrol-2-yl, (N(CH₃).CH:CH.CH:C-) ou
   1-méthyl-pyrrol-3-yl, (CH:CH.N(CH₃).CH:C-) ou
   thiophen-2-yl, (S.CH:CH.CH:C-) ou
   thiophen-3-yl, (CH:CH.S.CH:C-) ou
   pyrazol-1-yl, (N:CH.CH:CH.N-) ou
   1H-pyrazol-3-yl, (CH:CH.NH.N:C-) ou
   1H-pyrazol-4-yl, (CH:N.NH.CH:C-) ou
   1-méthyl-pyrazol-3-yl, (CH:CH.N(CH₃).N:C-) ou
   imidazol-1-yl (CH:N.CH:CH.N-) ou
   1H-imidazol-2-yl, (NH.CH:CH.N:C-) ou
   1H-imidazol-4-yl, (N:CH.NH.CH:C-) ou
   oxazol-2-yl, (O.CH:CH.N:C-) ou
   oxazol-4-yl, (N:CH.O.CH:C-) ou
   oxazol-5-yl, (O.CH:N.CH:C-) ou
   isoxazol-5-yl (O.N:CH.CH:C-) ou
   isoxazol-4-yl, (CH:N.O.CH:C-) ou
   isoxazol-3-yl, (CH:CH.O.N:C-) ou
   thiazol-2-yl, (S.CH:CH.N:C-) ou
   thiazol-4-yl, (N:CH.S.CH:C-) ou
   thiazol-5-yl, (S.CH:N.CH:C-) ou
   isothiazol-5-yl, (S.N:CH.CH:C-) ou
   isothiazol-4-yl, (CH:N.S.CH.C-) ou
   isothiazol-3-yl, (CH:CH.S.N:C-) ou
   [1,2,4]triazol-1-yl, (CH:N.CH:N.N-) ou
   1H-[1,2,4]triazol-3-yl, (N:CH.NH.N:C-) ou
   [1,2,4]oxadiazol-3-yl, (N:CH.O.N:C-) ou
   [1,2,4]oxadiazol-5-yl, (O.N:CH.N:C-) ou
   5-méthyl-[1,2,4]oxadiazol-3-yl, (N:C(CH₃).O.N:C-) ou
   1H-tétrazol-5-yl, (NH.N:N.N:C-);
- un groupe alkoxy (R₁O-) ou alkylthio (R₁S-) dans lequel le radical R représente:
   · un radical alkyl en C₁-C₅ tel que défini précédemment,
   · un radical monofluorométhyl ou trifluorométhyl,
   · un radical cyclopropyl ou cyclobutyl ou cyclopentyl;
- un groupe amino de type II 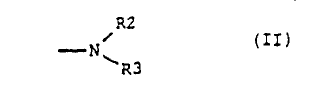 dans lequel R₂ et R₃, identiques ou différents représentent l'hydrogène, ou un radical alkyl en C₁-C₅ tel que défini précédemment ou un radical cyclopropyl ou cyclobutyl ou un radical trifluorométhyl;
- un groupe amino cyclique saturé de type III 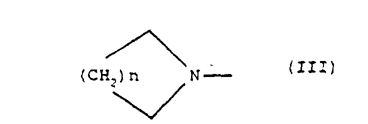 dans lequel n peut prendre les valeurs entières 1 ou 2,
- un groupe alkoxycarbonyl de préférence un groupe méthoxycarbonyl (CH₃OCO-) ou un groupe éthoxycarbonyl (CH₃CH₂OCO-);
ainsi que les sels d'addition des composés de formule générale (I) avec des acides minéraux ou les acides organiques pharmaceutiquement acceptables.

Tous les composés de cette invention ont été comparés à la Buspirone, 4-butyl-4-méthyl-1-[4-(4-pyrimid-2-yl-pipérazin-1-yl)-butyl]-pipéridine-2,6-dione, seul agoniste des récepteurs HT_{1A} actuellement disponible sur le marché et à la 8-OH-DPAT, 7-dipropylamino-5,6,7,8-tétrahydro-naphtalen-1-ol qui est l'agoniste 5-HT_{1A} standard.

Comme la Buspirone et la 8-OH-DPAT, les composés de l'invention possèdent une forte affinité pour les récepteurs 5-HT_{1A}. Cependant, les composés de l'invention montrent, en général, une sélectivité *in vitro* supérieure à celle de la Buspirone et de la 8-OH-DPAT vis à vis des récepteurs dopaminergiques du sous-type D₂. La sélectivité 5-HT_{1A} versus D₂ est définie dans la présente demande comme étant le rapport des constantes d'affinité (Ki) D₂ /(Ki) 5-HT_{1A}. Les composés de l'invention pourraient donc présenter moins d'effets secondaires indésirables que la Buspirone et la 8-OH-DPAT, en particulier les désordres neurologiques et / ou endocrines occasionnés par l'occupation des récepteurs du sous-type D₂ (CNS Drugs 1996, 5 (suppl. 1), 21-35).

L'activité agoniste 5-HT_{1A} de plusieurs composés de l'invention a pu être mise en évidence après administration orale chez le rat. L'activité agoniste 5-HT_{1A} des composés en question *in vivo* est alors en général supérieure à celle de la Buspirone et de la 8-OH-DPAT. L'activité centrale des composés de l'invention et des standards chez le rat a été évaluée par leur capacité à provoquer la rétraction de la lèvre inférieure de l'animal, un marqueur sensible et spécifique d'une activité agoniste 5-HT_{1A} centrale (Pharmacol. Biochem. Behav. 1989, 33, 821-27).

Plusieurs composés de l'invention possèdent donc une forte affinité pour les récepteurs 5-HT_{1A} *in vitro* et montrent une activité agoniste au niveau de ces récepteurs *in vivo.* De ce fait, les composés de l'invention sont considérés comme pouvant être utiles dans le traitement des multiples pathologies impliquant des dysfonctionnements sérotoninergiques telles que l'anxiété, la dépression, les troubles obsessionnels compulsifs, les crises de panique, l'agressivité, l'abus d'alcool, les troubles sexuels, les troubles du sommeil, la perception de la douleur, le vomissement, la régulation de la sécrétion gastrique, la régularisation de la prise alimentaire, les maladies immunes, les désordres vasculaires et cérébrovasculaires tel que l'hypertension arterielle ou la migraine.

L'invention concerne également les sels d'addition et éventuellement les hydrates des sels d'addition des composés de formule générale (I) avec les acides minéraux ou les acides organiques pharmaceutiquement acceptables.

L'invention a aussi pour objet les compositions pharmaceutiques contenant à titre de principe actif au moins un des dérivés de formule générale (I) ou un de ses sels ou hydrates de ses sels en combinaison avec un ou plusieurs excipients, adjuvants ou véhicules pharmaceutiquement acceptables. A titre d'exemple on peut citer les complexes d'inclusion, en particulier les complexes d'inclusion formés par les composés de l'invention avec les β-cyclodextrines.

Les compositions pharmaceutiques selon l'invention peuvent être des compositions administrables par voie orale, nasale, sublinguale, rectale ou parentérale. Il est généralement avantageux de formuler de telles compositions pharmaceutiques sous forme de dose unitaire. Chaque dose comprend alors une quantité prédéterminée du principe actif, associée au véhicule, excipients et / ou adjuvants appropriés, calculée pour obtenir un effet thérapeutique donné. A titre d'exemple de forme de dose unitaire administrable par voie orale on peut citer les comprimés, les gélules, les granules, les poudres et les solutions ou suspensions orales. Les formulations appropriées pour la forme d'administration choisie sont connues et décrites, par exemple dans le Remington, The Science and Practice of Pharmacy, 19ième edition, 1995, Mack Publishing Company et peuvent donc être facilement préparées par l'homme de l'art.

Il est connu que la posologie varie d'un individu à l'autre, selon la nature et l'intensité de l'affection, la voie d'administration choisie, le poids, l'age et le sexe du malade en conséquence les doses efficaces devront être déterminées en fonction de ces paramètres par le spécialiste en la matière. A titre indicatif, les doses efficaces pourraient s'échelonner entre 0.001 mg/Kg et 100 mg/Kg/jour.

Les composés de formule générale (I) peuvent exister sous plusieurs formes tautomères. De telles formes tautomères quoique non explicitement rapportées dans la présente demande pour simplifier la représentation graphique des schémas sont néanmoins inclues dans le champs d'application de l'invention.

L'invention s'étend enfin au procédé de préparation des dérivés de pyridin-2-yl-méthylamine de formule générale (I).

Le procédé chimique utilisé pour la préparation des composés de formule générale (I) dépend en particulier de la nature des substituants **A** et **x**.

Les composés de formule (Ia) dans lesquels :
- **x** est un atome d'hydrogène ou un atome de fluor;
- **A, u, v, w, y** et **z** ont la même signification que précédemment;
peuvent être obtenus par l'un des deux procédés (a) ou (b) décrits dans le schéma A.

### Schéma A

Selon le procédé (a), le composé de formule (Ia) est préparé par une réaction classique d'amination réductrice entre l'aldéhyde de formule (IV) et l'amine primaire de formule (V). L'expression "une réaction classique d'amination réductrice" signifie que l'aldéhyde (IV) et l'amine (V) sont mis en réaction dans le solvant approprié et que le mélange des réactifs (IV) et (V) est ensuite soumis à l'agent réducteur selon une méthode bien connue de l'homme de l'art. L'agent réducteur en question peut être un hydrure de bore simple ou complexe tel que par exemple le borohydrure de sodium, le borohydrure de potassium, le cyanoborohydrure de sodium ou le triacétoxyborohydrure de sodium.

Selon le procédé (b), la condensation entre le dérivé azido de formule (VI) et l'aldéhyde de formule (IV) en présence de triphénylphosphine ou de tri-n-butylphosphine dans le méthanol conduit à l'imine intermédiaire de formule (VII). L'imine de formule (VII) n'est pas isolée pure mais réduite in situ en l'amine de formule (la) au moyen d'un hydrure de bore simple ou complexe tel que par exemple le borohydrure de sodium ou le borohydrure de potassium. Le terme "in situ" signifie que l'imine de formule (VII) ne subit aucune procédure de purification mais que le mélange des réactifs (IV), (VI) et (Ph)₃P ou (nBu)₃P, dans le solvant approprié, est utilisé directement dans l'étape de réduction.

Les composés de formule (Ib), cas particuliers des composés de formule (la) dans lesquels :
- **x** est un atome d'hydrogène,
- **A, u, v, w, y,** et **z** sont tels que définis précédemment,
peuvent être préparés selon la méthode décrite dans le schéma B.

### Schéma B

La condensation entre la pipéridin-4-yl-méthylamine, disponible commercialement, et l'aldéhyde de formule (IV) dans un solvant tel que le benzène, le toluène, le cyclohexane ou le dichlorométhane à température ambiante ou au reflux du solvant avec élimination de l'eau formée à l'aide d'un agent desséchant ou par entraînement azéotropique conduit à l'imine de formule (VIII). L'imine de formule (VIII), non isolée pure, est ensuite acylée au moyen du chlorure d'acide approprié, disponible commercialement ou préparé selon une méthode classique à partir de l'acide carboxylique correspondant pour donner l'imine acylée de formule (IX). La réaction d'acylation en question est effectuée en présence d'une base, généralement une amine tertiaire, pour piéger l'acide chlorhydrique libéré au cours de la réaction. L'imine acylée de formule (IX), non isolée pure, est ensuite convertie en l'amine de formule (Ib) par réduction :
- soit dans un solvant protique ou un mélange de solvant dont l'un au moins des constituants est un solvant protique au moyen de borohydrure de sodium, de borohydrure de potassium ou de cyanoborohydrure de sodium;
- soit dans un solvant aprotique au moyen de triacétoxyborohydrure de sodium.

La séquence réactionnelle telle que décrite dans le schéma B peut être effectuée, si on le désire, selon une technique "one-pot". Le terme "one-pot" signifie que les étapes successives sont réalisées dans un récipient unique sans manipulation autre que l'addition séquentielle des réactifs, l'échange de solvant ou l'ajout d'un cosolvant.

Les composés de formule (Ic), cas particuliers des composés de formule (Ia) dans lesquels :
- **u et v** représentent un atome d'hydrogène,
- **w** est un atome d'hydrogène ou un radical méthyl,
- **x** est un atome d'hydrogène ou un atome de fluor,
- **y** et **z** ont la même signification que précédemment,
- **A** est choisi parmi :
   . un radical pyrrol-1-yl, pyrazol-1-yl, imidazol-1-yl ou [1,2,4]triazol-1-yl,
   . un groupe amino de type (II) ou un groupe amino cyclique de type (III),
peuvent être préparés selon le procédé résumé dans le schéma C.

### Schéma C

Les composés de formule (Ic) peuvent être obtenus directement à partir des composés de formule (Ia) dans lesquels le groupe **A** est un atome de fluor ou un atome de chlore par réaction avec un réactif approprié du type (HA) ou (Na⁺A⁻). Ce procédé, quand il est applicable, présente l'avantage d'éviter une étape de protection et une étape de déprotection lors de la préparation de l'aldéhyde de formule (IV). Le réactif symbolisé par (HA) représente une amine primaire ou secondaire, disponible commercialement, tel que la méthylamine, l'éthylamine, la n-propylamine, l'isopropylamine, l'isobutylamine, la diméthylamine, la N-éthylméthylamine, la N-méthylpropylamine, la diéthylamine, la cyclobutylamine, la cyclopropylamine, l'azétidine ou la pyrrolidine. La réaction entre le composé de formule (Ia: **A** = F ou Cl) et le réactif (HA) qui donne le composé de formule (Ic) dans lequel **A** est un groupe amino de type (II) ou un groupe amino cyclique de type (III) est généralement accomplie en présence d'un excès du réactif (HA) à une température comprise entre 25°C et 150°C.

Le réactif ionisé (Na⁺A⁻) symbolise le sel de sodium du réactif (HA) obtenu après abstraction d'un proton au moyen d'une base forte telle que par exemple l'hydrure de sodium. Les réactifs utilisés avantageusement sous la forme ionisés (Na⁺A⁻) pour la substitution de l'atome de fluor ou de chlore présent dans le composé (Ia: **A** = F ou Cl) sont les sels de sodium du pyrrole, du pyrazole, de l'imidazole ou du [1,2,4]triazole. La réaction entre les composés de formule (Ia: **A** = F ou Cl) et le réactif (Na⁺A⁻) qui conduit au composé de formule (Ic) dans lequel **A** est un radical pyrrol-1-yl, pyrazol-1-yl, imidazol-1-yl, [1,2,4]triazol-1-yl est généralement effectué à une température comprise entre 25°C et 100°C dans un solvant aprotique polaire.

Les composés de formule (Ia), (Ib) et (Ic) qui constituent l'ensemble des composés de formule (I) sont purifiés suivant une ou plusieurs méthodes choisies parmi la cristallisation, les techniques de chromatographie en phase liquide, l'extraction, la filtration. Ils peuvent être ensuite, si on le désire :
. salifiés au moyen d'un acide pharmaceutiquement acceptable;
. engagés dans la formation d'un complexe d'inclusion.

La préparation des amines primaires de formule (V) ainsi que des dérivés du type azido de formules (VI) est détaillée dans le schéma D.

### Schéma D

L'époxyde de formule (XI) est obtenu à partir de la pipéridone acylée de formule (X) selon une méthode analogue à la méthode décrite par Popp (J. Heterocyclic Chem. 1978, 15, 675-76).

L'époxyde de formule (XI) traité par un excès du complexe fluorure d'hydrogéne-pyridine conduit régiosélectivement à la fluorhydrine de formule (XII) (Synthesis 1994, 225-38). La fonction alcool primaire de la fluorhydrine de formule (XII) est ensuite activée sous forme d'un ester d'acide sulfonique pour donner le composé de formule (XIII) dans lequel «LG » symbolise un groupe méthyl-4-phénylsulfonyloxy, méthanesulfonyloxy ou trifluorométhanesulfonyloxy.

Selon la voie (c), la réaction du dérivé de formule (XIII) avec du phtalimide de potassium donne le composé de formule (XIV) qui, par traitement au moyen d'un excès d'hydrate d'hydrazine, d'éthylènediamine ou d'éthanolamine conduit à l'amine primaire de formule (V).

Selon la voie (d), la réaction du dérivé de formule (XIII) avec un azoture alcalin tel que par exemple l'azoture de sodium ou de lithium conduit au composé de formule (VI), qui peut à son tour :
- soit être engagé dans une condensation du type Staudinger-aza-Wittig tel que décrit précédemment, schéma A procédé (b) ;
- soit être converti en l'amine primaire de formule (V) par réduction de la fonction azido au moyen d'un sel métallique tel que par exemple du chlorure stanneux dans un solvant protique ou un mélange de solvants protiques, voie (e).

Le procédé de préparation des aldéhydes de formule (IV) dépend de la nature du groupe **A** et de la nature des substituants **u, v** et **w**.

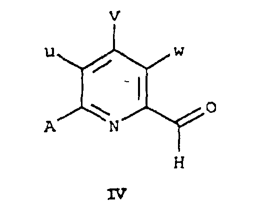

L'aldéhyde de formule (IVa) dans lequel :
. les substituants **u, v** et **w** représentent un atome d'hydrogène,
. le groupe **A** est un atome de fluor,
peut être obtenu par le procédé décrit dans le schéma E.

### Schéma E

L'aldéhyde de formule (IVa) est préparé par une coupure oxydante de l'énamine de formule (XV) au moyen de periodate de sodium selon un protocole expérimental analogue à celui décrit par Coe (Tetrahedron Lett. 1994, 35, 219-22). L'énamine de formule (XV), en général non isolée pure, peut être elle-même préparée à partir de la 2-fluoro-6-méthyl-pyridine, disponible commercialement, selon une méthode analogue à celle décrite par Bredereck (Chem. Ber. 1968, 101, 4048-56).

La méthode de préparation de l'aldéhyde de formule (IVb) dans lequel :
. les substituants **u**, **v et w** représentent un atome d'hydrogène,
. le groupe **A** est un atome de chlore,
est décrite dans Chem. Pharm. Bull. 1990, 38, 2446-58.

Les aldéhydes de formule (IVc) dans lesquels:
. les substituants **u, v** et **w** représentent un atome d'hydrogène;
. le groupe **A** représente:
   - un groupe alkoxy (R₁O-) ou un groupe alkylthio (R₁S-) dans lequel R₁ est tel que défini précédemment,
   - un groupe amino de type (II) (R₂R₃N-) dans lequel R₂ et R₃; identiques ou différents sont tels que définis précédemment;
. un groupe amino cyclique de type (III), de préférence un radical azétidin-1-yl;
peuvent être préparés selon le procédé décrit dans le schéma F.

### Schéma F

La fonction aldéhyde du composé de formule (IVa) est d'abord protégé sous la forme d'un groupe [1,3]dioxolan-2-yl dans des conditions opératoires similaires à celles utilisées pour la formation du 2-[1,3]dioxolan-2-yl-pyridine à partir du pyridine-2-carbaldéhyde (J. Heterocyclic Chem. 1987, 24, 623-28). La substitution de l'atome de fluor présent dans le composé de formule (XVI) au moyen du réactif approprié (HA) ou (Na⁺A⁻) permet ensuite d'introduire le groupe **A** désiré. Le réactif symbolisé par (HA) représente une amine primaire ou secondaire du type mono ou di(C₁-C₅alkyl)amine ou une amine cyclique telles que l'azétidine ou la pyrrolidine. La réaction entre l'acétal de formule (XVI) et le réactif (HA) qui conduit au composé de formule (XVII) dans lequel A est un groupe amino du type mono ou di(C₁-C₅alkyl)amino ou un radical azétidin-1-yl ou un radical pyrrolidin-1-yl est accomplie en présence d'un excès du réactif (HA) à une température de 100°C dans un solvant polaire.

Le réactif ionisé (Na⁺A⁻) symbolise un alcoolate ou un thiolate de sodium obtenu par déprotonation de l'alcool ou du thiol correspondant au moyen d'une base forte. La réaction entre l'acétal de formule (XVII) et le réactif (Na⁺A⁻) qui conduit au composé de formule (XVII) dans lequel A représente un groupe du type (C₁-C₅)alkoxy ou (C₁-C₅)alkylthio est effectuée à une température comprise entre 25°C et 100°C dans un solvant polaire. La fonction aldéhyde présente dans le composé de formule (IVc) est ensuite régénérée par hydrolyse acide de la fonction acétal du composé de formule (XVII) au moyen d'une solution aqueuse d'acide formique à une température comprise entre 20°C et 60°C.

Les aldéhydes de formule (IVd) dans lesquels
· les substituants **u, v** et **w** représentent un atome d'hydrogène,
· le groupe **A** est soit un radical furan-2-yl soit un radical furan-3-yl,
peuvent être obtenus selon le procédé décrit dans le schéma G.

### Schéma G

Le brevet JP 05255251 décrit la préparation du trifluorométhanesulfonic acid 6-méthyl-pyridin-2-yl ester de formule (XXIV) à partir du 6-méthyl-pyridin-2-ylamine. Dans la présente application, le composé (XXIV) a été obtenu par réaction d'un dérivé approprié de l'acide trifluorométhane sulfonique sur le 6-méthyl-pyridin-2-ol, disponible commercialement.

Le dérivé de formule (XXIV) peut être ensuite couplé avec les organozinciques dérivant du 2-lithiofurane ou du 3-lithiofurane, en présence d'un catalyseur au palladium approprié, pour donner respectivement le 2-furan-2-yl-6-méthyl-pyridine ou le 2-furan-3-yl-6-méthyl-pyridine de formule (XXV). Le 2-lithiofurane et le 3-lithiofurane ont été obtenus selon les méthodes décritent dans J. Heterocyclic Chem. 1975, 195-96. L'intermédiaire N-oxyde de formule (XXVI) est ensuite préparé par traitement du composé de formule (XXV) au moyen d'un peroxyde organique tel que par exemple l'acide 3-chloroperoxybenzoïque dans un solvant halogéné tel que par exemple le dichlorométhane ou le chloroforme.

La réaction entre le dérivé N-oxyde de formule (XXVI) et l'anhydride trifluoroacétique, selon la méthode décrite par Matsumoto (Heterocycles, 1986, 24, 2169-72), conduit intermédiairement au trifluoro-acétic-acid 6-furan-2-yl-pyridin-2-ylméthyl ester ou au trifluoro-acétic-acid 6-furan-3-yl-pyridin-2-yl-méthyl ester qui n'est pas isolé pur mais saponifié in situ au moyen d'une solution aqueuse d'une base inorganique pour donner le composé de formule (XXVII).

Le solvant de réaction du protocole expérimental original de Matsumoto (N,N-diméthylformamide) peut être avantageusement remplacé par un solvant éthéré anhydre tel que par exemple le 1,4-dioxane ou le tétrahydrofurane. L'oxydation de l'alcool de formule (XXVII) en l'aldéhyde de formule (IVd) peut être effectuée au moyen de dioxyde de manganèse ou d'un dérivé activé du diméthylsulfoxyde tel que par exemple du diméthylsulfoxyde activé par le complexe trioxyde de sulfure-pyridine ou par le chlorure d'oxalyle, selon des techniques bien connues de l'homme de l'art.

Le dérivé de formule (IVd) dans lequel le groupe **A** est soit un radical furan-2-yl soit un radical furan-3-yl peut être également obtenu selon le procédé chimique utilisé pour la préparation des aldéhydes de formule (IVe) dans lesquels
. les substituants **u** et **v** représentent un atome d'hydrogène ou un radical méthyl,
. le substituant **w** est un atome d'hydrogène ou un atome de fluor ou un radical méthyl sans que toutefois plus d'un radical méthyl ne soit présent simultanément sur les positions 3, 4 et 5 du cycle pyridinique et que lorsque **w** est un atome de fluor alors **u** et **v** sont des atomes d'hydrogène,
. le groupe **A** est choisi parmi un radical furan-2-yl ou furan-3-yl ou thiophen-2-yl ou pyrrol-2-yl ou 1-méthyl-pyrrol-2-yl.
Le procédé utilisé pour la préparation des aldéhydes de formule (IVe) est résumé dans le schéma H.

L'ensemble des alcools primaires de formule (LVII), composés de départ utilisés dans le procédé résumé dans le schéma H sont des composés connus ou préparés selon des méthodes classiques :
- le (6-chloro-pyridin-2-yl)-méthanol (LVIIa) est un composé dont la méthode de préparation est décrite dans la littérature chimique (Tetrahedron 1982, 38, 3277-80) ;
- le (5-méthyl-6-chloro-pyridin-2-yl)-méthanol (LVIIb) est obtenu par réduction du 5-méthyl-6-chloro-pyridine-2-carboxylic acid éthyl ester au moyen de borohydrure de sodium dans l'éthanol, lui même préparé selon la méthode décrite par Hoornaert (Tetrahedron 1996, 52, 2591-2602) ;
- le (3-méthyl-6-chloro-pyridin-2-yl)-méthanol (LVIIc) est obtenu par réduction du 3-méthyl-6-chloro-pyridine-2-carbaldéhyde au moyen de borohydrure de sodium dans le méthanol, lui même préparé par méthylation du 6-chloro-pyridine-2-carbaldéhyde (IVb) selon la méthode décrite par Comins (J. Org. Chem. 1990, 55, 69-73) ;
- le (4-méthyl-6-bromo-pyridin-2-yl)-méthanol (LVIId) est obtenu à partir du 2-bromo-4,6-diméthyl-pyridine par la méthode de Matsumoto modifiée. La prépraration du 2-bromo-4,6-diméthyl-pyridine est effectuée à partir de la 2-amino-4,6-diméthyl-pyridine commerciale selon un procédé analogue à celui décrit par Adams (J. Amer. Chem. Soc. 1954, 76, 3168-71).

### Schéma H

La fonction alcool primaire du composé de formule (LVII) est d'abord protégée, par exemple sous forme d'un groupe triméthylsilanyl-éthoxyméthyl, abrégé SEM, selon un protocole expérimental bien connu de l'homme de l'art. Lorsque le substituant en position 6 est un atome de brome, le composé de formule (XXVIIIb) peut être engagé directement dans l'étape suivante. Lorsque le substituant en position 6 est un atome de chlore, le composé (XXVIIIa) est préalablement convertit en l'intermédiaire (XXXI) avant d'être engagé dans l'étape suivante. Le composé de formule (XXX) intermédiaire dans la préparation du triflate de formule (XXXI) est obtenu en appliquant une méthode analogue à celle décrite par Sieburth (J. Amer. Chem. Soc. 1991, 113, 8163-64).

Le couplage des dérivés de formule (XXXI) ou (XXVIIIb) avec les esters ou les acides boroniques ou les stannanes dérivés des hetérocycles aromatiques désirés, symbolisés par (AM), en présence d'un catalyseur au palladium approprié selon les méthodes classiques de Suzuki ou de Stille, conduit au composé de formule (XXXII).

Dans les cas où les acides boronique ou les stannanes des hetérocycles aromatiques désirés (AM) ne sont pas disponibles commercialement, ils ont été préparés par transmétallation des hetérocycles lithiés correspondants, eux mêmes obtenus suivant des protocoles expérimentaux bien connus de l'homme de l'art. La coupure du groupement trimethylsilanyl-éthoxyméthyl au moyen de fluorure de tétrabutylammonium selon la méthode décrite dans Tetrahedron Lett. 1988, 29, 5417-18 conduit à l'alcool primaire de formule (XXXIII) qui est oxydé en l'aldéhyde de formule (IVe) dans des conditions expérimentales identiques à celles décrites précédemment pour l'oxydation de (XXVII) en l'aldéhyde (IVd).

Les aldéhydes de formule (IVf), (IVg) et (IVh) décris ci-après, dérivent tous d'un précurseur commun le 2-[1,3]dioxolan-2-yl-pyridine-2-carbonitrile de formule (XXXVI) dont la méthode préparation est résumée dans le schéma I.

### Schéma I

L'intermédiaire N-oxyde de formule (XXXV), obtenu par oxydation du dérivé de formule (XXXIV) au moyen d'un peroxyde organique, traité dans des conditions analogues à celles décritent par Fife (J. Org. Chem. 1983, 48, 1375-77), donne le composé de formule (XXXVI).

Les aldéhydes de formules (IVf) dans lesquels :
. les substituants **u, v** et **w** représentent un atome d'hydrogène;
. le groupe **A** représente un radical 1*H*-imidazol-2-yl ou thiazol-2-yl ou oxazol-2-yl;
sont préparés selon le procédé résumé dans le schéma J, voie f.

### Schéma J. voie f

Le dérivé de formule (XXXVII) est obtenu par addition de méthanol sur le composé de formule (XXXVI). La condensation d'un dérivé bifonctionnel tel que l'éthylènediamine, le 2-aminoéthanethiol ou le 2-aminoéthanol ou de leur chlorhydrates sur le dérivé de formule (XXXVII) conduit aux composés de formule (XXXVIII) dans lequel Q représente soit un groupe NH soit un atome de soufre soit un atome d'oxygène.

La réaction de condensation en question est effectuée par chauffage du composé de formule (XXXVII) et du réactif bifonctionnel désiré en l'absence de solvant ou au reflux d'un solvant alcoolique. L'oxydation des dérivés de formule (XXXVIII) au moyen de dioxide de manganèse, de peroxyde de nickel ou de permanganate de baryum conduit aux composés de formule (XXXIX). La réaction d'oxydation en question est effectuée, en général, au reflux d'un solvant apolaire inerte, en éliminant éventuellement l'eau formée au cours de la réaction par une des techniques classiques bien connues de l'homme de l'art. L'hydrolyse acide de l'acétal de formule (XXXIX) dans des conditions analogues à celles utilisées pour l'hydrolyse de l'acétal de formule (XVII) conduit à l'aldéhyde (IVf).

Les aldéhydes de formule (IVg) dans lesquels :
. les substituants **u, v** et **w** représentent un atome d'hydrogène,
. le groupe **A** représente un radical oxadiazol-3-yl ou méthyl-5-oxadiazol-3-yl,
sont obtenus selon le procédé résumé dans le schéma J, voie g.

### Schéma J. voie g

L'addition du chlorhydrate de l'hydroxylamine sur le composé de formule (XXXVI) donne le composé de formule (XXXX). La condensation d'un dérivé approprié de l'acide acétique tel que le chlorure d'acétyle, sur l'intermédiaire de formule (XXXX) conduit au dérivé de formule (XXXXI) dans lequel R₄ est un radical méthyl.

D'une manière analogue la condensation d'un dérivé approprié de l'acide formique tel que qu'un orthoformate d'alkyle, sur le composé de formule (XXXX) conduit au dérivé de formule (XXXXI) dans lequel R₄ est un atome d'hydrogène. L'aldéhyde de formule (IVg) est ensuite obtenu par hydrolyse de la fonction [1,3]dioxolan-2-yl du composé de formule (XXXXI) dans des conditions similaires à celles utilisées pour l'hydrolyse de l'acétal (XVII) en l'aldéhyde (IVc).

Les aldéhydes de formule (IVh) dans lesquels :
. le substituant **u** est un atome d'hydrogène ou un radical méthyl,
. les substituants **v** et **w** représentent un atome d'hydrogène,
. le groupe **A** représente un radical 1*H*-pyrazol-3-yl ou 1-méthyl-1-pyrazol-3-yl,
peuvent être obtenus selon le procédé décrit dans le schéma J, voie h.

### Schéma J, voie h

L'addition du bromure ou du chlorure de méthylmagnesium sur le composé de formule (XXXVI) donne le dérivé de formule (XXXXII). Le composé de formule (XXXXIII) est ensuite préparé par condensation du diméthylacétal du N,N-diméthylformamide ou d'un réactif équivalent tel que par exemple le tert-butoxy-bis(diméthylamino)méthane ou le tris(diméthylamino)méthane sur la cétone de formule (XXXXII). La réaction de condensation en question est effectuée dans un solvant anhydre tel que par exemple le tétrahydrofurane ou le N,N-diméthylformamide. La réaction de l'hydrate d'hydrazine sur l'intermédiaire de formule (XXXXIII) selon des méthodes bien connues de l'homme de l'art, conduit ensuite au composé de formule (XXXXIV).

Selon la voie (i), l'hydrolyse acide de l'acétal de formule (XXXXTV) dans des conditions similaires à celles utilisées précédemment conduit à l'aldéhyde de formule (IVh-1).

Selon la voie (j), le composé de formule (XXXXIV) est d'abord méthylé régiosélectivement au moyen d'iodure de méthyle en milieu basique puis la fonction acétal hydrolysée en l'aldéhyde (IVh-2) dans des conditions similaires à celles utilisées pour l'hydrolyse de l'acétal de formule (XVII) en l'aldéhyde (FVc).

L'aldéhyde de formule (IVi) dans lequel :
. les substituants **u, v** et **w** représentent un atome d'hydrogène,
. le groupe **A** représente un radical isopropyl,
est décrit dans le brevet WO 93/21158 où il a été préparé par méthylation du 6-éthyl-pyridine-2-carbaldéhyde. Dans la présente application, l'aldéhyde de formule (IVi) a été obtenu avantageusement selon le procédé résumée dans le schéma K.

### Schéma K

Le dérivé insaturé de formule (XXXXV) peut être préparé par une réaction de Wittig classique entre la 1-(6-[1,3]dioxolan-2-yl-pyridin-2-yl)-éthanone de formule (XXXXII) et l'anion dérivé du bromure de (méthyl)triphénylphosphonium, lui même obtenu par traitement du bromure de (méthyl)triphénylphosphonium au moyen de tertiobutylate de potassium. La réduction de la double liaison du 2-[1,3]dioxolan-2-yl-6-isopropényl-pyridine de formule (XXXXV) sous faible pression d'hydrogène en présence d'un catalyseur approprié tel que par exemple du palladium sur charbon, conduit au 2-[1,3]dioxolan-2-yl-6-isopropyl-pyridine de formule (XXXXVI). L'hydrolyse de l'acétal de formule (XXXXVI) dans des conditions analogues aux conditions utilisées précédemment pour l'hydrolyse de l'acétal de formule (XVII) en l'aldéhyde (IVc) donne l'aldéhyde (IVi).

L'aldéhydes de formule (IVj) dans lesquels
· les substituants **u, v** et **w** représentent un atome d'hydrogène,
· le groupe **A** représente un groupe 1-fluoro-1-éthyl,
peut être préparé le procédé décrit dans le schéma L.

### Schéma L

La réduction de la fonction cétone du composé de formule (XXXXII), effectuée au moyen de borohydrure de sodium dans le méthanol à température ambiante, conduit à l'alcool secondaire de formule (XXXXVII). L'acétal fluoré de formule (XXXXVIII) est ensuite préparé par traitement de l'alcool de formule (XXXVII) au moyen de trifluorosulfure de diéthylamine, abrégé DAST, selon une méthode classique connue de l'homme de l'art. L'aldéhyde (IVj) est obtenu par l'hydrolyse acide de la fonction acétal du composé de formule (XXXXVIII) dans des conditions identiques à celles décrites précédemment pour l'hydrolyse de l'acétal (XVII) en l'aldéhyde (IVc).

L'aldéhyde de formule (IVk) dans lequel :
· les substituants **u, v** et **w** représentent un atome d'hydrogène,
· le groupe **A** représente un radical oxazol-5-yl,
peut être obtenu selon la méthode décrite dans le schéma M.

### Schéma M

La méthode de préparation du 6-[1,3]-dioxolan-2-yl-pyridine-2-carbaldéhyde de formule (XXXXIX) est décrite dans la littérature chimique (Monatsh. Chem. 1993, 124, 881-91). La condensation du tosylméthylisocyanate, abrégé TOSMIC, sur le dérivé de formule (XXXXIX), selon un protocole expérimental analogue à celui rapporté dans Tetrahedron Lett. 1972, 2369-72, conduit à l'acétal de formule (L). L'aldéhyde (IVk) est ensuite obtenu par hydrolyse de la fonction acétal du composé de formule (L) dans des conditions opératoires similaires à celles décrites précédemment pour l'hydrolyse de l'acétal (XVII) en l'aldéhyde (IVc).

L'aldéhyde de formule (IVI) dans lequel :
· les substituants **u, v** et **w** représentent un atome d'hydrogène,
· le groupe **A** représente un radical cyclopropyl,
peut être préparé par le procédé décrit dans le schéma N.

### Schéma N

L'intermédiaire de formule (LI) est obtenu par une réaction de wittig entre l'aldéhyde de formule (XXXXIX) et le bromure de (méthyl)triphénylphosphonium en présence d'une base telle que par exemple du carbonate de potassium. Le dérivé cyclopropanique de formule (LII) est ensuite préparé par addition de l'anion dérivé de l'iodure de triméthylsulfonium sur le 2-[1,3]dioxolan-2-yl-6-éthényl-pyridine (LI) selon une méthode analogue à la méthode décrite dans J. Org. Chem. 1973, 38, 3942-44. La déprotonnation de l'iodure de triméthylsulfonium est effectuée au moyen de n-butyllithium dans le tétrahydrofurane. L'aldéhyde de formule (IVI) est ensuite obtenu à partir du dérivé de formule (LII) dans des conditions opératoires analogues aux conditions utilisées précédemment pour l'hydrolyse de l'acétal de formule (XVII) en l'aldéhyde (IVc).

Les aldéhydes de formule (IVm) dans lesquels :
. les substituants **u, v** et **w** représentent un atome d'hydrogène,
. le groupe **A** représente soit un groupe monofluorométhyl soit un groupe difluorométhyl,
sont obtenus par le procédé décrit dans le schéma O.

### Schéma O

Selon la voie (k), le (6-[1,3]-dioxolan-2-yl-pyridin-2-yl)-méthanol (LIII), précurseur de l'aldéhyde de formule (XXXXIX), traité par du trifluorosulfure de diéthylamine (DAST) dans un solvant halogéné donne l'acétal fluoré de formule (LIV). Le dérivé de formule (LIV) est ensuite converti en l'aldéhyde de formule (IVm-1) dans des conditions similaires à celles utilisées précédemmment pour l'hydrolyse de l'acétal (XVII) en l'aldéhyde (IVc).

Selon la voie (1), l'aldéhyde de formule (XXXXIX) traité par du trifluorosulfure de diéthylamine (DAST) conduit à l'acétal difluoré de formule (LV). L'acétal difluoré de formule (LV) est ensuite converti en l'aldéhyde de formule (IVm-2) dans des conditions expérimentales analogues à celles décrites précédemment, (voie k).

L'aldéhyde de formule (IVo) dans lequel :
. Les substituants **u, v** et **w** représentent un atome d'hydrogène,
. Le groupe **A** représente un groupe méthoxycarbonyl,
est obtenu par la méthode décrite dans le schéma P.

### Schéma P

Le 6-hydroxyméthyl-pyridine-2-carboxylic acid méthyl ester de formule (LVI) est préparé à partir du pyndine-2,6-carboxylic acid méthyl ester selon le protocole décrit dans Z.Naturforsh. 1994, 49b, 1127-36. L'oxydation de l'alcool de formule (LVI) dans des conditions expérimentales similaires à celles décrites pour l'oxydation de l'alcool de formule (XXVII) en l'aldéhyde (IVd), conduit ensuite à l'aldéhyde de formule (IVo).

Les méthodes de préparation de l'aldéhyde de formule (IVp) dans lequel :
. Les substituants **A, u** et w représentent un atome d'hydrogène,
. Le groupe **v** est un atome de chlore,
et de l'aldéhyde (IVr) dans lequel :
. les substituants **A, v** et **w** représentent un atome d'hydrogène,
. le groupe **u** est un radical méthyl, sont décrites dans Arch. Pharm (Weinheim Ger.) 1977, 310, 128-36.

L'aldéhyde de formule (IVq) dans lequel :
. les substituants **A, u** et **v** représentent un atome d'hydrogène,
. le groupe **w** représente un atome de fluor,
est un composé connu dont la préparation est décrite dans Tetrahedron 1983, 39, 2009-21.

Les aldéhydes de formule (IVs) dans lesquels :
. les substituants **u**, **v** et **w** représentent un atome d'hydrogène ou un radical méthyl sans toutefois que plus d'un radical méthyl ne soit présent simultanément sur les positions 3, 4 et 5 du noyau pyridinique,
. le groupe **A** est un groupe amino de type (II) (R₂ R₃ N-) dans lequel R₂ et R₃ sont tels que définis précédemment ou un groupe amino cyclique de type (III),
sont préparés par des procédés décrits dans le schéma Q.

### Schéma Q

Les alcools primaires de formule (LVII) dont les méthodes de préparation sont données dans la partie descriptive du schéma H sont mis en réaction avec une amine primaire ou secondaire du type mono ou di (C₁-C₅alkyl)amine, de préférence la méthylamine ou la diméthylamine, ou une amine cyclique de type (III), de préférence l'azétidine, pour donner le composé de formule (LVIII). Cette réaction est généralement accomplie à une température comprise entre 70°C et 120°C dans un solvant polaire. L'oxydation de la fonction alcool primaire du composé de formule (LIX) en l'aldéhyde de formule (IVs) est ensuite effectuée au moyen de dioxyde de manganèse activé au reflux d'un solvant halogéné tel que par exemple le chloroforme.

Les composés de formule (IVa), (IVb), (IVc), (IVd), (IVe), (IVf), (IVg), (IVh), (IVi), (IVj), (IVk), (IVl), (IVm), (IVn), (IVo), (IVp), (IVq), (IVr) et (IVs) constituent l'ensemble des composés de formule (IV).

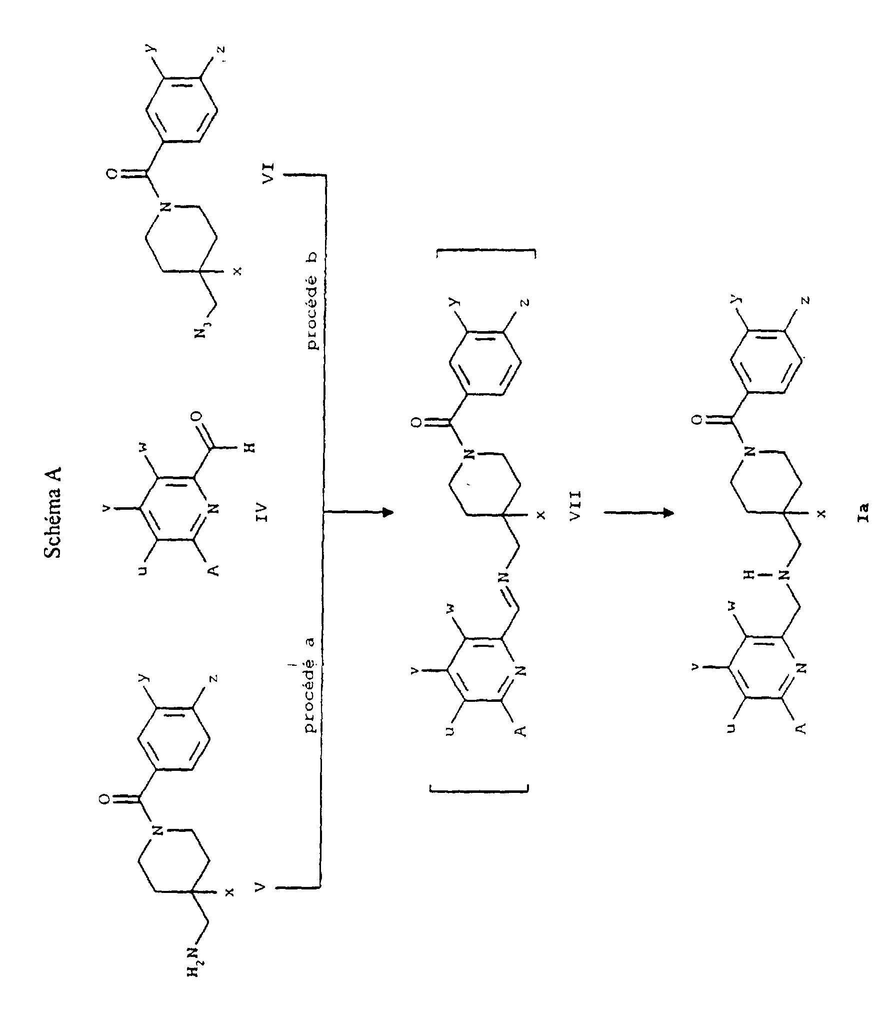

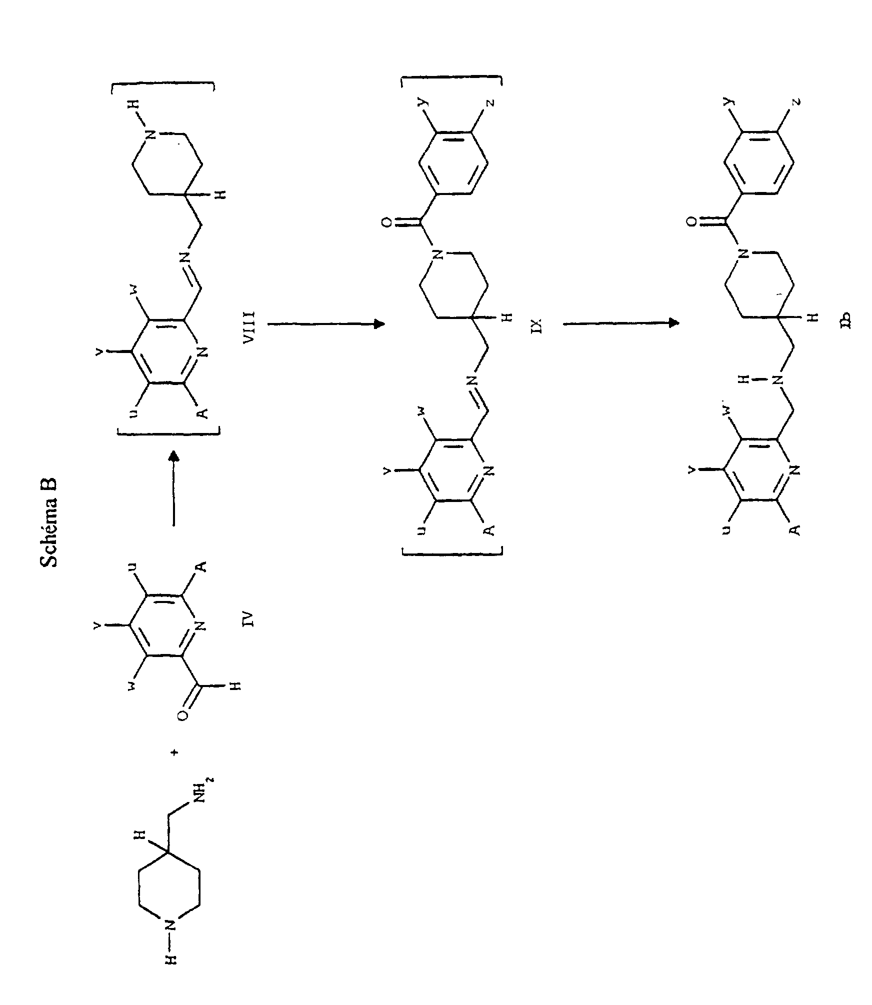

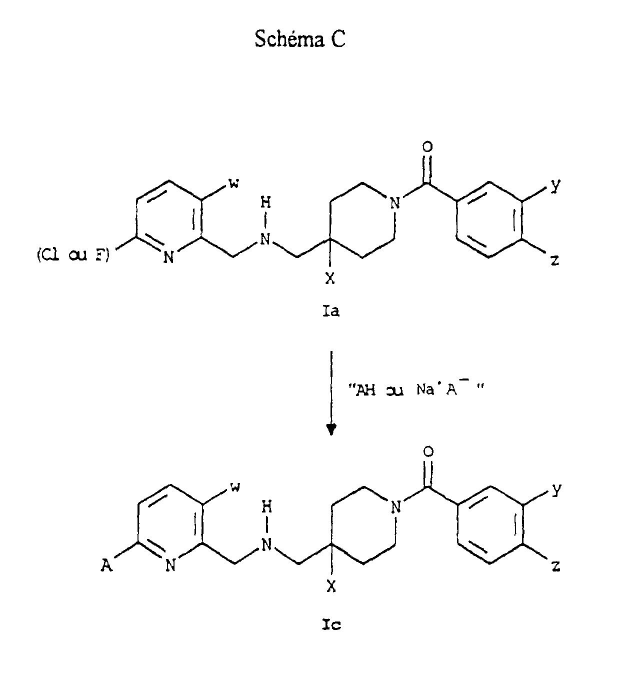

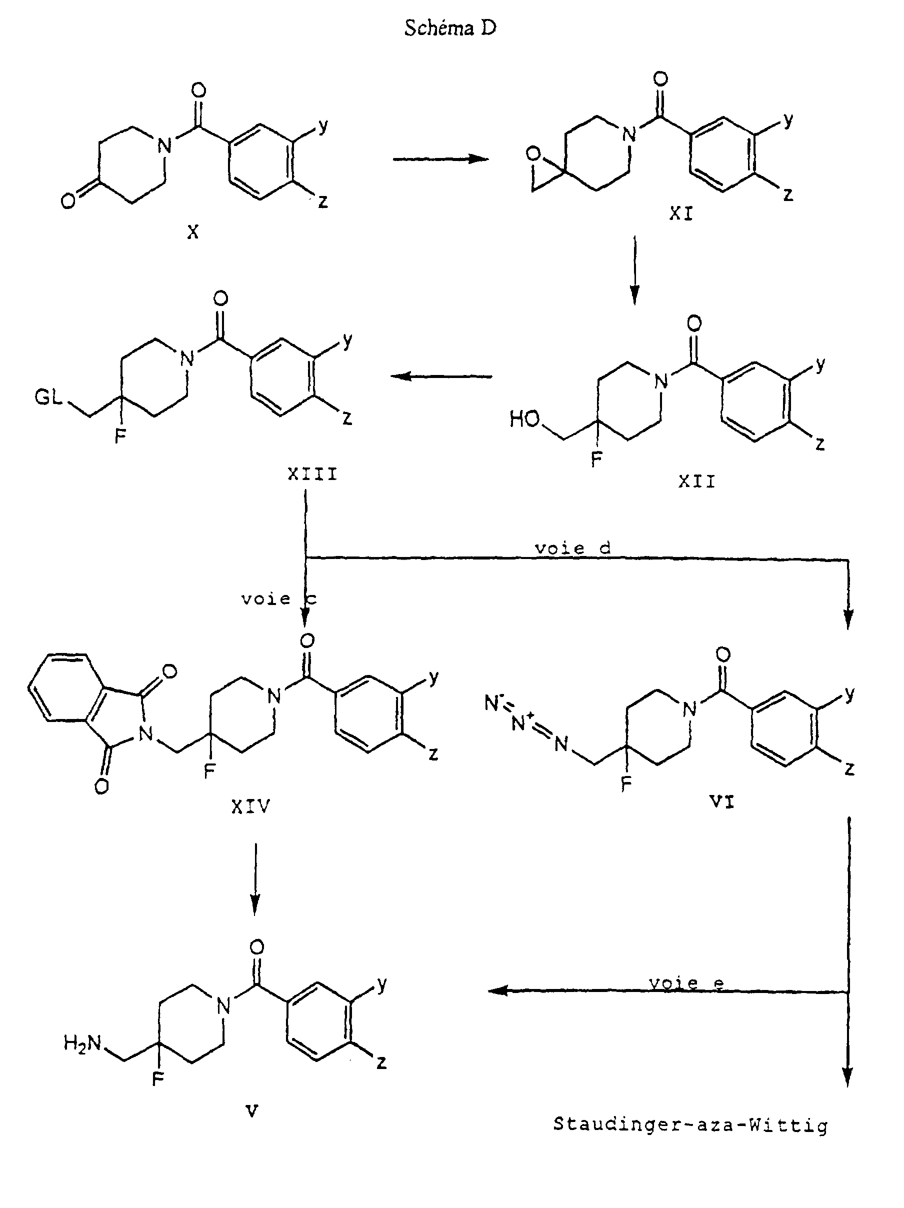

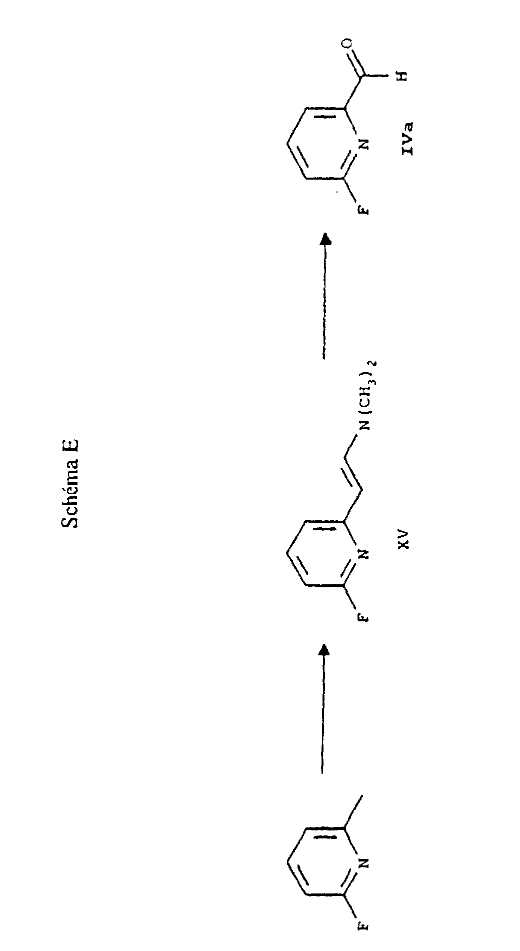

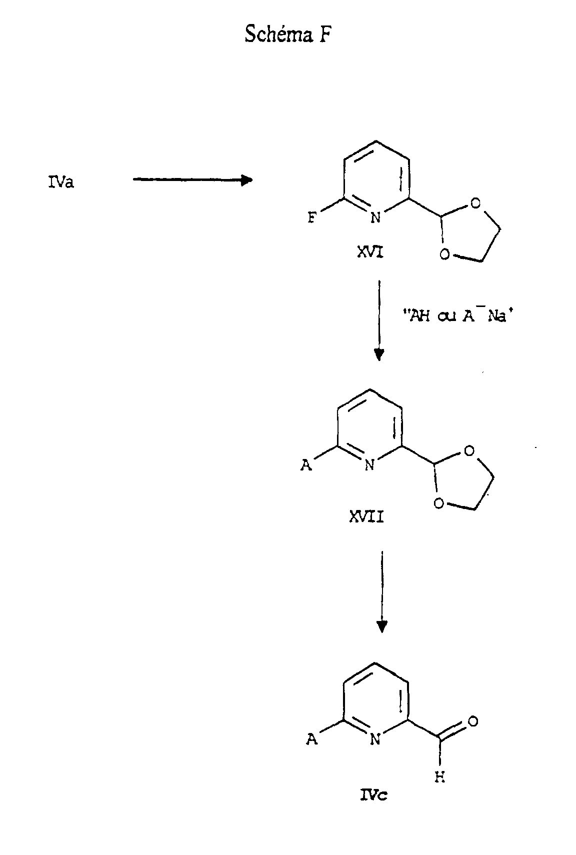

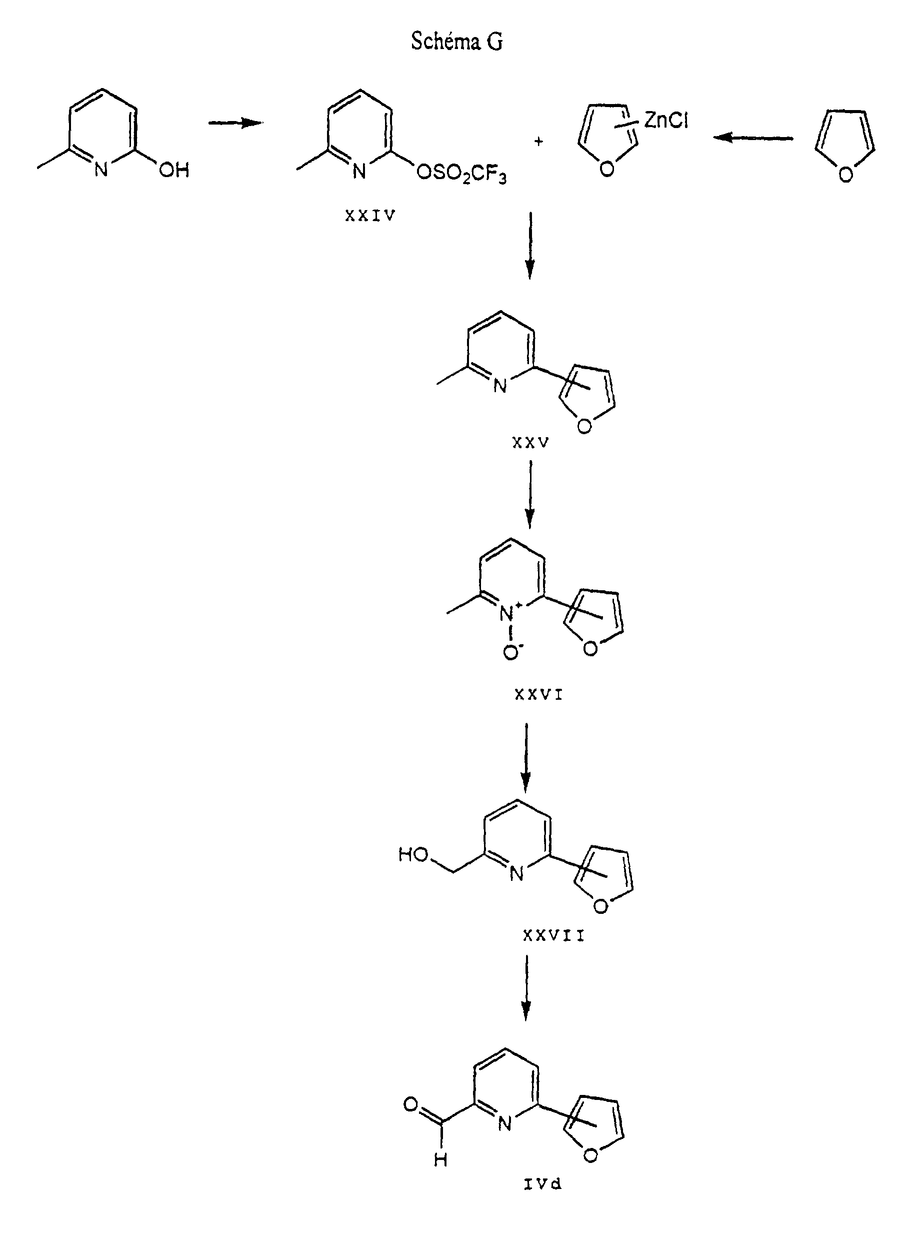

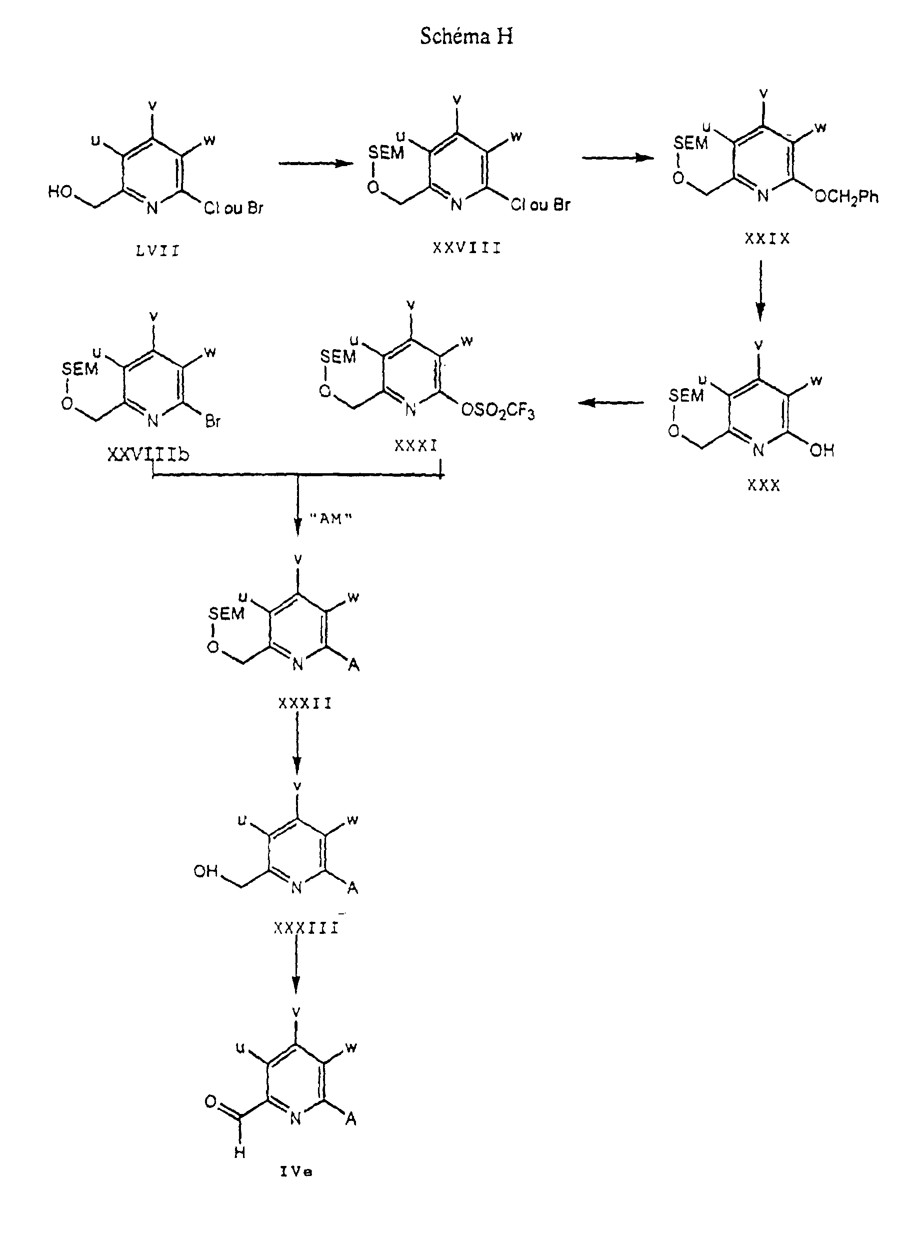

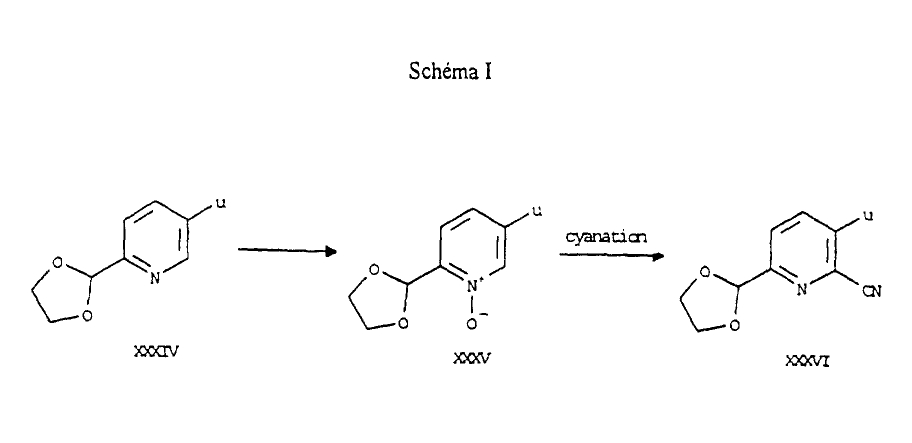

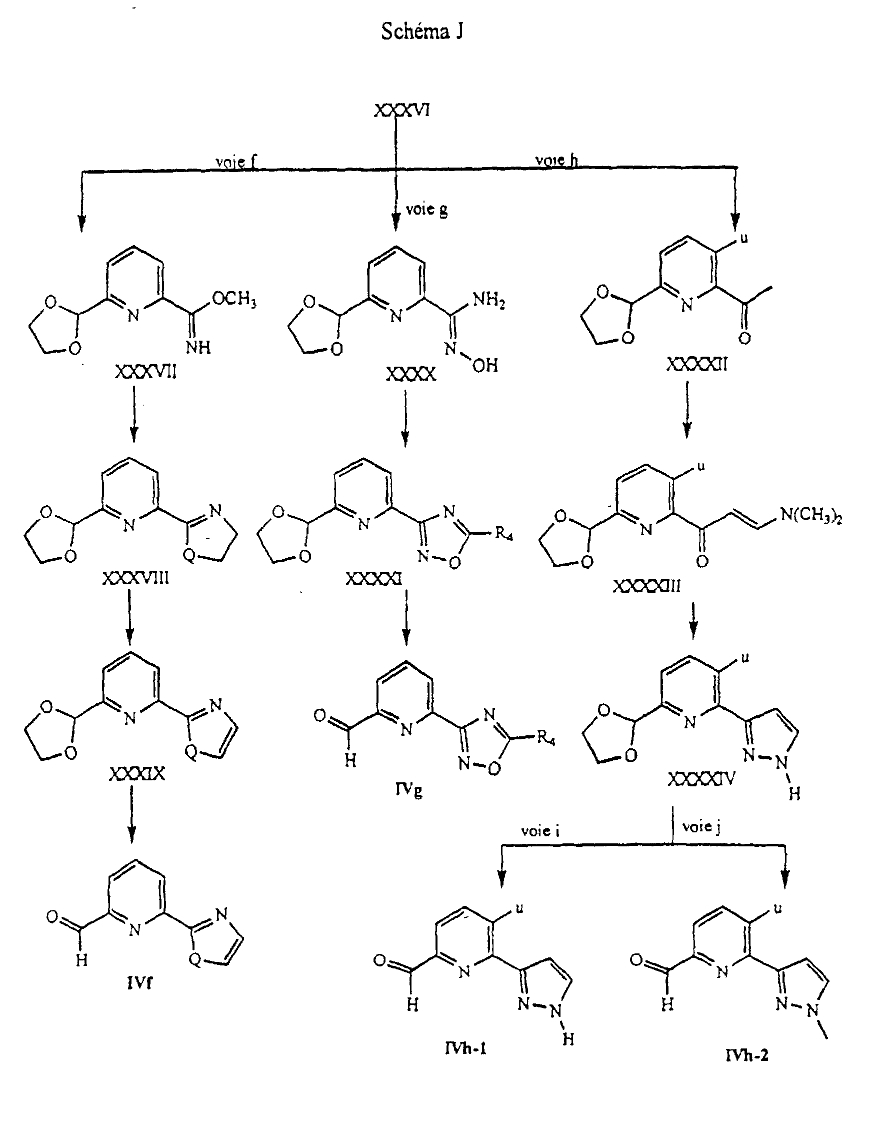

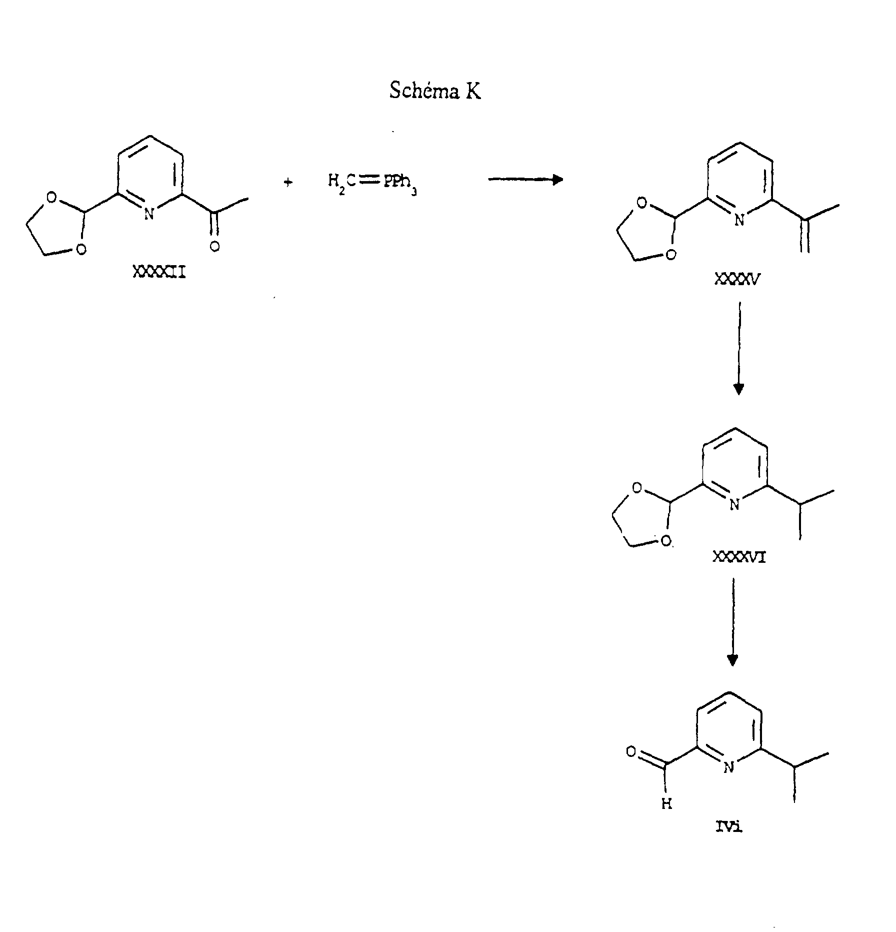

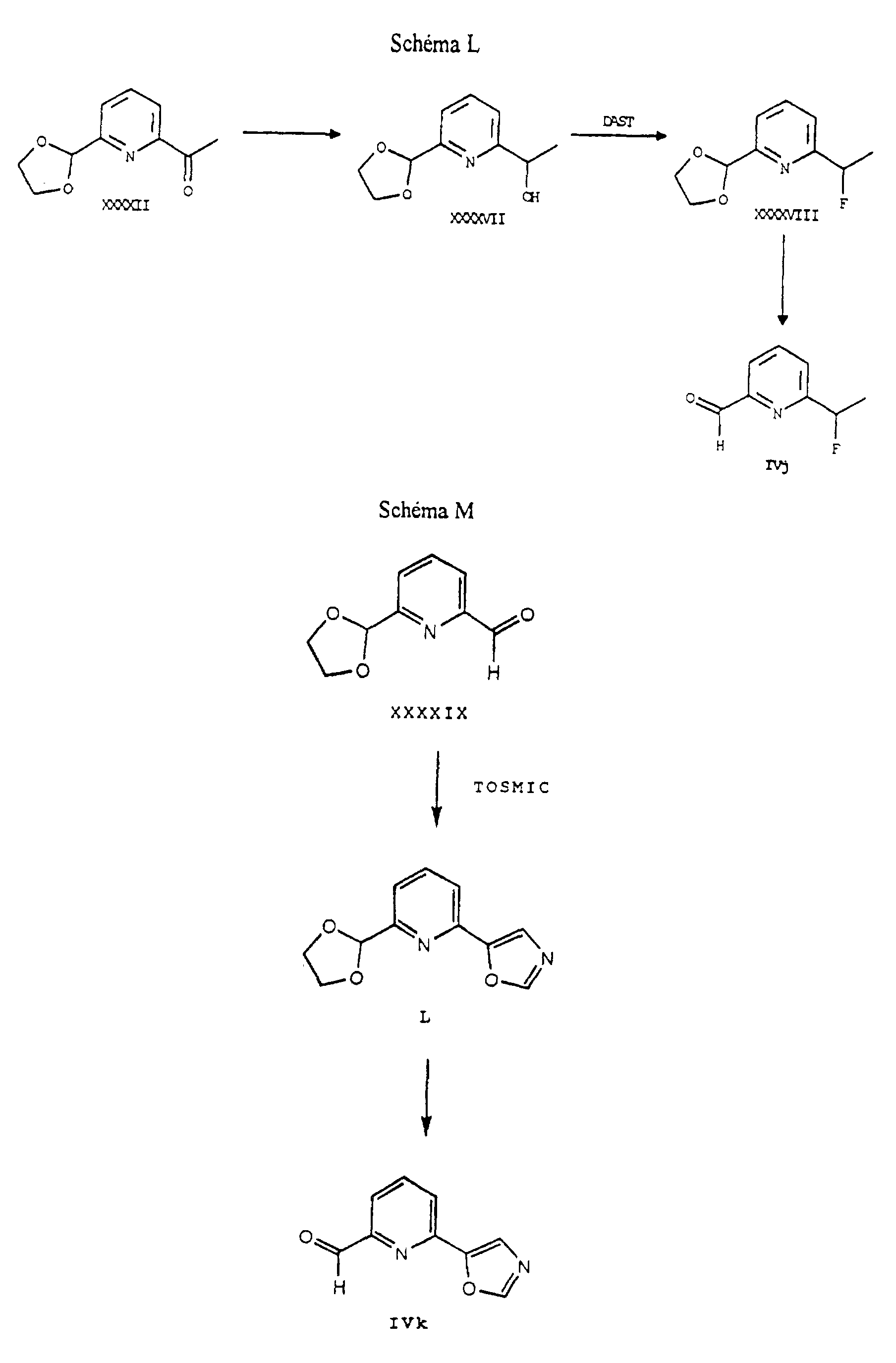

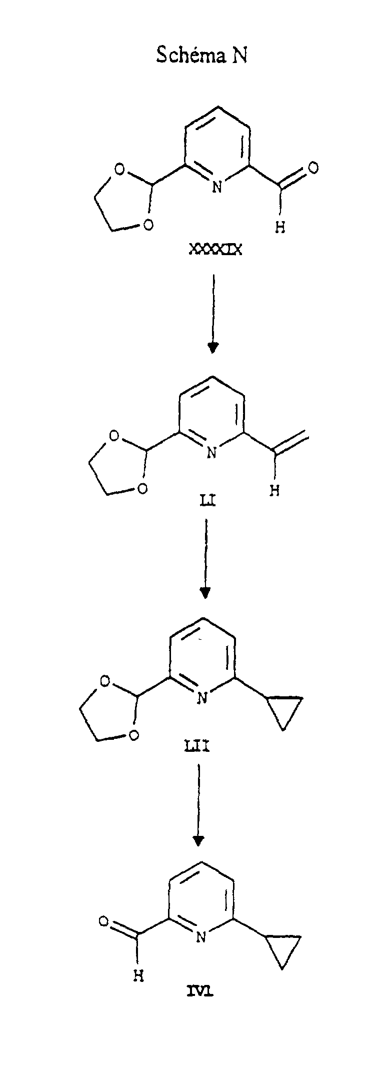

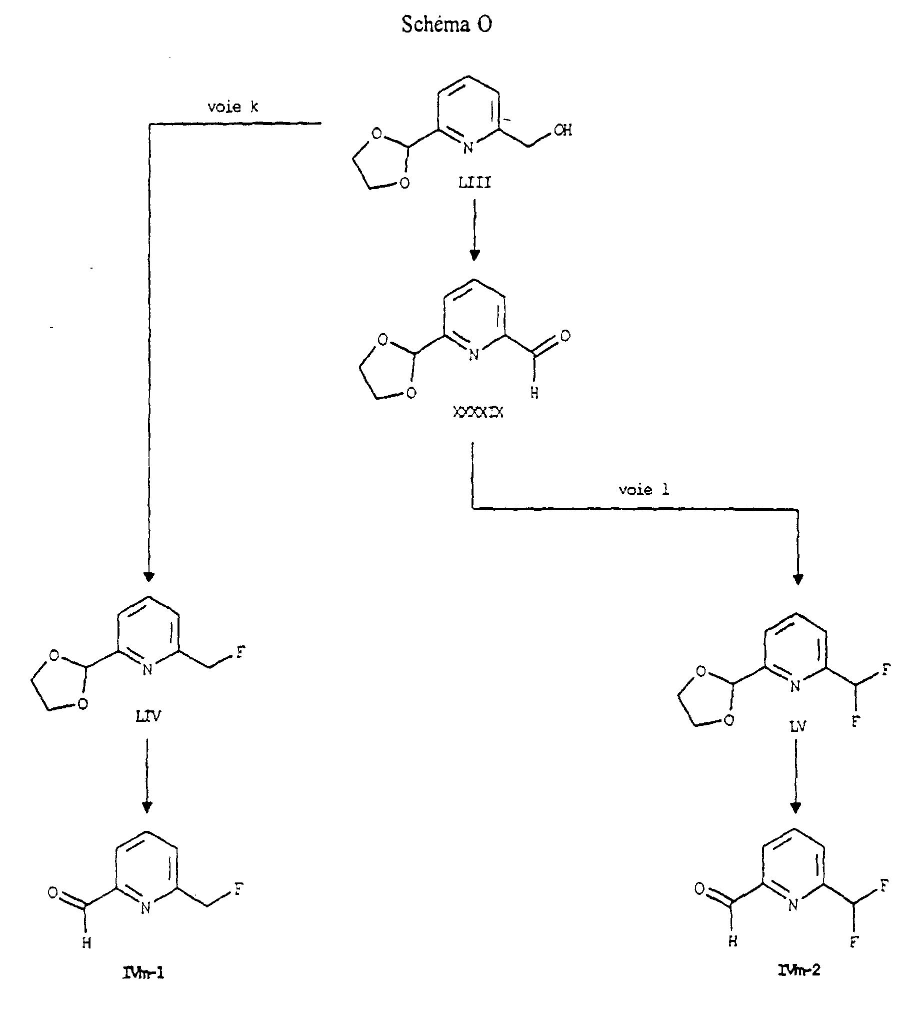

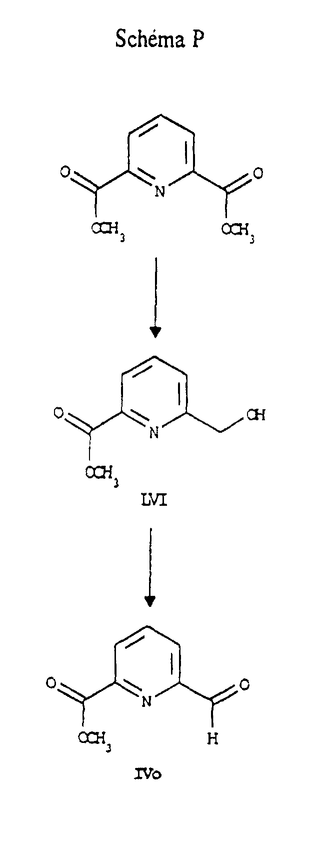

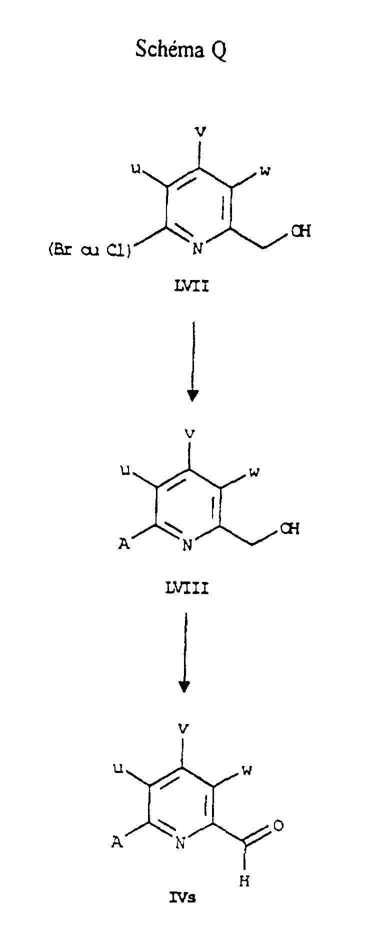

Les exemples suivants illustrent l'invention et ne la limitent en aucune façon.

Dans les exemples ci-après :
(i) l'avancement des réactions est suivi par chromatographie couche mince (CCM) et par conséquent les temps de réaction ne sont mentionnés qu'à titre indicatif.
(ii) des formes cristallines différentes peuvent donner des points de fusions différents, les points de fusion rapportés dans la présente demande sont ceux des produits préparés selon la méthode décrite et ne sont pas corrigés.
(iii) la structure des produits obtenus selon l'invention est confirmée par les spectres de résonance magnétique nucléaire (RMN), infrarouge (IR) et l'analyse centésimale, la pureté des produits finaux est vérifiée par CCM.
(iv) les spectres RMN sont enregistrés dans le solvant indiqué. Les déplacements chimiques (δ) sont exprimés en partie par million (ppm) par rapport au tetraméthylsilane. La multiplicité des signaux est indiquée par: s, singulet; d, doublet; t, triplet; q, quadruplet; m, multiplet; l, large.
(v) les différents symboles des unités ont leur signification habituelle: mg (milligramme); g (gramme); Kg (kilogramme); ml (millilitre); °C (degré Celsius); mmole (millimole); nmole (nanomole); cm (centimètre); µm (micromètre).
   Les abréviations ont la signification suivante: F ( point de fusion); Eb (point d'ébullition).
(vi) par "température ambiante" on entend une température comprise entre 20°C et 25°C.

Dans la présente application les pressions sont données en millibars.

### EXEMPLE 1 : Préparation du 6-Fluoro-pyridine-2-carbaldéhyde (IVa)

On mélange 30 g de 2-fluoro-6-méthyl-pyridine (270 mmoles) et 70 g de tert-butoxy-bis(diméthylamino)méthane (405 mmoles) sous atmosphère d'azote. On chauffe le mélange à 140°C pendant 24 heures. On dilue le mélange avec 50 ml de tétrahydrofurane et on ajoute la solution obtenue goutte à goutte dans une solution aqueuse de 115 g de periodate de sodium (538 mmoles). On agite une nuit à température ambiante, on élimine le précipité formé par filtration puis on évapore le tétrahydrofurane. On extrait le résidu par du dichlorométhane, la phase organique est séchée sur sulfate de magnésium, filtrée puis le solvant est évaporé sous vide. Le produit du titre est isolé par distillation au four à boules, Eb₇₇: 70-80°C. On récupère 34 g d'une huile jaune contenant environ 20 % de N,N-diméthylformamide.
¹H RMN (CDCl₃) δ : 7.16 (dd, 1H); 7.82 (dd, 1H); 7.96 (m, 1H); 9.90 (s, 1H).
IR (film) υ: 1713 cm⁻¹ (C=O).

### EXEMPLE 2 : Préparation du 6-diméthylamino-pyridine-2-carbaldéhyde (IVc-1)

### Etape 1 : (6-[1,3]dioxolan-2-yl-pyridin-2-yl)-diméthylamine

On mélange 0.60 g de 2-[1,3]dioxolan-2-yl-6-fluoro-pyridine (3.55 mmoles) et 2.50 ml de diméthylamine à 33 % dans l'éthanol (17.7 mmoles) puis on porte la température à 100°C pendant 12 heures. Après évaporation sous vide, on reprend le résidu par du chloroforme, on lave à l'eau, on sèche sur sulfate de magnésium, on filtre et on évapore le chloroforme sous vide. On obtient le produit du titre sous la forme d'une huile jaune qui est utilisée dans l'étape suivante sans autre purification.
¹H RMN (CDCl₃) δ: 3.07 (s, 6H); 4.10 (m, 4H); 5.72 (s, 1H); 6.46 (d, 1H); 6.73 (d, 1H); 7.45 (dd, 1H).

### Etape 2 : 6-diméthylamino-pyridine-2-carbaldéhyde

On mélange 0.60 g de (6-[1,3]dioxolan-2-yl-pyridin-2-yl)-diméthylamine (3.09 mmoles) dans 10 ml d'une solution aqueuse d'acide formique à 80 % puis on porte la solution à 60°C pendant 20 heures. On élimine les solvants par entraînement azéotropique avec du toluène, on reprend le résidu dans l'eau, on refroidi le mélange à 0°C puis on basifie le milieu par addition de carbonate de potassium. On extrait à l'acétate d'éthyle, on lave la phase organique à l'eau puis avec une solution aqueuse saturée en chlorure de sodium, on sèche sur sulfate de magnésium, on filtre on évapore le solvant sous vide. On obtient le produit du titre sous la forme d'une huile jaune (0.43 g) qui est utilisé dans l'étape suivante sans autre purification.
¹H RMN (CDCl₃)δ: 3.12 (s, 6H); 6.68 (d, 1H); 7.18 (d, 1H); 7.55 (dt, 1H); 9.87 (s, 1H).
IR (film) υ: 1697 cm⁻¹ (C=O).

### EXEMPLE 3 : Préparation du 6-furan-2-yl-pyridine-2-carbaldéhyde (IVd-1)

### Etape 1 : 2-furan-2-yl-6-méthyl-pyridine

Dans une solution de 4.36 ml de furan (60 mmoles) et 40 ml de tétrahydrofurane refroidie à 0°C, on introduit goutte à goutte 40 ml d'une solution de n-butyllithium 1.6 M dans l'hexane. On agite la solution pendant 3 heures à 0° C puis on la refroidie à - 40°C avant d'introduire 120 ml d'une solution de dichlorure de zinc 0.5 M dans le tétrahydrofurane. On agite pendant 2 heures à température ambiante puis le mélange réactionnel est ajouté à une solution de 9.17 g de trifluorométhanesulfonic acid 6-méthyl-pyridin-2-yl ester (40 mmoles) dans 20 ml de tétrahydrofurane contenant 2.30 g de tétrakis(triphénylphosphine)palladium (2 mmoles). Le mélange réactionnel est maintenu 2 heures aux reflux sous azote puis refroidi et extrait par HCl 1N. La phase aqueuse acide est neutralisée par NaOH 10N puis extraite par de l'acétate d'éthyle. La phase organique est lavée à l'eau, séchée sur sulfate de magnésium, filtrée et le solvant évaporé. Le produit attendu est isolé par rectification sous pression réduite.
Eb_{5.6.10}⁻² = 100-105°C.
On récupère 5.20 g d'une huile incolore.
Rendement: 81.5 %
¹H RMN (CDCl₃) δ: 2.56 (s, 3H); 6.48 (dd, 1H); 6.99 (m, 2H); 7.50 (m, 3H).

### Etape 2 : 2-furan-2-yl-6-méthyl-pyridine-1-oxyde

Dans une solution de 2.20 g de 2-furan-2-yl-6-méthyl-pyridine (13.8 mmoles) dans 50 ml de chloroforme refroidi à 0°C, on additionne par fraction 4.90 g d'acide métachloroperbenzoïque. On agite 12 heures à température ambiante puis on élimine le précipité par filtration et on lave le filtrat avec une solution aqueuse de bicarbonate de sodium à 5 %. Après séchage sur sulfate de magnésium, filtration et évaporation le produit du titre est isolé par chromatographie sur colonne de silice (éluant : chloroforme / méthanol; 99 : 1). On récupère 1.25 g d'une poudre blanche.
Rendement: 52 %
F: 79°C
¹H RMN (CDCl₃) δ : 2.58 (s, 3H); 6.60 (q, 1H); 7.18 (m, 2H); 7.58 (d, 1H); 7.85 (dd, 1H); 8.03 (d, 1H).

### Etape 3 : (6-furan-2-yl-pyridin-2-yl)-méthanol

On dissout 2.75 g de 2-furan-2-yl-6-méthyl-pyridine-1-oxyde (15.7 mmoles) dans 27 ml de tétrahydrofurane. Dans la solution refroidie à 0°C sous atmosphère d'azote, on ajoute 6.65 ml d'anhydride trifluoroacétique (47.1 mmoles). On agite 12 heures à température ambiante puis on additionne 25 ml d'une solution aqueuse de soude 4N. Le tétrahydrofurane est évaporé, on reprend le résidu par une solution aqueuse saturée en chlorure de sodium puis on extrait au chloroforme. La phase organique est séchée sur sulfate de magnésium, filtrée, concentrée sous vide. Le produit du titre est isolé par chromatographie sur colonne de silice (éluant: chloroforme / acétate d'éthyle; 90 : 10). On récupère 2.03 g d'une huile jaune.
Rendement: 73.8 %
¹H RMN (CDCL₃) δ : 3.99 (si, 1H(échangeable)); 4.74 (s, 2H); 6.53 (q, 1H); 7.09 (m, 2H); 7.57 (m, 2H); 7.71 (t, 1H).

### Etape 4 : 6-furan-2-yl-pyridine-2-carbaldéhyde

Dans une solution de 2 g de (6-furan-2-yl-pyridin-2-yl)-méthanol (11.4 mmoles) et 50 ml de chloroforme, on ajoute 8 g de dioxyde de manganèse. Le mélange réactionnel est porté au reflux pendant 1 heure 30 minutes avec élimination de l'eau formée en continu. Le solide en suspension est éliminé par filtration sur célite puis le solvant évaporé. Le produit du titre est isolé par chromatographie sur colonne de silice (éluant : chloroforme). On récupère 1.45 g d'un solide jaune.
Rendement: 73.4 %
F: 46-48°C
¹H RMN (CDCl₃) δ : 6.55 (q, 1H); 7.16 (d, 1H); 7.55 (d, 1H); 7.82 (m, 3H); 10.07 (s, 1H)
IR (KBr) υ: 1717 cm⁻¹ (C=O).

### EXEMPLE 4 : 6-furan-3-yl-pyridine-2-carbaldéhyde (IVd-2)

En procédant comme dans l'exemple 3 mais en remplaçant dans l'étape 1 le 2-lithiofuran par du 3-lithiofuran obtenu par échange halogène / métal à partir du 3-bromofuran, on prépare le composé du titre.
F: 59-61°C
¹H RMN (CDCl₃) δ: 6.94 (q, 1H); 7.54 (t, 1H); 7.68 (dd, 1H); 7.83 (m, 2H); 8.12 (s, 1H); 10.05 (s, 1H).
IR (KBr) υ:1713 cm⁻¹ (C=O).

### EXEMPLE 5 : 6-(1H-pyrrol-2-yl)-pyridine-2-carbaldéhyde (IVe-1)

### Etape 1 : 2-chloro-6-(2-triméthylsilanyl-éthoxyméthoxyméthyl)-pyridine

Dans une solution contenant 2.50 g de (6-chloro-pyridin-2-yl)-méthanol (17.4 mmoles), 3.30 ml de diisopropyléthylanune (19.1 mmoles) et 20 ml de dichlorométhane refroidie à 0°C maintenue sous atmosphère d'azote, on ajoute goutte à goutte 3.20 ml de chlorométhyl-2-(triméthylsilyl)éthyl éther (18.2 mmoles). On agite 3 heures à température ambiante puis on évapore le dichlorométhane, on reprend le résidu dans l'eau, on extrait à l'éther diéthylique, la phase organique est lavée à l'eau puis séchée sur sulfate de sodium. Après filtration et évaporation du solvant le produit du titre est isolé par chromatographie sur colonne de silice (éluant: dichlorométhane). On récupère 3.65 g d'huile incolore.
Rendement: 76.6 %
¹H RMN (CDCl₃) δ: 0 (s, 9H); 0.95 (t, 2H); 3.68 (t, 2H); 4.67 (s, 2H); 4.80 (s, 2H); 7.24 (d, 1H); 7.35 (d, 1H); 7.64 (t, 1H).

### Etape 2 : 2-benzyloxy-6-(2-triméthylsilanyl-éthoxyméthoxyméthyl)-pyridine

Dans une suspension de 0.91 g d'hydrure de sodium (22.6 mmoles) dans 20 ml de N,N-diméthylformamide refroidie à 0°C et maintenue sous atmosphère d'azote, on ajoute goutte à goutte 2.10 ml d'alcool benzylique (19.9 mmoles) dilués dans 3 ml de N,N-diméthylformamide. On agite pendant 1 heure 30 minutes à 0°C puis on additionne goutte à goutte une solution de 3.65 g de 2-chloro-6-(2-triméthyl silanyl-éthoxyméthoxyméthyl)-pyridine (13.3 mmoles) dans 3 ml de N,N-diméthylformamide. On porte la réaction à 40°C pendant 12 heures, le mélange réactionnel est versé dans l'eau glacé, extrait à l'éther diéthylique puis les phases organiques combinées sont lavées à l'eau, séchées sur sulfate de magnésium. Après filtration et évaporation du solvant le produit du titre est isolé par chromatographie sur colonne de silice (éluant : dichlorométhane / hexane; 60 : 40). On récupère 3 g d'une huile incolore.
Rendement: 65.3 %
¹H RMN (CDCl₃) δ: 0 (s, 9H); 0.94 (t, 2H); 3.67 (t, 2H); 4.61 (s, 2H); 4.80 (s, 2H); 5.35 (s, 2H); 6.66 (d, 1H); 6.97 (d, 1H); 7.34 (m, 3H); 7.42 (m, 2H); 7.54 (t, 1H),

### Etape 3 : 6-(2-triméthylsilanyl-éthoxyméthoxyméthyl)-pyridin-2-ol

Dans une solution de 7 g de 2-benzyloxy-6-(2-triméthylsilanyl-éthoxyméthoxyméthyl)-pyridine dans 75 ml d'éthanol saturée en hydrogène, on ajoute 7g de Nickel de Raney. On agite la suspension vigoureusement sous faible pression d'hydrogène à température ambiante pendant 1 heure 30 minutes. On élimine le solide en suspension par filtration sur célite puis on évapore l'éthanol. Le produit du titre est isolé par chromatographie sur colonne de silice (éluant: dichlorométhane / méthanol; 98 : 2). On récupère 4 g d'une huile incolore.
Rendement: 76.8 %
¹H RMN (CDCl₃) δ : 0 (s, 9H); 0.93 (t, 2H); 3.61 (t, 2H); 4.50 (s, 1H); 4.75 (s, 2H); 6.21 (d, 1H); 6.47 (d, 1H); 7.36 (d, 1H); 7.41 (d, 1H); 12.28 (s, 1H (échangeable)).

### Etape 4 : trifluorométhanesulfonic acid 6-(2-triméthylsilanyl-éthoxyméthoxyméthyl)-pyridine-2-yl ester

Dans une solution de 3.80 g de 6-(2-triméthylsilanyl-éthoxyméthoxyméthyl)-pyridine-2-ol (14.8 mmoles) et 35 ml de pyridine contenant 0.10 g de 4-N,N-diméthylaminopyridine maintenue à 0°C sous atmosphère d'azote, on ajoute goutte à goutte 2.63 ml d'anhydride trifluorométhanesulfonique (14.9 mmoles). On agite la solution pendant 2 heures à 0°C puis le mélange est versé dans de l'eau glacée et extrait à l'éther diéthylique. La phase organique est lavée avec une solution aqueuse d'hydrogénosulfate de potassium puis à l'eau, séchée sur sulfate de magnésium, filtrée et évaporée. Le produit du titre est isolé par chromatographie sur colonne de silice (éluant : dichlorométhane). On récupère 4.30 g d'une huile incolore.
Rendement: 75 %
¹H RMN (CDCl₃) δ: 0 (s, 9H); 0.93 (t, 2H); 3.66 (t, 2H); 4.68 (s, 2H); 4.80 (s, 2H); 7.05 (d, 1H); 7.52 (d, 1H); 7.87 (t, 1H).

### Etape 5 : 2-[6-(2-triméthylsilanyl-éthoxyméthoxyméthyl)-pyridin-2-yl]pyrrole-1-carboxylic acid tert-butyl ester

La préparation du 1-tert-butoxycarbonylpyrrol-2-yl boronic acid est effectuée selon la méthode décrite dans Synthesis, 1991, 613-15.
Dans une solution de 4 g de trifluorométhanesulfonic acid 6-(2-triméthylsilanyl-éthoxyméthoxyméthyl)-pyridin-2-yl ester (10.3 mmoles) et 40 ml de benzène dégazé par barbotage d'azote, on ajoute 9.68 g de carbonate de thallium (20.6 mmoles), 1 g de tétrakis(triphénylphosphine)palladium (0.86 mmole) et 2.45 g de 1-tert-butoxycarbonylpyrrol-2-yl boronic acid (11.6 mmoles). On agite pendant 23 heures sous atmosphère d'argon. Les insolubles sont éliminés par filtration sur célite puis la solution concentrée sous vide. Le produit du titre est isolé par chromatographie sur colonne de silice (éluant: dichlorométhane /acétate d'éthyle; 99 : 1). On récupère 3.90 g d'une huile jaune.
Rendement: 93.6 %
¹H RMH (CDCl₃) δ: 0 (s, 9H); 0.94 (t, 2H); 1.32 (s, 9H); 3.67 (t, 2H); 4.73 (s, 2H); 4.82 (s, 2H); 6.21 (t, 1H); 6.37 (m, 1H); 7.27 (d, 1H); 7.32 (m, 1H); 7.34 (d, 1H); 7.68 (t, 1H).

### Etape 6 : [6-(1H-pyrrol-2-yl)-pyridin-2-yl]-méthanol

Dans une solution de 3 ml de fluorure de tétrabutylammonium 1.1M dans le tétrahydrofurane, 45 ml de tétrahydrofurane et 5 ml d'hexaméthylphophoramide, contenant 10 g de tamis moléculaire (4 angstroem), on ajoute 3 g de 2-[6-(2-triméthylsilanyl-éthoxyméthoxyméthyl)-pyridin-2-yl]pyrrole-1-carboxylic acid tert-butyl ester (7.41 mmoles). On agite à 45°C sous argon pendant 8 heures. Après filtration des insolubles sur célite le tétrahydrofurane est évaporé. Le produit du titre est purifié par chromatographie sur colonne de silice (éluant: dichlorométhane / acétate d'éthyle; 99 : 1). On récupère 0.50 g d'un solide blanc.
Rendement: 38.7 %
F: 73-75°C
¹H RMN (CDCl₃) δ: 3.61 (sl, 1H); 4.74 (s, 2H); 6.30 (dd, 1H); 6.73 (m, 1H); 6.92 (m, 1H); 6.98 (d, 1H); 7.45 (d, 1H); 7.62 (t, 1H); 9.56 (sl, 1H).

### Etape 7 : 6-(1H-pyrrol-2-yl)-pyridine-2-carbaldéhyde

Dans une solution de 0.30 g de [6-(IH-pyrrol-2-yl)-pyridin-2-yl]-méthanol (1.72 mmoles) dans 6 ml de dichlorométhane, on ajoute 0.75 g de dioxyde de manganèse (8.61 mmoles). On agite vigoureusement la suspension pendant 3 heures à température ambiante puis on élimine les insolubles par filtration sur célite. Après évaporation du dichlorométhane, le résidu est purifié par chromatographie sur colonne de silice (éluant: dichlorométhane). On récupère 0.20 g d'une poudre blanche.
Rendement: 69 %
F: 112-113°C
¹H RMN (CDCl₃) δ. 6.31 (dd, 1H); 6.77 (m, 1H); 6.95 (m, 1H); 7.63-7.81 (m, 3H); 9.67 (sl, 1H); 10.03 (s, 1H).
IR (KBr) υ: 1701 cm⁻¹ (C=O).

### EXEMPLE 6 : Préparation du 6-thiophen-2-yl-pyridine-2-carbaldéhyde (IVe-2)

En procédant comme dans l'exemple 5 mais en remplaçant dans l'étape 5 le 1-tert-butoxycarbonylpyrrol-2-yl boronic acid par le thiophen-2-yl boronic acid disponible commercialement, on obtient le composé du titre sous forme d'un solide jaune.
F: 48-50 °C
¹H RMN (CDCl₃) δ: 7.15 (d, 1H); 7.46 (dd, 1H); 7.69 (dd, 1H); 7.78-7.88 (m, 3H); 10.16 (s, 1H).
IR (KBr) υ: 1714 cm⁻¹ (C=O).

### EXEMPLE 7 : Préparation du 6-thiazol-2-yl-pyridine-2-carbaldéhyde (IVf-1)

### Etape 1 : 6-[1,3]dioxolan-2-yl-pyridine-2-carbonitrile

On dissout 3.34 g de 2-[2,3]dioxolan-2-yl-pyridine 1-oxyde (19.98 mmoles) dans 40 ml de dichlorométhane puis on ajoute successivement 2.93 ml de cyanotriméthylsilane (21.97 mmoles) et 2.53 ml de chlorure de diéthylcarbamoyle (19.96 mmoles). On agite la solution pendant 240 heures à température ambiante sous atmosphère d'azote. On verse lentement le mélange réactionnel dans une solution aqueuse glacée de carbonate de potassium à 10 %, on extrait par du chloroforme puis la phase organique est lavée par une solution aqueuse de carbonate de potassium à 10 %. On sèche sur sulfate de magnésium, on filtre et on évapore le chloroforme sous vide. Le composé du titre est isolé par chromatographie sur colonne de silice (éluant: chloroforme). On récupère 2.75 g d'un solide blanc.
Rendement: 78.1 %
F: 64°C
¹H RMN (CDCl₃) δ: 4.14 (m, 4H); 5.86 (s, 1H); 7.71 (dd, 1H); 7.78 (dd, 1H); 7.91 (t, 1H).

### Etape 2 : 6-[1,3]dioxolan-2-yl-pyridine-2-carboximidic acid méthyl ester

A une solution de 2 g de 2-[1,3]dioxolan-2-yl-pyridine-2-carbonitrile (11.35 mmoles) dans 10 ml de méthanol on ajoute 0.20 g de méthylate de sodium (3.7 mmoles). Le mélange réactionnel est agité pendant 24 heures sous atmosphère d'azote. On évapore le méthanol puis on reprend le résidu par une solution aqueuse saturée en chlorure de sodium et l'on extrait par de l'acétate d'éthyle. La phase organique est séchée sur sulfate de sodium, filtrée et le solvant évaporé. On obtient 2.30 g d'un solide blanc qui est utilisé directement dans l'étape suivante sans autre purification.

### Etape 3 : 2-(4,5-dihydro-thiazol-2-yl)-6-[1,3]dioxolan-2-yl-pyridine

On mélange 1 g de 6-[1,3]dioxolan-2-yl-pyridine-2-carboximidic acid méthyl ester (4.80 mmoles) et 0.47 g de 2-aminoéthanethiol (2.16 mmoles). On porte le mélange à 130°C pendant 1 heure 30 minutes. Le mélange réactionnel est repris dans du chloroforme, lavé à l'eau puis séché sur sulfate de magnésium. Après filtration et évaporation du solvant le produit du titre est isolé par chromatographie sur colonne de silice (éluant: chloroforme / méthanol; 98 : 2). On récupère 1.10 g de solide.
Rendement: 97 %
F: 50-52°C
¹H RMN (CDCl₃) δ: 3.36 (t, 2H); 4.15 (m, 4H); 4.55 (t, 2H); 5.91 (s, 1H); 7.60 (dd, 1H); 7.81 (t, 1H); 8.03 (dd, 1H).

### Etape 4 : 2-thiazol-2-yl-6-[1,3]dioxolan-2-yl-pyridine

A une solution de 1.33 g de 2-(4,5-dihydro-thiazol-2-yl)-6-[1,3]dioxolan-2-yl-pyridine (5.62 mmoles) dans 50 ml de benzène maintenue au reflux avec élimination de l'eau formée en continue, on additionne 10 g d'hydrate de peroxyde de nickel par fraction de 1 g pendant 20 heures. Les insolubles sont éliminés par filtration sur célite puis on évapore le solvant. Le produit du titre est isolé par chromatographie sur colonne de silice (éluant : chloroforme /acétate d'éthyle; 85 : 15). On récupère 0.42 g d'un solide blanc.
Rendement: 31.5 %
F: 71 °C
¹H RMN (CDCl₃) δ: 4.16 (m, 4H); 5.91 (s, 1H); 7.42 (d, 1H); 7.54 (dd, 1H); 7.81 (d, 1H); 7.89 (t, 1H); 8.17 (dd, 1H).

### Etape 5 : 6-thiazol-2-yl-pyidine-2-carbaldéhyde

Une solution de 0.93 g de 2 thiazol-2-yl-6-[1,3]dioxolan-2-yl-pyridine (3.97 mmoles) dans 8 ml d'acide formique et 2 ml d'eau est chauffé à 60°C pendant 1 heure 30 minutes. Les solvants sont éliminés par entraînement azéotropique avec du toluène. On reprend le résidu dans l'eau glacée, on neutralise par une solution aqueuse de soude 4N puis on extrait à l'acétate d'éthyle. La phase organique est lavée par une solution saturée de chlorure de sodium puis séchée sur sulfate de magnésium, filtrée et le solvant évaporé. Le produit du titre est cristallisé par addition d'éther isopropylique. On récupère 0.59 g d'un solide blanc.
Rendement: 78 %
F: 91°C
¹H RMN (CDCl₃) δ: 7.48 (d, 1H); 7.96 (m, 3H); 8.38 (m, 1H); 10.09 (s, 1H).
IR (KBr) υ: 1705 cm⁻¹ (C=O).

### EXEMPLE 8 : Préparation du 2-[1,3]dioxolan-2-yl-6-(1H-imidazol-2-yl)-pyridine-2-carbaidéhyde (IVf-2)

En procédant comme dans l'exemple 7 mais en remplaçant dans l'étape 3 le 2-aminoéthanethiol par de l'éthylènediamine et dans l'étape 4 l'hydrate de peroxyde de nickel par du permanganate de baryum, on obtient le composé du titre sous forme d'un solide jaune.
F: 148°C
¹H RMN (CDCl₃) δ: 4.77 (s, 1H (échangeable)); 7.22 (d, 2H), 7.89 (m, 2H); 8.35 (d, 1H); 10.03 (s, 1H).
IR (KBr) υ: 1704 cm⁻¹ (C=O).

### EXEMPLE 9 : Préparation du 6-(methyl-5-oxadiazol-3-yl)-pyridine-2-carbaldéhyde (IVg-1)

### Etape 1 : 6-[1,3]dioxolan-2-yl-N-hydroxy-pyridine-2-carboxamidine

On mélange 0.88 g de 2-[1,3]dioxolan-2-yl-pyridine-2-carbonitrile (4.99 mmoles), 1.74 g de chlorhydrate d'hydroxylamine (25 mmoles) et 3.45 g de carbonate de potassium (25 mmoles) dans 15 ml d'éthanol puis on porte le mélange réactionnel au reflux pendant 4 heures. Après évaporation de l'éthanol, le produit du titre est cristallisé par addition d'eau dans le résidu. On récupère 0.80 g d'une poudre incolore.
Rendement: 76.6 %
F: 148°C
¹H RMN (DMSOd6) δ: 4.06 (m, 4H); 5.77 (s, 1H); *5.82* (s, 2H(échangeables)); 7.52 (m, 1H); 7.85 (m, 2H); 9.98 (s, 1H(échangeable)).

### Etape 2 : 2-[1,3]dioxolan-2-yl-6-(méthyl-5-oxadiazol-3-yl)-pyridine

On dissout 0.30 g de 6-[1,3]dioxolan-2-yl-N-hydroxy-pyridine-2-carboxamidine (1.43 mmole) dans 0.60 ml de pyridine puis on additionne 0.15 ml de chlorure d'acétyle (2.11 mmoles). Le mélange réactionnel est porté au reflux du solvant pendant 2 heures 15 minutes. On verse le mélange dans une solution aqueuse glacée de sulfate acide de potassium puis on extrait à l'acétate d'éthyle. On lave la phase organique par une solution aqueuse saturée en chlorure de sodium, on séche sur sulfate de magnésium, on filtre et on évapore l'acétate d'éthyle. Le produit du titre est isolé par chromatographie sur colonne de silice (éluant: chloroforme /acétate d'éthyle; 80 : 20).
On récupère 0.15 g d'une poudre blanche
Rendement: 45 %
F: 90-91°C
¹H RMN (CDCl₃) δ: 2.67 (s, 3H); 4.13 (m, 4H); 5.95 (s, 1H); 7.68 (dd, 1H); 7.87 (t, 1H); 8.07 (t, 1H).

### Etape 3 : 6-(méthyl-5-oxadiazol-3-yl)-pyridine-2-carbaldéhyde

L'hydrolyse acide de 0.74 g de 6-[1,3]dioxolan-2-yl-N-hydroxy-pyridine-2-carboxamidine (3.17 mmoles) est effectuée selon un protocole analogue à celui utilisé dans l'étape 5 de l'exemple 7.
On obtient 0.42 g du composé du titre sous forme d'une poudre blanche.
Rendement: 70 %
F: 123°C
¹H RMN (CDCl₃) δ: 2.74 (s, 3H); 8.04 (t, 1H); 8.10 (dd, 1H); 8.34 (dd, 1H); 10.23 (s, 1H).
IR (KBr) υ: 1703 cm⁻¹ (C=O).

### EXEMPLE 10: Préparation du 6-(1H-pyrazol-3-yl)-pyridine-2-carbaldéhyde (IVh-1a)

### Etape 1: 1-(6-[1,3]dioxolan-2-yl-pyridin-2-yl)-éthanone

On dissout 0.88 g de 2-[1,3]dioxolan-2-yl-pyridine-2-carbonitrile (4.99 mmoles) dans 10 ml de tétrahydrofurane. Dans la solution refroidie à -10°C on introduit goutte à goutte 3.50 ml d'une solution de bromure de méthylmagnesium 3M dans l'éther diéthylique. On agite le mélange réactionnel pendant 3 heures à température ambiante sous atmosphère d'azote. On verse le mélange dans une solution aqueuse saturée de chlorure d'ammonium, on extrait à l'acétate déthyle, la phase organique est lavée avec une solution aqueuse saturée de chlorure d'ammonium puis séchée sur sulfate de magnesium, filtrée et concentrée sous vide. Le produit du titre est isolé par chromatographie sur colonne de silice (éluant: chloroforme). On récupére 0.80 g d'une huile jaune.
Rendement: 83 %
¹H RMN (CDCl₃) **Erreur! Signet non défini.:** 2.72 (s, 3H); 4.15 (m, 4H); 5.89 (s, 1H); 7.70 (dd, 1H); 7.86 (t, 1H); 8.01 (dd, 1H).
IR (film) υ: 1700 cm⁻¹ (C=O).

### Etape 2: 3-diméthylamino-1-(6-[1,3]dioxolan-2-yl-pyridin-2-yl)-propénone

On mélange 0.80 g de 1-(6-[1,3]dioxolan-2-yl-pyridin-2-yl)-éthanone (4.14 mmoles) et 1 ml de N,N-diméthylformamide diméthylacétal (7.53 mmoles). On porte le mélange au reflux pendant 12 heures et on évapore l'excès de N,N-diméthylformamide diméthylacétal sous vide.

On obtient le produit du titre sous forme d'une huile orange qui est utilisée directement dans l'étape suivante sans autre purification.

### Etape 3 : 2-[1,3]dioxolan-2-yl-6-( 1H-pyrazol-3-yl)-pyridine

Le produit brut obtenu dans l'étape 2 est repris dans 5 ml d'éthanol puis on ajoute 0.80 ml d'hydrate hydrazine (25.68 mmoles). On porte la solution au reflux pendant 5 minutes. L'éthanol est évaporé, on reprend le résidu dans l'eau et l'on extrait au chloroforme. La phase organique est lavée à l'eau, séchée au sulfate de magnésium, filtrée, évaporée. Le produit du titre est isolé par chromatographie sur colonne de silice (éluant : chloroforme / méthanol; 98 : 2). On obtient 0.65 g d'une gomme jaune pâle.
Rendement pour l'étape 2 et l'étape 3 combinées: 72.3 %
¹H NMR (CDCl₃) δ: 4.15 (m, 4H); 5.90 (s, 1H); 6.80 (d, 1H); 7.48 (dd, 1H); 7.65 (d, 1H); 7.76 (m, 2H); 11.23 (sl, 1H).

### Etape 4 : 6-(1H-pyrazol-3-yl)-pyridine-2-carbaldéhyde

La déprotection de la fonction acétal du 2-[1,3]dioxolan-2-yl-6-(1H-pyrazol-3-yl)-pyridine est effectuée sur 0.65 g de produit (2.99 mmoles) selon un protocole analogue à celui utilisé dans l'étape 5 de l'exemple 7. Le composé du titre est isolé par chromatographie sur colonne de silice (éluant: chloroforme / méthanol; 98 : 2). On obtient 0.31 g d'une mousse blanche.
Rendement: 59.9 %
¹H RMN (DMSOd6) δ: 6.91 (d, 1H); 7.80 (m, 2H); 8.03 (t, 1H); 8.20 (d, 1H); 9.99 (s, 1H); 13.17 (sl, 1H(échangeable)).
IR (film) υ: 1710 cm⁻¹ (C=O).

### EXEMPLE 11 : Préparation du 6-(1-méthyl-pyrazol-3-yl)-pyridine-2-carbaldéhvde (IVh-2a)

### Etape 1: 2-[1,3]dioxolan-2-yl-6-(1-méthyl-pyrazol-3-yl)-pyridine

Dans une suspension de 0.48 g d'hydrure de sodium (20 mmoles) dans 4 ml de N,N-diméthylformamide, on additionne goutte à goutte 2.50 g de 2-[1,3]dioxolan-2-yl-6-(1H-pyrazol-3-yl)-pyridine (11.5 mmoles) dissout dans 4 ml de N,N-diméthylformamide. On agite le mélange réactionnel pendant 1 heure sous athmosphère d'azote puis on ajoute goutte à goutte dans le mélange réactionnel 0.93 ml d'iodure de méthyle (15 mmoles) dilué dans 1 ml de N,N-diméthylformamide. On agite la solution pendant 12 heures à température ambiante puis on verse le mélange dans l'eau glacée et on extrait à l'acétate d'éthyle. La phase organique est lavée par une solution aqueuse saturée en chlorure de sodium, séchée sur sulfate de magnésium, filtrée, concentrée sous vide. Le produit du titre est isolé par chromatographie sur colonne de silice (éluant : chloroforme / méthanol; 99 : 1). On obtient 0.83 g d'un solide blanc.
Rendement : 31%
F : 88° C
¹H RMN (CDCl₃) δ: 3.99 (s, 3H); 4.11 (m, 4H); 5.90 (s, 1H); 6.91 (d, 1H); 7.36 (d, 1H); 7.42 (dd, 1H); 7.72 (t, 1H); 7.90 (dd, 1H).

### Etape 2: 6-(1-méthyl-pyrazol-3-yl)-pyridine-2-carbaldéhyde

La déprotection de 0.77 g de 2-[1,3]dioxolan-2-yl-6-(1-méthyl-pyrazol-3-yl)-pyridine (3.31 mmoles) selon un protocole analogue à celui utilisé dans l'étape 5 de l'exemple 7, donne 0.48 g du produit du titre (2.51 mmoles) après purification par chromatographie sur colonne de silice (éluant : acétate d'éthyle).
Rendement : 75.8 %
F : 98-100°C
¹H RMN (CDCl₃) δ: 3.98 (s, 3H); 6.93 (d, 1H); 7.42 (d, 1H); 7.84 (m, 2H); 8.09 (dd, 1H); 10.12 (s, 1H);
IR (KBr) υ: 1710 cm⁻¹ (C=O).

### EXEMPLE 12 : Préparation du 6-isopropyl-pyridine-2-carbaldéhyde (IVi)

### Etape 1 : 2-[1,3]dioxolan-2-yl-6-isopropènyl-pyridine

Dans une suspension de 5.25 g bromure de (méthyl)triphénylphosphonium (14.7 mmoles) dans 30 ml de tétrahydrofurane maintenue sous atmosphère d'azote, on ajoute par fractions 1.62 g de tertiobutylate de potassium (14.4 mmoles). On agite le mélange pendant 45 minutes puis on introduit goutte à goutte une solution de 0.95 g 1-(6-[1,3]dioxolan-2-yl-pyridin-2-yl)-éthanone (4.92 mmoles) dans 5 ml de tétrahydrofurane. On agite le mélange pendant 12 heures à température ambiante puis on le verse dans une solution aqueuse saturée en chlorure d'ammonium. On extrait à l'acétate d'éthyle, la phase organique est lavée par une solution aqueuse saturée en chlorure de sodium, séchée sur sulfate de magnésium, filtrée, concentrée sous vide. Le résidu est repris dans l'éther diéthylique et on élimine le précipité formé par filtration. Après évaporation du solvant, le produit du titre est isolé par chromatographie sur colonne de silice (éluant : hexane / acétate d'éthyle; 85 : 15). On obtient 0.67 g d'une huile jaune pâle.
Rendement: 71.6 %
¹H RMN (CDCl₃) δ: 2.22 (s, 3H); 4.16 (m, 4H); 5.31 (m, 1H); 5.87 (s, 1H); 5.91 (m, 1H); 7.44 (m, 2H); 7.70 (t, 1H).

### Etape 2 : 2-[1,3]dioxolan-2-yl-6-isopropyl-pyridine

Une suspension de 0.60 g de 2-[1,3]dioxolan-2-yl-6-isopropényl-pyridine (3.15 mmoles) et 0.10 g de palladium sur charbon à 10 %, dans 7 ml de méthanol est agitée vigoureusement sous faible pression d'hydrogène pendant 4 heures à température ambiante. Le solide est éliminé par filtration sur célite puis on évapore le méthanol. Le produit du titre est isolé par chromatographie sur colonne de silice (éluant : chloroforme / acétate d'éthyle; 97 : 3). On obtient 0.40 g d'une huile jaune pâle.
Rendement: 66 %
¹H RMN (CDCl₃) δ: 1.30 (d, 6H); 3.07 (m, 1H); 4.13 (m, 4H); 5.83 (s, 1H); 7.17 (d, 1H); 7.36 (d, 1H); 7.66 (t, 1H).

### Etape 3 : 6-isopropyl-pyridine-2-carbaldéhyde

La déprotection de 0.36 g de 2-[1,3]dioxolan-2-yl-6-isopropyl-pyridine (1.87 mmole) selon un protocole similaire à celui utilisé dans l'étape 5 de l'exemple 7 donne 0.31 g du produit du titre (1.81 mmole) après purification par chromatographie sur colonne de silice (éluant : chloroforme).
Rendement: 96.8 %
¹H RMN (CDCl₃) δ. 1.34 (d, 6H); 3.10 (m, 1H); 7.37 (m, 1H); 7.73 (d, 2H); 10.03 (s, 1H).
IR (film) υ: 1712 cm⁻¹ (C=O).

### EXEMPLE 13 : Préparation du 6-oxazol-5-yl-pyridine-2-carbaldéhyde (IVk)

### Etape 1 : 2-[1,3]dioxolan-2-yl-6-oxazol-5-yl-pyridine

On mélange 1 g de 6-[1,3]dioxolan-2-yl-pyridine-2-carbaldéhyde (5.58 mmoles), 1.10 g de tosylméthylisocyanate (5.63 mmoles) et de 0.80 g de carbonate de potassium (5.79 mmoles) dans 15 ml de méthanol. La suspension est portée au reflux pendant 2 heures. On évapore le méthanol, on reprend le résidu par une solution aqueuse saturée en chlorure de sodium et on extrait par de l'acétate d'éthyle. La phase organique est séchée sur sulfate de magnésium, filtrée puis concentrée sous vide. Le produit du titre est isolé par chromatographie sur colonne de silice (éluant : hexane / acétate d'éthyle; 50 : 50). On obtient 1.14 g d'une huile jaune.
Rendement: 93.6 %
¹H RMN (CDCl₃) δ: 4.13 (m, 4H); 5.86 (s, 1H); 7.48 (d, 1H), 7.63 (d, 1H); 7.77 (m, 2H); 7.95 (s, 1H).

### Etape 2 : 6-oxazol-5-yl-pyridine-2-carbaldéhyde

La déprotection de 0.95 g de 2-[1,3]dioxolan-2-yl-6-oxazol-5yl-pyridine (4.35 mmoles) selon un protocole analogue à celui utilisé dans l'étape 5 de l'exemple 7 donne 0.32 g du produit du titre après purification par chromatographie sur colonne de silice (éluant : chloroforme /acétate d'éthyle; 80 :20).
Rendement: 42.3 %
F: 151°C
¹H RMN (CDCl₃) δ: 7.81-8.01 (m, 5H); 10.08 (s, 1H).
IR (KBr) υ: 1703 cm⁻¹ (C=O).

### EXEMPLE 14 : Préparation du 6-cyclopropyl-pyridine-2-carbaldéhyde (IVI)

### Etape 1 : 2-[1,3]dioxolan-2-yl-vinyl-pyridine

Un mélange de 3.10 g de 6-[1,3]dioxolan-2-yl-pyridine-2-carbaldéhyde (17.3 mmoles), 9.26 g de bromure de (méthyl)triphénylphosphonium (25.9 mmoles) et de 4.80 g de carbonate de potassium (34.8 mmoles) dans 60 ml de 1,4-dioxane, est maintenu au reflux pendant 5 heures. Le solide est éliminé par filtration puis on évapore le solvant. Le résidu est repris dans l'acétate d'éthyle, la solution est lavée à l'eau puis avec une solution aqueuse saturée en chlorure de sodium, séchée sur sulfate de magnésium, filtrée. Après évaporation du solvant, on isole le composé du titre par chromatographie sur colonne de silice (éluant : hexane / acétate d'éthyle ; 80 : 20). On obtient 1.63 g d'une huile jaune.
Rendement: 53.2 %
¹H RMN (CDCl₃) δ: 5.47 (dd, 1H); 5.83 (s, 1H); 6.18 (dd, 1H); 6.83 (dd, 1H); 7.36 (m, 2H); 7.67 (t, 1H).

### Etape 2 : 2-cyclopropyl-6-[1,3]dioxolan-2-yl-pyridine

Dans une suspension de 2.71 g d'iodure de triméthylsulfonium (13.3 mmoles) dans 25 ml de tétrahydrofurane refroidie à -15°C, on introduit goutte à goutte 5.60 ml d'une solution de n-butyllilhium 1.6 M dans l'hexane. On agite la solution à -15°C pendant 20 minutes sous azote, puis on ajoute goutte à goutte un solution de 1.57 g de 2-[1,3]dioxolan-2-yl-6-vinyl-pyridine (8.90 mmoles) dans 5 ml de tétrahydrofurane. Après 1 heure d'agitation à -15°C, la suspension est agitée 3 heures à température ambiante. Le tétrahydrofurane est évaporé, on reprend le résidu dans l'eau et on extrait à l'acétate d'éthyle. La phase organique est lavée par une solution aqueuse saturée en chlorure de sodium, séchée sur sulfate de magnésium, filtrée, concentrée sous vide. Le produit du titre est isolé par chromatographie sur colonne de silice (éluant : hexane / acétate d'éthyle; 50 : 50). On obtient 1.24 g d'une huile incolore.
Rendement : 73 %
¹H RMN (CDCl₃) δ: 0.98 (m, 4H); 2.06 (m, 1H); 4.09 (m, 4H); 5.76 (s, 1H); 7.03 (dd, 1H); 7.25 (dd, 1H); 7.55 (t, 1H).

### Etape 3: 6-cyclopropyl-pyridine-2-carbaldéhyde

La déprotection de 1.14 g de 2-cyclopropyl-6-[1,3]dioxolan-2-yl-pyridine (5.96 mmoles) selon un protocole similaire à celui utilisé dans l'étape 5 de l'exemple 7, donne 0.75 g du produit du titre après purification par chromatographie sur colonne de silice (éluant : hexane / acétate d'éthyle; 90 : 10).
Rendement: 85 %
¹H RMN (CDCl₃) δ: 1.09 (m, 4H); 2.10 (m, 1H); 7.33 (m, 1H); 7.68 (m, 2H); 9.96 (s, 1H).
IR (film) υ: 1713 cm⁻¹ (C=O).

### EXEMPLE 15 : Préparation du 6-(1-fluoro-éthyl)-pyridine-2-carbaldéhyde (IVj)

### Etape 1: 1-(6-[1,3]dioxolan-2-yl-pyridin-2-yl)-éthanol

Dans une solution de 0.90 g de 1-(6-[1,3]dioxolan-2-yl-pyridin-2-yl)-éthanone (4.66 mmoles) dans 15 ml de méthanol, on ajoute par fractions, 0.50 g de borohydrure de potassium (9.27 mmoles) On agite 12 heures à température ambiante puis on évapore le méthanol et on reprend le résidu dans du chloroforme. La phase organique est lavée à l'eau puis séchée sur sulfate de sodium, filtrée, concentrée sous vide. On obtient 0.70 g d'une huile jaune pâle, utilisée dans l'étape suivante sans autre purification.
¹H RMN (CDCl₃) δ: 1.47 (d, 3H); 1.84 (s, 1H(échangeable)); 4.11 (m, 4H); 4.88 (m, 1H); 5.82 (s, 1H); 7.25 (d, 1H); 7.41 (d, 1H); 7.75 (t, 1H).

### Etape 2: 2-[1,3]dioxolan-2-yl-6-(1-fluoro-éthyl)-pyridine

Dans une solution de 0.60 g de 1-(6-[1,3]dioxolan-2-yl-pyridin-2-yl)-éthanol (3.07 mmoles) dans 25 ml de dichlorométhane refroidie à -78°C et maintenue sous une atmosphère d'azote, on additionne 0.81 ml de trifluorosulfure de diéthylamine (6.14 mmoles). On agite 30 minutes à -78°C puis 2 heures à température ambiante. On verse lentement le mélange dans une solution aqueuse de bicarbonate de sodium à 10 %. La phase organique est séchée sur sulfate de magnésium, filtrée et le solvant est évaporé. Le produit du titre est isolé par chromatographie sur colonne de silice (éluant : chloroforme). On obtient 0.42 g d'une huile jaune.
Rendement pour les 2 étapes combinées: 53.4 %
¹H RMN (CDCl₃) δ: 1.69 (dd, 3H); 4.11 (m, 4H); 5.77 (dq, 1H); 5.83 (s, 1H); 7.48 (d, 2H); 7.79 (t, 1H).

### Etape 3: 6-(1-fluoro-éthyl)-pyridine-2-carbaldéhyde

La déprotection de 0.40 g de 2-[1,3]dioxolan-2-yl-6-(1-fluoro-éthyl)-pyridine (2 mmoles) selon un protocole analogue à celui utilisé dans l'étape 5 de l'exemple 7, donne 0.32 g du produit du titre sous la forme d'une huile jaune utilisée dans l'étape suivante sans autre purification.
¹H RMN (CDCl₃) δ: 1.72 (dd, 3H); 5.75 (dq, 1H); 7.70 (dd, 1H); 7.90 (m, 2H); 10.03 (s, 1H).
IR (film) υ: 1715 cm⁻¹ (C=O).

### EXEMPLE 16 : Préparation du 6-(1-fluoro-méthyl)-pyridine-2-carbaldéhyde (IVm-1)

En procédant comme dans l'exemple 15 mais en remplaçant le 1-(6-[1,3]dioxolan-2-yl-pyridin-2-yl)-éthanol par le 1-(6-[1,3]dioxolan-2yl-pyridin-2-yl)-méthanol dans l'étape 2, on obtient le composé du titre sous forme d'un solide jaune.
F: 60°C
¹H RMN (CDCl₃) δ: 5.55 (d, 2H, J = 47 Hz); 7.68 (dd, 1H); 7.90 (m, 2H); 15.35 (s, 1H).
IR (KBr) υ: 1721 cm⁻¹ (C=O).

### EXEMPLE 17 : Préparation du 6-(1,1-difluoro-méthyl)-pyridine-2-carbaldéhyde (IVm-2)

### Etape 1 : 2-[1,3]dioxolan-2-yl-6-(1,1-difluoro-méthyl)-pyridine

Dans une solution de 2.40 g de 6-[1,3]dioxolan-2-yl-pyridine-2-carbaidéhyde (13.4 mmoles) dans 20 ml de chloroforme, on ajoute goutte à goutte 2.21 ml de trifluorosulfure de diéthylamine. On agite à température ambiante sous azote pendant 12 heures puis le mélange est versé dans une solution glacée de bicarbonate de sodium à 20 %. La phase organique est séchée sur sulfate de magnésium, filtrée, concentrée sous vide. Le produit du titre est isolé par chromatographie sur colonne de silice (éluant : chloroforme). On obtient 1.60 g d'une huile jaune.
Rendement: 59.3 %
¹HRMN (CDCI₃) δ: 4.11 (m, 4H); 5.85 (s, 1H); 6.65 (t, 1H); 7.63 (m, 2H); 7.88 (t, 1H).

### Etape 2 : 6-(1,1-difluoro-méthyl)-pyridine-2-carbaldéhyde

La déprotection de 0.85 g de 2-[1,3]dioxolan-2-yl-6-(1,1-difluoro-méthyl)-pyridine (4.20 mmoles) selon un protocole similaire à celui utilisé dans l'étape 5 de l'exemple 7, donne 0.70 g du produit sous forme d'une huile jaune, utilisée dans l'étape suivante sans autre purification.
¹HRMN (CDCl₃)δ: 6.69 (t, 1H); 7.85 (m, 1H); 8.03 (m, 2H); 10.05 (s, 1H).
IR (film) υ: 1721 cm⁻¹ (C=O).

### EXEMPLE 18 : Préparation du 6-formyl-pyridine-2-carboxvlic acid méthyl ester (IVo)

Une solution de 3 g de 6-hydroxym éthyl-pyridine-2-carboxylic acid méthyl ester (16.5 mmoles) dans 70 ml de 1,2-dichloroéthane contenant 15 g de dioxyde de manganèse (165 mmoles) est chauffée au reflux pendant 4 heures avec élimination de l'eau formée en continu. Le solide est éliminé par filtration sur célite puis le dichlorométhane évaporé. Le produit du titre est isolé par chromatographie sur colonne de silice (éluant : dichlorométhane /acétate d'éthyle; 70 : 30). On récupère 2.33 g d'une huile jaune.
Rendement: 79 %
¹HRMN (CDCl₃) δ: 1.36 (t, 3H); 4.41 (m, 2H); 8.13 (dd, 1H); 8.24 (t, 1H); 8.32 (dd, 1H); 10.02 (s, 1H).
IR (film) υ: 1700 cm⁻¹ (C=O).

### EXEMPLE 18a : Préparation du 5-méthyl -6-méthylamino-pyridine-2-carbaldéhyde (IVs-1)

### Etape 1 : (6-chloro-5-méthyl-pyridin-2-yl)-méthanol

Dans une solution de 1.20 g de 6-chloro-5-méthyl-pyridine-2-carboxilic acid éthyl ester (6.00 mmoles) et 10 ml d'éthanol maintenue à température ambiante, on ajoute par fractions 0.40 g de borohydrure de sodium (10.5 mmoles). On agite pendant 4 heures puis le mélange est versé dans une solution aqueuse de chlorure de sodium et extrait à l'acétate d'éthyle. La phase organique est séchée sur sulfate de sodium, filtrée concentrée sous vide. Le produit du titre est isolé par chromatographie sur colonne de silice (éluant : chloroforme). On récupère 0.69 g d'une huile incolore.
Rendement: 73 %
¹HR MN (CDCI₃) δ: 2.37 (s, 3H); 4.70 (s, 2H); 2.94 (s; large); 7.17 (d, 1H); 7.55 (d, 1H)

### Etape 2 : (5-méthyl-6-méthylamino-pyridin-2-yl)-méthanol

0.96 g de (6-chloro-5-méthyl-pyridin-2-yl)-méthanol (6.09 mmoles) et 7 ml de méthylamine à 33% dans l'éthanol sont chauffés à 120°C dans une bombe pendant 96 heures. 2 ml de méthylamine à 33% dans l'éthanol sont ajoutés toute les 24 heures. Le mélange réactionnel est concentré sous vide et le composé du titre isolé par chromtographie sur colonne de silice (éluant: cyclohexane / acétate d'éthyle; 40 : 60).
On récupère 0.34 g d'un solide blanc.
Rendement: 37 %
F: 87°C
¹H RMN (CDCl₃) δ: 2.06 (s, 3H); 3.05 (d; 3H); 4.13 (s, 1H); 4.24 (s, 1H); 4.59 (d, 2H); 6.39 (d, 1H); 7.18 (d, 1H)

### Etape 3 : 5-méthyl-6-méthylamino-pyridine-2-carbaldéhyde

Une suspension de 0.33 g de (5-méthyl-6-méthylamino-pyridin-2-yl)-méthanol (2.17 mmoles), 1.6 g de dioxyde de manganèse (18.4 mmoles) dans 12 ml de chloroforme est chauffée au reflux pendant 1 heure. Les insolubles sont éliminés par filtration sur célite puis le solvant évaporé sous vide. Le produit du titre est purifié par chromatographie sur colonne de silice (éluant : chloroforme). On récupère 0.25 g d'un solide jaune.
Rendement: 77 %
F: 79°C
¹H RMN (CDCl₃) δ: 2.15 (s, 3H); 3.12 (d, 3H); 4.36 (s, 1H); 7.22 (d, 1H), 7.35 (d, 1H); 9.93 (s, 1H)

### EXEMPLE 18b : Préparation du 5-méthyl-6-diméthylamino-pyridine-2-carbaldéhyde (IVs-2)

En procédant comme dans l'exemple 18a mais en remplaçant dans l'étape 2 la méthylamine à 33 % dans l'éthanol par de la diméthylamine à 33 % dans l'éthanol, on prépare le composé du titre obtenu sous forme d'une huile jaune.
¹H RMN (CDCl₃) δ: 2.37 (s, 3H); 2.93 (s, 6H); 7.45 (d, 1H); 7.50 (d, 1H); 9.93 (s, 1H)

### EXEMPLE 18c : Préparation du 3-méthyl-6-diméthylamino-pyridine-2-carbaldéhvde (IVs-3)

Dans une solution de 0.53 ml de N,N,N'-triméthyléthylène diamine (4.24 mmoles) et 10 ml de tétrahydrofurane refroidie à -60°C, on introduit 2.65 ml d'une solution de n-butyllithium 1.6M dans l'hexane. On agite la solution pendant 15 minutes à -40°C puis on la refroidie à -70°C et on introduit goutte à goutte une solution de 0.50 g de 6-chloro-pyridine-2-carbaldéhyde (3.53 mmoles) et 4 ml de tétrahydrofurane. La solution orange est agitée pendant 30 minutes à - 70°C puis on introduit 1.28 ml de tétraméthyléthylènediamine (8.48 mmoles) puis après 10 minutes 5.30 ml d'une solution de n-butylithium 1.6M dans l'hexane. La solution brune est agitée pendant 2 heures à -78°C puis on ajoute goutte à goutte 1.50 ml d'iodure de méthyle (25 mmoles). Après une heure d'agitation à -78°C puis 10 minutes à 20°C le mélange réactionnel est versé dans une solution acqueuse saturée en chlorure de sodium et extrait à l'acétate d'éthyle. La phase organique est lavée avec une solution aqueuse saturée en chlorure de sodium, séchée sur sulfate de magnésium, filtrée et le solvant évaporé. On récupère 0.23 g du produit du titre après purification par chromatographie sur colonne de silice (éluant: Hexane / acétate d'éthyle; 95 : 5 ).
Rendement: 41.9 %
¹H RMN (CDCl₃) δ: 2.64 (s, 1H); 7.42 (d, 1H); 7.61 (d, 1H); 10.09 (s, 1H)
IR (film) υ: 1710 cm⁻¹ (C=O).

### EXEMPLE 19 : Préparation du (4-aminométhyl-4-fluoro-pipéridin-1-yl)-(3-chloro-4-fluoro-phényl)-méthanone (V-I)

### Etape 1 : (4-fluoro-4-hydroxyméthyl-pipéridin-l-yl)-(3-chloro-4-fluoro-phényl)-méthanone

On dissout 11.9 g de (1-oxa-6-aza-spiro[2.5]oct-6-yl)-(3-chloro-4-fluoro-phényl)-méthanone (44.12 mmoles) dans 20 ml de dichlorométhane. Dans la solution refroidie à 0°C et maintenue sous azote, on introduit 13 ml de complexe HF-pyridine (441 mmoles) On agite la solution pendant 12 heures à température ambiante puis on verse le mélange dans l'eau glacée. Le milieu est basifté par addition de carbonate de potassium puis extrait au chloroforme. La phase organique est lavée par une solution aqueuse d'acide chlorhydrique 1N, séchée sur sulfate de magnésium, filtrée et concentrée sous vide. Le produit du titre est purifié par recristallisation dans un mélange éthanol / acétate d'éthyle. On obtient 6.40 g d'une poudre cristalline blanche.
Rendement: 50 %
F: 188-90°C

| Analyse C₁₃H₁₄ClF₂ NO₂: | | | | |
|---|---|---|---|---|
| Calc. % | C 53.90 | H 4.87 | Cl 12.24 | N 4.83 |
| Tr. | 54.08 | 4.86 | 12.28 | 4.70 |

¹H RMN (CDCl₃) δ: 1.35-2.15 (m, 4H); 1.63 (s, 1H (échangeable)); 2.95-3.50 (m, 3H); 3.64 (d, 2H); 4.29-4.70 (m, 1H); 7.12-7.32 (m, 2H); 7.47 (dd, 1H).
IR (KBr) υ: 1612 cm⁻¹ (C=O); 3328 cm⁻¹ (O-H).

### Etape 2 : toluène-4-sulfonic acid 1-(3-chloro-4-fluoro-benzoyl)-4-fluoro-pipéridin-4-yl-méthyl ester

Dans une solution de 6.70 g de (4-fluoro-4-hydroxyméthyl-pipéridin-1-yl)-(3-chloro-4-fluoro-phényl)-méthanone (23.12 mmoles) dans 40 ml de pyridine refroidie à 0°C et maintenue sous atmosphère d'azote, on ajoute lentement 4.87 g de chlorure de paratoluène sulfonyle (25.5 mmoles). On agite pendant 12 heures à température ambiante puis le mélange est versé dans l'eau glacée, on extrait par du chloroforme puis on lave la phase organique par une solution aqueuse d'acide chlorhydrique 1N. La phase organique est séchée sur sulfate de magnésium, filtrée puis concentrée sous vide. Le produit du titre est purifié par recristallisation dans un mélange dichlorométhane / éther diisopropylique. On obtient 7.73 g d'une poudre cristalline blanche.
Rendement: 76 %
F: 92°C
¹H RMN (CDCl₃) δ: 1.35-1.95 (m, 4H); 2.43 (s, 3H); 2.95-3.90 (m, 2H); 3.60 (dd, 1H); 3.97 (d, 2H); 4.25-4.70 (m, 1H); 7.15 (t, 1H); 7.25 (m, 2H); 7.34 (d, 1H); 7.44 (dd, 1H); 7.76 (d, 2H).

### Etape 3 : 2-[1-(chloro-4-fluoro-benzoyl)-4-fluoro-pipéridin-4-yl-méthyl]-isoindole-1,3-dione.

Un mélange de 5.42 g de toluène-4-sulfonic acid 1-(3-chloro-4-fluoro-benzoyl)-4-fluoro-pipéridin-4-yl méthyl ester (12.2 mmoles) et de 2.96 g de phtalimide de potassium (16 mmoles) dans 70 ml de N,N-diméthylformamide est chauffé à 150 °C pendant 5 heures.

Le mélange est versé dans l'eau glacée puis on extrait au chloroforme. Après évaporation des solvants sous vide, le produit du titre est isolé par chromatographie sur colonne de silice (éluant: chloroforme / acétate d'éthyle; 90 : 10). On obtient 4.02 g d'une poudre cristalline blanche.
Rendement: 79 %
F: 133-34°C

| Analyse C₂₁H₁₇F₂ClN₂O₃: | | | | |
|---|---|---|---|---|
| Calc. % | C 60.22 | H 4.09 | Cl 8.46 | N 6.69 |
| Tr. | 60.12 | 3.97 | 8.51 | 6.71 |

¹H RMN (CDCl₃) δ: 1.90 (m, 4H); 3.25 (m, 2H); 3.65 (m, 1H); 3.90 (d, 2H); 4.55 (m, 1H); 7.12 (t, 1H); 7.27 (m, 1H); 7.48 (dd, 1H); 7.75 (m, 2H); 7.88 (m, 2H).
IR (KBr) υ: 1716 et 1776 cm⁻¹ (C=O).

### Etape 4 : (4-aminométhyl-4-fluoro-pipéridin-1-yl)-(3-chloro-4-fluoro-phényl)-méthanone

On mélange 0.10 g de 2-[1-(3-chloro-4-fluoro-benzoyl)-4-fluoro-pipéridin-4-ylméthyl]-isoindole-1,3-dione (0.238 mmole) et 0.50 ml d'éthanolamine (8.28 mmoles) puis on porte la solution obtenue à 55°C sous atmosphère d'azote pendant 2 heures. Le mélange est versé dans l'eau glacée puis extrait au chloroforme. La phase organique est lavée avec une solution aqueuse saturée en chlorure de sodium, séchée sur sulfate de magnésium, filtrée et le solvant évaporé. On obtient 0.06 g de produit du titre sous la forme d'une huile incolore, utilisé dans l'étape suivante sans autre purification.
¹H RMN (CDCl₃) δ: 1.21-1.75 (m, 4H dont 2H échangeables); 1.90 (m, 2H); 2.79 (d, 2H); 3.22 (m, 2H); 3.62 (m, 1H); 4.49 (m, 1H); 7.14 (t, 1H); 7.25 (m, 1H); 7.44 (dd, 1H).

### EXEMPLE 20 : Préparation du (4-aminométhyl-4-fluoro-pipéridin-1-yl)-(3,4-dichloro-phényl)-méthanone (V-2)

En procédant comme dans l'exemple 19 mais en remplaçant dans l'étape 1 la (1-oxa-6-aza-spiro[2.5]oct-6-yl)-(3-chloro-4-fluoro-phényl)-méthanone par la (1-oxa-6-aza-spiro[2.5]oct-6-yl)-(3,4-dichloro-phényl)-méthanone, dans l'étape 2 la (4-fluoro-4-hydroxyméthyl-pipéridin-1-yl)-(3-chloro-4-fluoro-phényl)-méthanone par la (4-fluoro-4-hydroxyméthyl-pipéridin-1-yl)-(3,4-dichloro-phényl)-méthanone, dans l'étape 3 le toluène-4-sulfonic acid 1-(3-chloro-4-fluoro-benzoyl)-4-fluoro-pipéridin-4-yl méthyl ester par le toluène-4-sulfonic acid 1-(3,4-dichloro-benzoyl)-4-fluoro-pipéridin-4-yl- méthyl ester et dans l'étape 4 la 2-[1-(3-chloro-4-fluoro-benzoyl)-4-fluoro-pipéridin-4-ylméthyl]-isoindole-1,3-dione par la 2-[1-(3,4-dichloro-benzoyl)-4-fluoro-pipéridin-4-yl-méthyl]-isoindole-1,3-dione, on obtient le composé du titre sous forme d'une huile jaune.
¹HRMN (CDCI₃) δ : 1.24-1.80 (m, 4H dont 2H échangeables); 1.94 (m, 2H); 2.82 (d, 2H); 3.28 (m, 2H); 3.59 (m, 1H); 4.52 (m, 1H); 7.26 (m, 1H); 7.48 (m, 2H).

### EXEMPLE 20a : Préparation du (4-aminométhyl-4-fluoro-pipéridin-1-yl)-(3-chloro-4-méthyl- phényl)-méthanone (V-3)

En procédant comme dans l'exemple 19 mais en utilisant comme produit de départ la (1-oxa-6-aza-spiro [2,5]oct-6-yl)-(3-chloro-4-méthyl-phényl)-méthanone, on obtient le composé du titre sous forme d'une huile jaune.
¹H RMN (CDCl₃) δ: 1.23 (m, 2H); 1.59 (m, 2H); 1.95 (m, 2H); 2.40 (s, 2H); 2.87 (d, 2H); 3.14 (m, 1H): 3.37 (m, 1H); 3.60 (m, 1H); 4.56 (m, 1H); 7.21 (dd, 1H); 7.27 (d, 1H); 7.39 (d, 1H)

### EXEMPLE 21 : Préparation du (4-azidométhyl-4-fluoro-pipéridin-1-yl)-(3-chloro-4-fluoro-phényl)-méthanone (VI-1)

On mélange 0.70 g de toluène-4-sulfonic acid 1-(3-chloro-4-fluoro-benzoyl)-4-fluoro-pipéridin-4-ylméthyl ester (1.57 mmole), 0.308 g d'azoture de sodium (4.70 mmoles) et 0.20 g d'azoture de tétrabutylammonium (0.70 mmole) dans 3.50 ml de diméthylsulfoxyde. On porte le mélange à 110 °C sous atmosphère d'azote pendant 20 heures. On verse le mélange réactionnel dans l'eau glacée puis on extrait à l'acétate d'éthyle. La phase organique est lavée à l'eau puis avec une solution aqueuse saturée en chlorure de sodium, séchée sur sulfate de magnésium, filtrée et concentrée sous vide. Le produit du titre est isolé par chromatographie sur colonne d'alumine neutre (éluant : hexane / acétate d'éthyle; 70 : 30); On obtient 0.313 g d'une huile jaune.
Rendement : 63 %
¹HRMN (CDCI₃) δ: 1.63 (m, 2H); 1.98 (m, 2H); 3.25 (m, 2H); 3.36 (d, 2H); 3.66 (m, 1H); 4.54 (m, 1H); 7.14-7.50 (m, 3H).
IR (film) υ : 1635 cm⁻¹ (C=O); 2102 cm-1 (N₃).

### EXEMPLE 22 : Préparation du (4-azidométhyl-4-fluoro-pipéridin-1-yl)-(3,4-dichloro-phényl)-méthanone (VI-2)

De façon analogue à l'exemple 21 mais en partant du toluène-4-sulfonic acid 1-(3,4-dichloro-benzoyl)-4-fluoro-pipéridin-4-ylméthyl ester à la place du toluène-4-sulfonic acid 1- (3-chloro-4-fluoro-benzoyl)-4-fluoro-pipéridin-4-ylméthyl ester, on obtient le produit du titre sous forme d'une huile jaune.
¹H RMN (CDCl₃) δ: 1.55 (m, 2H); 1.96 (m, 2H); 3.20 (m, 2H); 3.33 (d, 2H); 3.60 (m, 1H); 4.54 (m, 1H); 7.20 (dd, 1H); 7.47 (m, 2H).
IR (film) υ: 1638 cm⁻¹ (C=O); 2102 cm⁻¹ (N₃).

### EXEMPLE 23: Préparation du (3,4-dichloro-phényl)-(4-{[(6-fluoro-pyridin-2-ylméthyl)-amino]-méthyl}-pipéridin-1-yl)-méthanone (I-51)

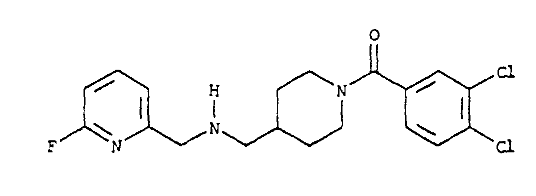

On mélange 0.29 g de 6-fluoro-pyridine-2-carbaldéhyde (2.31 mmoles) et 0.264 g de pipéridin-4-ylméthylamine (2.31 mmoles) dans 20 ml de benzène. On porte la solution au reflux sous atmosphère d'azote pendant 2 heures avec élimination de l'eau formée en continu. On évapore le solvant puis on reprend le résidu dans 2 ml de tétrahydrofurane. La solution obtenue est refroidie à 0°C, on additionne successivement 0.50 ml de triéthylamine (3.50 mmoles) puis goutte à goutte 0.469 g de chlorure de 3,4-dichlorobenzoyle (2.24 mmoles) dilué dans 1 ml de tétrahydrofurane. On agite pendant 2 heures à température ambiante. On ajoute 10 ml de méthanol puis par fractions, 0.25 g de borohydrure de potassium (4.62 mmoles). Après 4 heures à température ambiante, les solvants sont évaporés, le résidu est repris dans l'eau et extrait au chloroforme. La phase organique est lavée à l'eau, séchée sur sulfate de magnésium, filtrée, concentrée sous vide. Le produit du titre est isolé par chromatographie sur colonne de silice (éluant: chloroforme / méthanol; 95 : 5). On obtient 0.40 g d'un solide jaune.
Rendement: 40 %
F: 78°C
¹H RMN (CDCl₃) δ: 1.18 (m, 2H); 1.77 (m, 3H); 1.99 (s, 1H (échangeable)); 2.52 (d, 2H); 2.86 (m, 2H); 3.66 (m, 1H); 3.82 (s, 2 H); 4.67 (m, 1H); 6.78 (dd, 1H); 7.22 (m, 2H);7.46 (m, 2H); 7.72 (q, 1H).

### EXEMPLE 24 : Préparation du (4-{[(6-azétidin-1-yl-pyridin-2-ylméthyl)-amino]-méthyl}-pipéridin-1yl)-3,4-dichloro-phényl)-méthanone (I-17)

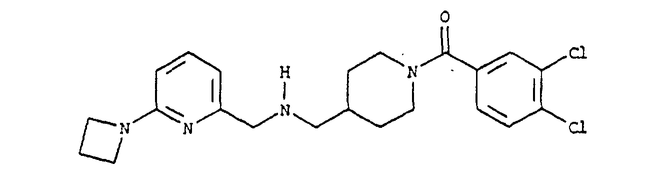

Dans une solution de 0.70 g de (3,4-dichloro-phényl)-(4-{[(6-fluoro-pyridine-2-ylméthyl)-amino]-méthyl}-pipéridin-1-yl)-méthanone (1.77 mmoles) dans 10 ml de tétrahydrofurane on ajoute 0.121 g d'azétidine (3.15 mmoles). On porte la solution à 100°C pendant 32 heures. Le solvant est évaporé sous vide puis le résidu est repris dans l'eau, extrait au chloroforme. La phase organique est lavée à l'eau puis avec une solution saturée en chlorure de sodium, séchée sur sulfate de magnésium, filtrée et concentrée sous vide. Le produit du titre est isolé par chromatographie sur colonne de silice (éluant: chloroforme / méthanol; 90 : 10). On obtient 0.401 g d'une huile jaune.
Rendement : 52.3 %
¹H RMN (CDCl₃) δ: 1.22 (m, 2H); 1.78 (m, 3H); 2.13 (s, 1H (échangeable); 2.34 (m, 2H); 2.51 (d, 2H); 2.75 (m, 1H); 3.01 (m, 1H); 3.60 (m, 1H); 3.69 (s, 2H); 3.97 (t, 4H); 4.61 (m, 1H); 6.10 (d, 1H); 6.48 (d, 1H); 7.18 (dd, 1H); 7.34 (dd, 1H); 7.42 (m, 2H).

On dissout 0.34 g du produit du titre (0.784 mmole) dans 1 ml d'éthanol puis on ajoute 0.067 g d'acide oxalique (0.745 mmole). Après dissolution, le sel est précipité par addition d'acétate d'éthyle, filtré, lavé à l'acétate d'éthyle puis séché à 50 °C sous vide. On obtient 0.290 g de l'oxalate du composé du titre sous la forme d'une poudre cristalline blanche.
F: 220-21°C

| Analyse C₂₄H₂₈Cl₂N₄O₅: | | | | |
|---|---|---|---|---|
| Calc. % | C 55.07 | H 5.39 | Cl 13.55 | N 10.70 |
| Tr. | 54.87 | 5.41 | 13.29 | 10.63 |

¹H RMN (DMSOd6) δ: 1.19 (m, 2H); 1.80 (m, 2H); 2.00 (m, 1H); 3.35 (m, 2H); 2.75 (m, 1H); 2.88 (d, 2H); 3.04 (m, 1H); 3.49 (m, 1H); 3.95 (t, 4H); 4.09 (s, 2H); 4.39 (m, 1H); 6.31 (d, 1H); 6.68 (d, 1H); 7.35 (dd, 1H); 7.53 (t, 1H); 7.65 (d, 1H); 7.70 (d, 1H).
IR (KBr) υ:1632 et 1710 cm⁻¹ (C=O).

### EXEMPLE 25 : Préparation du (3-chloro-4-fluoro-phényl)-(4-fluoro-4-{[(6-furan-2-yl-pyridin-2-ylméthyl)-amino]-méthyl}-pipéridin-1-yl)-méthanone (I-32)

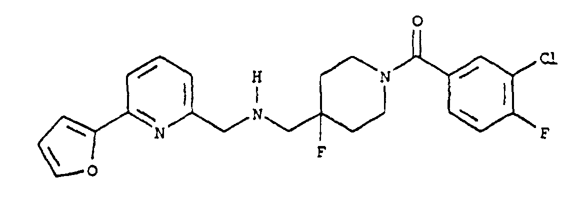

On mélange 0.687 g de (4-aminométhyl-4-fluoro-pipéridin-1-yl)-(3-chloro-4-fluoro-phényl)-méthanone (2.38 mmoles) et 0.371 g de 6-furan-2-yl-pyridine-2-carbaldéhyde (2.38 mmoles) dans 25 ml de toluène. La solution est chauffée au reflux sous atmosphère d'azote pendant 2 heures en éliminant l'eau formée en continu. Le toluène est évaporé, le résidu est repris dans 25 ml de méthanol puis on ajoute par fractions, 0.257 g de borohydrure de potassium (4.51 mmoles). On agite le mélange réactionnel pendant 5 heures à température ambiante puis on évapore le méthanol. On reprend le résidu dans le chloroforme, la phase organique est lavée à l'eau puis avec une solution aqueuse saturée en chlorure de sodium, séchée sur sulfate de magnésium, filtrée et concentrée sous vide. Le produit du titre est isolé par chromatographie sur colonne de silice, (éluant : chloroforme / méthanol; 98 : 2). On obtient 0.65 g d'une huile jaune.
Rendement: 61.2 %
¹H RMN (CDCl₃) δ: 1.40-2.20 (m, 5H dont 1H échangeable); 2.81 (d, 2H); 3.28 (m, 2H); 3.57 (m, 1H); 3.94 (s, 2H); 4.48 (m, 1H); 6.51 (q, 1H); 7.02 (d, 1H); 7.19 (m, 4H); 7.55 (m, 3H).

On dissout 0.620 g du produit du titre (1.39 mmole) dans 20 ml d'éthanol puis on ajoute 0.160 g d'acide fumarique (1.38 mmole). On concentre la solution, le sel est précipité par addition d'acétate d'éthyle, filtré, lavé à l'acétate d'éthyle puis séchée sous vide à 50 °C. On obtient 0.520 g du fumarate du composé du titre sous la forme d'une poudre cristalline blanche.
F: 158°C

| Analyse C₂₇H₂₆ClF₂N₃O₆: | | | | |
|---|---|---|---|---|
| Calc. % | C 57.71 | H 4.66 | Cl 6.31 | N 7.48 |
| Tr. | 57.96 | 4.70 | 6.31 | 7.45 |

¹H RMN (DMSOd6) δ: 1.72 (m, 1H); 1.85 (m, 3H); 2.81 (d, 2H); 2.95-3.55 (m, 3H); 3.91 (s, 2H); 4.25 (m, 1H); 6.60 (s, 2H); 6.64 (q, 1H); 7.09 (d, 1H); 7.34 (d, 1H); 7.47 (m, 2H); 7.62 (m, 2H); 7.82 (t, 2H).
IR (KBr) υ: 1621 et 1701 cm⁻¹ (C=O).

### EXEMPLE 26 : Préparation du (3-chloro-4-fluoro-phényl)-(4-fluoro-4-{[(6-diméthylamino-pyridin-2-ylméthyl)-amino]-méthyl}-pipéridin-1-yl)-méthanone (1-13)

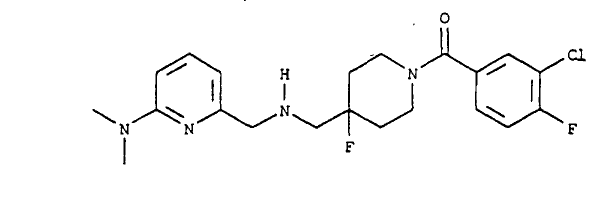

En procédant comme dans l'exemple 25 mais en remplaçant le 6-furan-2-yl-pyridine-2-carbaldéhyde par le 6-diméthylamino-pyridine-2-carbaldéhyde, on obtient le composé du titre sous la forme d'une huile jaune après purification sur colonne de silice, (éluant: chloroforme /méthanol ; 97 : 3).
Rendement: 69.5 %
¹H RMN (CDCl₃) δ: 1.57 (m, 1H); 1.73 (m, 1H); 2.00 (m, 2H); 2.15 (s, 1H (échangeable); 2.75 (d, 2H); 3.04 (s, 6H); 3.25 (m, 2H); 3.55 (m, 1H); 3.72 (s, 2H); 4.62 (m, 1H); 6.34 (d, 1H); 6.43 (d, 1H); 7.14 (t, 1H); 7.27 (m, 1H); 7.35 (dd, 1H); 7.46 (dd, 1H).

On dissout 0.650 g du produit du titre (1.53 mmole) dans 20 ml d'éthanol puis on ajoute 0.170 g d'acide fumarique (1.46 mmole). On concentre la solution, le sel est précipité par addition d'acétate d'éthyle, filtré, lavé à l'acétate d'éthyle puis séchée sous vide à 50°C. On obtient 0.560 g du fumarate du composé du titre sous la forme d'une poudre cristalline blanche.
F: 159°C

| Analyse C₂₅H₂₉ClF₂N₄O₅: | | | | |
|---|---|---|---|---|
| Calc. % | C 55.71 | H 5.42 | Cl 6.58 | N 10.40 |
| Tr. | 55.87 | 5.39 | 6.52 | 10.38 |

¹H RMN (DMSOd6) δ: 1.65 (m, 1H); 1.85 (m, 3H); 2.82 (d, 2H); 3.00 (s, 6H); 3.10-3.60 (m, 3H); 3.75 (s, 2H); 4.25 (m, 1H); 6.50 (d, 1H); 6.59 (d, 1H); 6.60 (s, 2H); 7.46 (m, 3H); 7.68 (dd, 1H).
IR (KBr) υ: 1637, 1686 et 1700 cm⁻¹ (C=O).

### EXEMPLE 27 : Préparation du (3,4-dichloro-phényl)-(4-fluoro-4-{[(6-diméthylamino-pyridin-2-ylméthyl)-amino]-méthyl}-pipéridin-1-yl)-méthanone (I-14)

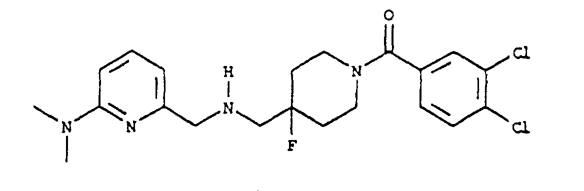

En procédant comme dans l'exemple 25 mais en remplaçant le 6-furan-2-yl-pyridine-2-carbaldéhyde par le 6-diméthylamino-pyridine-2-carbaldéhyde et la (4-aminométhyl-4-fluoro-pipéridin-1-yl)-(3-chloro-4-fluoro-phényl)-méthanone par la (4-aminométhyl-4-fluoro-pipéridin-1-yl)-(3,4-dichloro-phényl)-méthanone, on obtient le composé du titre sous la forme d'une huile jaune après purification sur colonne de silice (éluant : acétate d'éthyle).
Rendement : 57.8%
¹H RMN (CDCl₃) δ: 1.40-1.85 (m, 2H); 2.02 (s, 1H (échangeable)); 2.04(m, 2H); 2.76 (d, 2H); 3.05 (s, 6H); 3.10-3.65 (m, 3H); 3.73 (s, 2H); 4.48 (m, 1H); 6.35 (d, 1H); 6.44 (d, 1H); 7.20 (dd, 1H); 7.36 (dd, 1H); 7.47 (m, 2H).

On dissout 0.450 g du produit du titre (1.02 mmole) dans 10 ml d'éthanol puis on ajoute 0.115 g d'acide fumarique (0.99 mmole). On concentre la solution, la cristallisation est amorcée par addition d'acétate d'éthyle. Le précipité est filtré, lavé à l'acétate d'éthyle puis séché sous vide à 50°C. On obtient 0.470 g de fumarate du composé du titre sous la forme d'une poudre cristalline blanche.
F: 174°C

| Analyse C₂₅H₂₉Cl₂FN₄O₅: | | | | |
|---|---|---|---|---|
| Calc.% | C 54.06 | H 5.26 | Cl 12.77 | N 10.09 |
| Tr | 53.82 | 5.34 | 12.61 | 9.83 |

¹H RMN (DMSOd6) δ: 1.71 (m, 1H); 1.83 (m, 1H); 2.79 (d, 2H); 2.99 (s, 6H); 3.05-3.50 (m, 3H); 3.72 (s, 2H); 4.23 (m, 1H); 6.48 (d, 1H); 6.56 (d, 1H); 6.58 (s, 2H); 7.37 (dd, 1H); 7.44 (dd, 1H); 7.69 (m, 2H).
IR (KBr) υ: 1636 et 1702 cm⁻¹ (C=0).

### EXEMPLE 28 : Préparation du (3-Chloro-4-fluoro-phényl)-(4-fluoro-4-{[(5-méthyl-6-méthylamino-pyridine-2-ylméthyl)-amino]-méthyl}-pipéridin-1-yl)-méthanone (I-55)

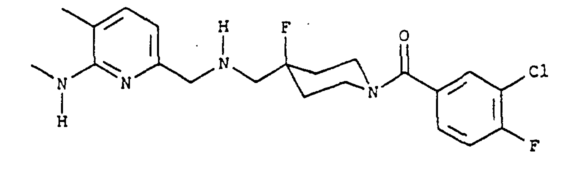

En procédant comme dans l'exemple 25 mais en remplaçant le 6-furan-2-yl-pyridine-2-carbaldéhyde par le 5-méthyl-6-méthylamino-pyridine-2-carbaldéhyde, on obtient le composé du titre sous la forme d'une huile jaune après purification sur colonne de silice (éluant : dichlorométhane / méthanol; 95 : 5).
Rendement: 55.6%
¹H RMN (CDCl₃) δ: 1.60 (m, 2H); 1.98 (m, 6H); 2.72 (d, 2H); 2.95 (s, 3H); 3.11 (m, 1H); 3.32 (m, 1H); 3.51 (m, 1H); 3.68 (s, 2H); 4.08 (s, 1H); 4.44 (m, 1H); 6.38 (d, 1H); 7.10 (m, 2H); 7.21 (m, 1H); 7.42 (dd, 1H)

La salification du produit du titre par l'acide fumarique dans l'éthanol donne 0.345 g de fumarate du composé du titre sous la forme d'une poudre cristalline blanche.
F: 163-64°C

| Analyse C₂₅H₂₉ClF₂N₄O₅ | | | | |
|---|---|---|---|---|
| Calc. % | C 55.71 | H 5.42 | Cl 6.58 | N 10.39 |
| Tr. | 55.64 | 5.42 | 6.46 | 10.36 |

¹H RMN (DMSOd6) δ: 1.72 (m, 2H); 1.78 (m, 3H); 2.00 (s, 3H); 2.80 (d, 2H); 2.83 (s, 3H); 3.06 (m, 1H); 3.25 (m, 1H); 3.51 (m, 1H); 3.69 (s, 2H); 4.26 (m, 1H); 5.91 (d, 1H); 6.44 (d, 1H); 6.59 (s, 2H); 7.15 (d, 1H); 7.46 (m, 2H); 7.65 (dd, 1H)
IR (KBr) υ: 1638 et 1690 cm⁻¹

### EXEMPLE 29 : Préparation du (3-chloro-4-fluoro-phényl)-(4-fluoro-4-{[(5-méthyl-6-diméthylamino-pyridin-2-ylméthyl)-amino]-méthyl}-pipéridin-1-yl)-méthanone (I-61)

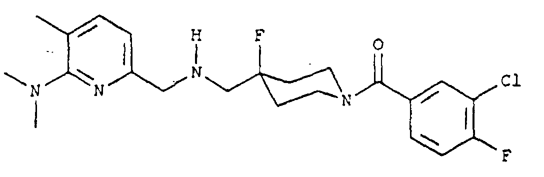

En procédant comme dans l'exemple 25 mais en remplaçant le furan-2-yl-pyridine-2-carbaldéhyde par le 5-méthyl-6-diméthylamino-pyridine-2-carbaldéhyde et le solvant de réaction (toluène) par du benzène, on isole le composé du titre par chromatographie sur colonne de silice (éluant : acétate d'éthyle / méthanol; 98 : 2).
Rendement: 66.7%
¹H RMN (CDCl₃) δ:1.68 (m, 2H); 2.04 (m, 2H); 2.26 (s, 3H); 2.80 (d, 2H); 2.85 (s, 6H); 3.17 (m, 1H); 3.39 (m, 1H); 3.60 (m, 1H), 3.79 (s, 2H); 4.52 (m, 1H); 6.69 (d, 1H); 7.17 (t, 1H); 7.28 (m, 2H); 7.48 (d, 1H)

La salification du produit du titre par l'acide oxalique dans l'éthanol donne 0 46 g d'oxalate du composé du titre sous forme d'une poudre cristalline blanche.
F: 205°C

| Analyse C₂₄H₂₉ClF₂N₄O₅: | | | | |
|---|---|---|---|---|
| Calc.% | C 54.70 | H 5.55 | Cl 6.73 | N 10.63 |
| Tr. | 54.62 | 5.55 | 6.76 | 10.48 |

¹H RMN (DMSOd6) δ: 1.75 (dt, 1H); 1.85 (m, 2H); 1.98 (m, 1H); 2.24 (s, 3H); 2.82 (s, 6H); 3.07 (m, 1H); 3.18 (d, 2H); 3.26 (m, 1H); 3.44 (m, 1H); 4.08 (s, 2H); 4.30 (m, 1H); 6.89 (d, 1H); 7.45 (m, 3H); 7.67 (dd, 1H)
IR (KBr) υ: 1636 et 1704 cm⁻¹

Les composés de formule générale (I), obtenus à partir des intermédiaires ou d'intermédiaires analogues à ceux des exemples 1 à 22, selon les procédés similaires à ceux des exemples 23 à 29 et comportant les substituants désirés sont rassemblés dans le tableau 1 ci-après.

### Dérivés de formule (I):

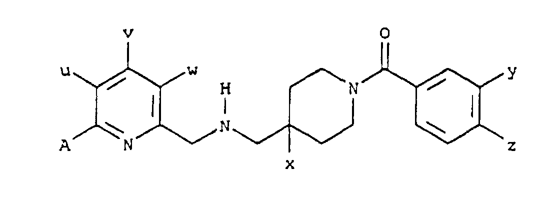

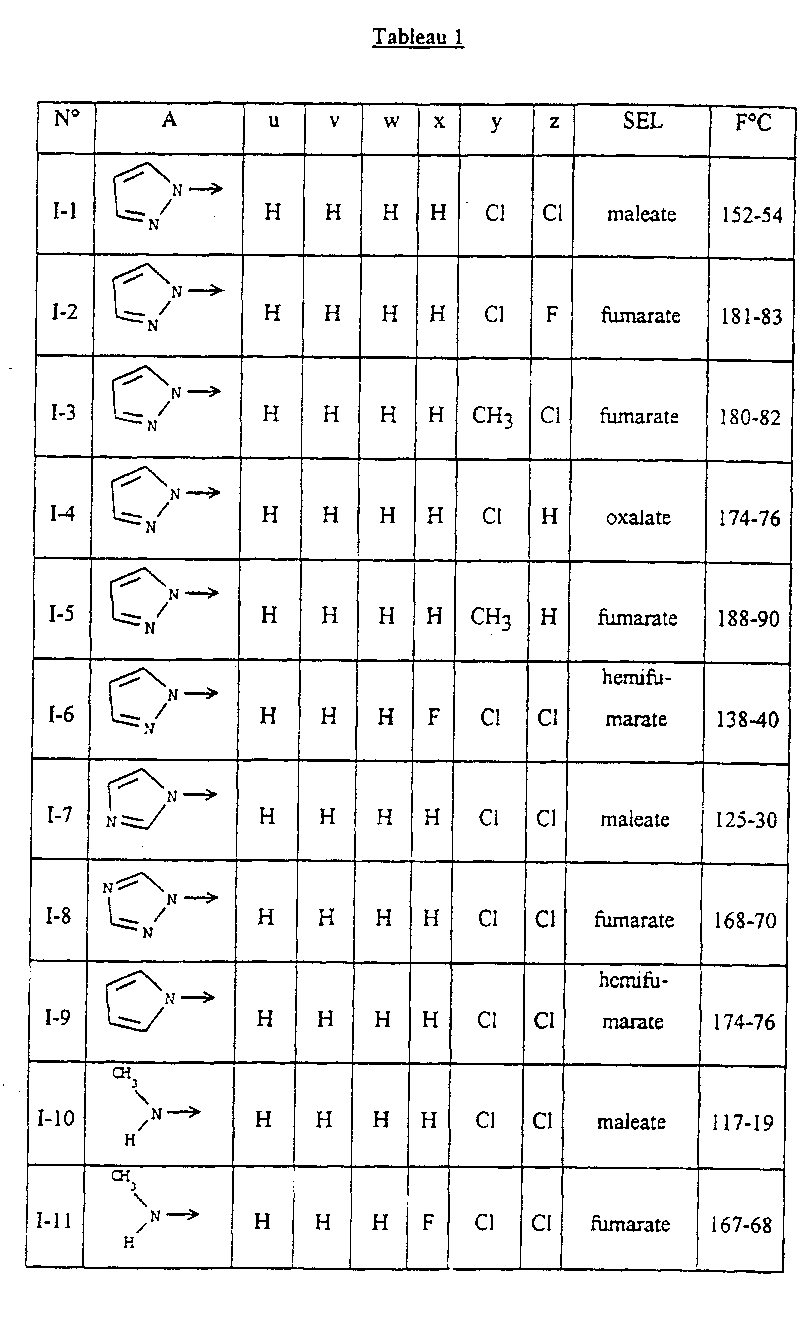

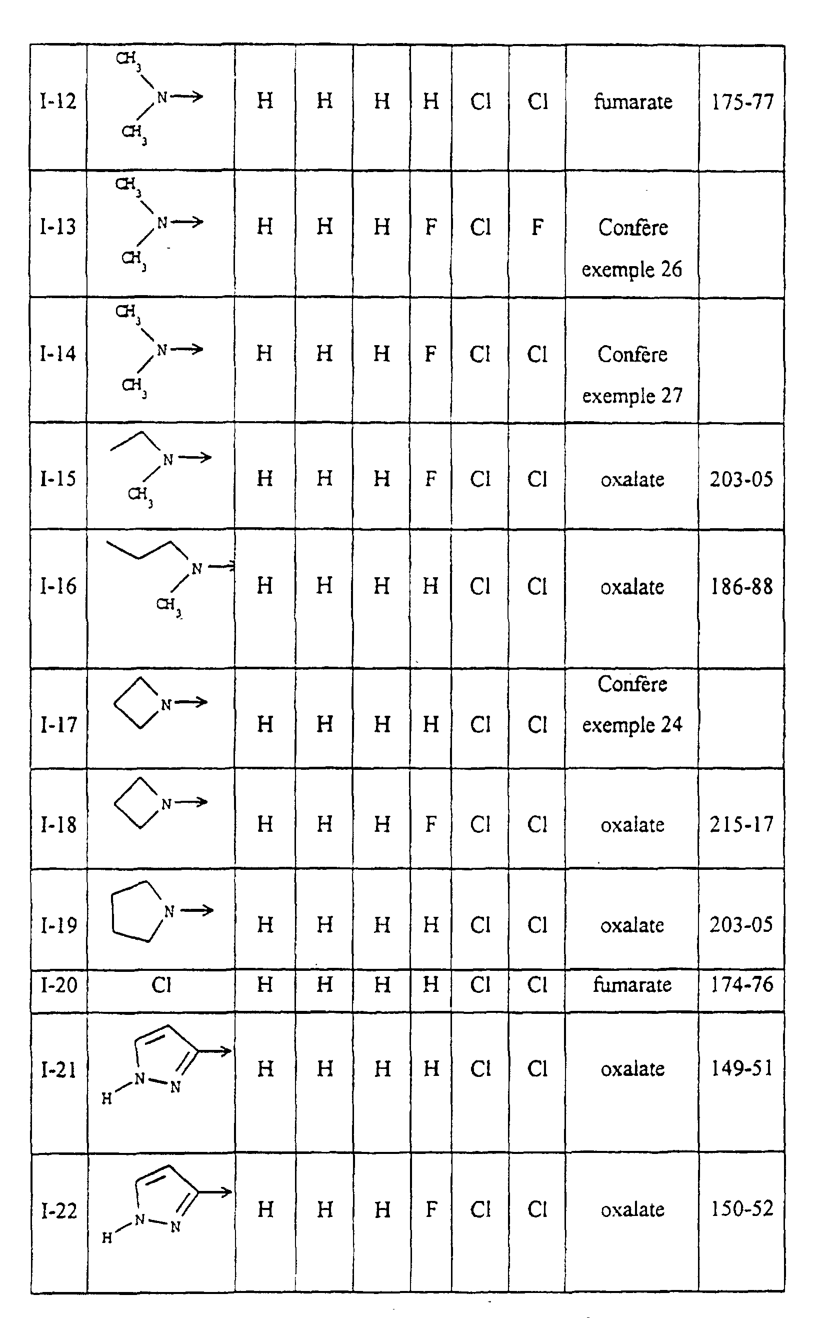

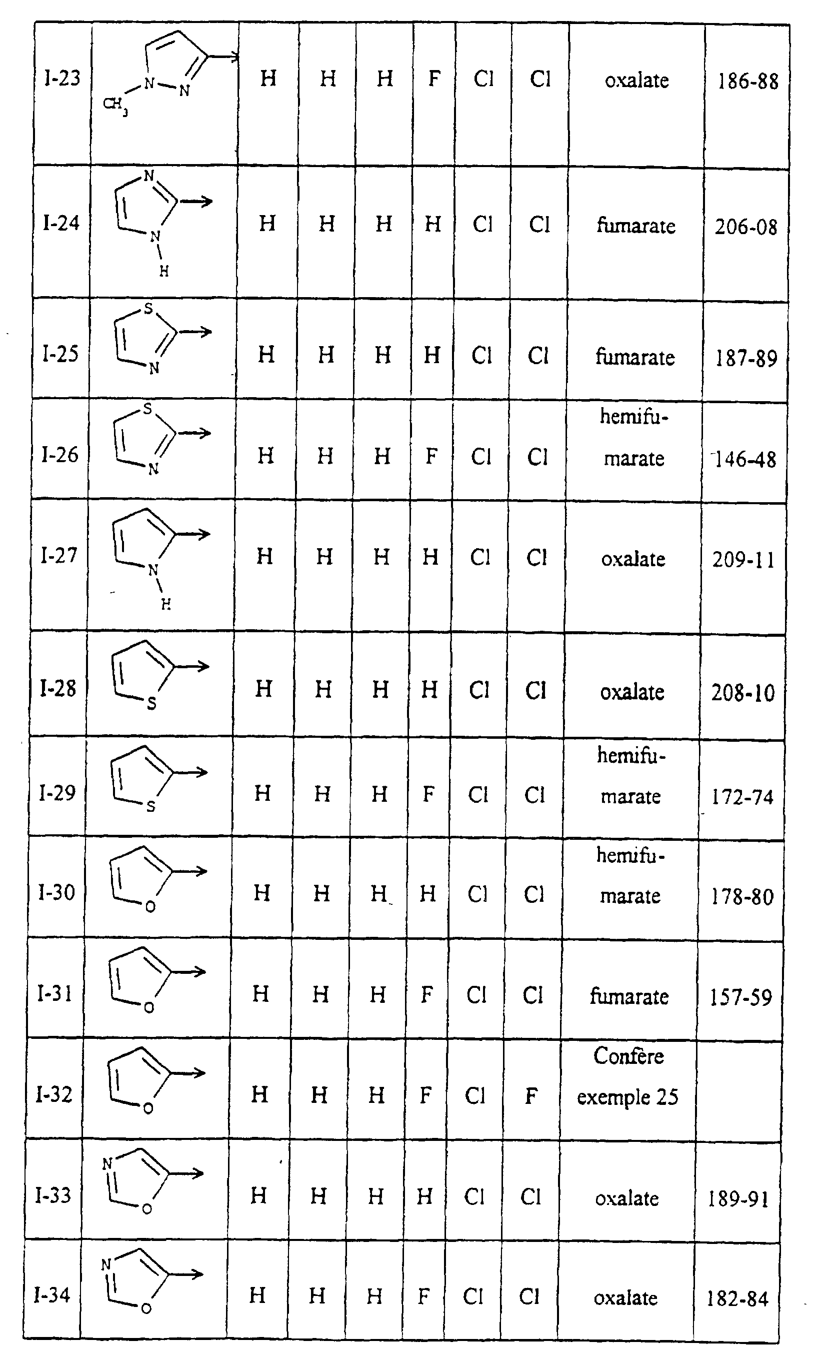

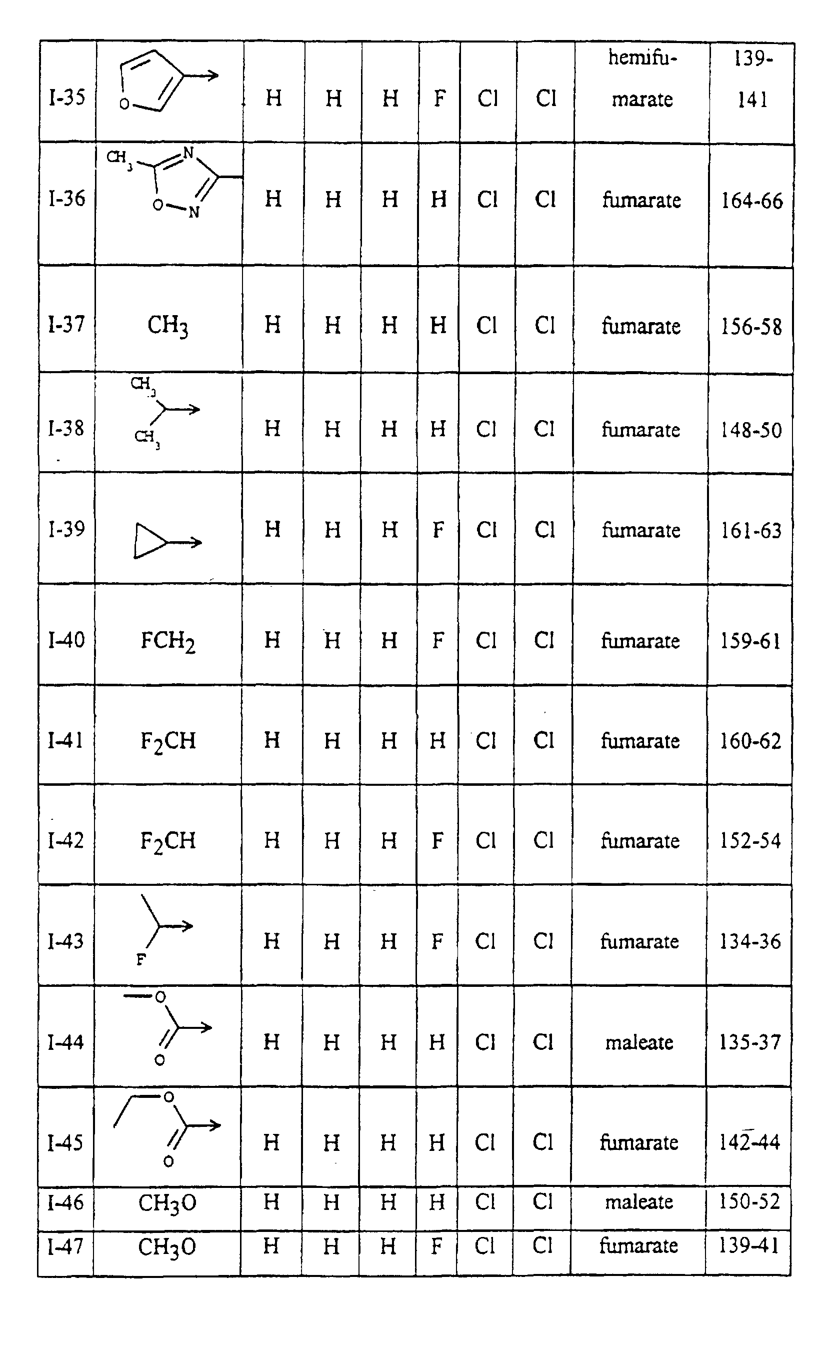

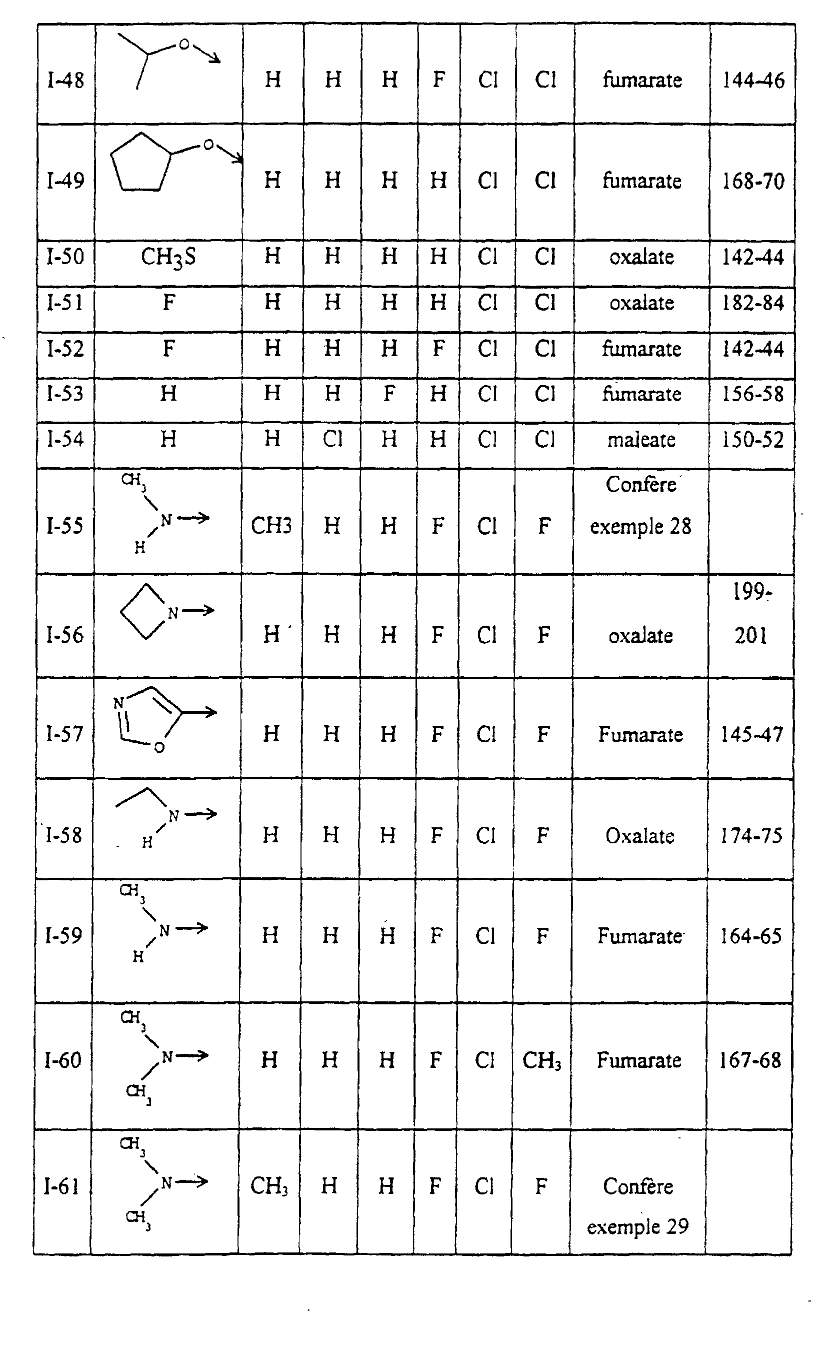

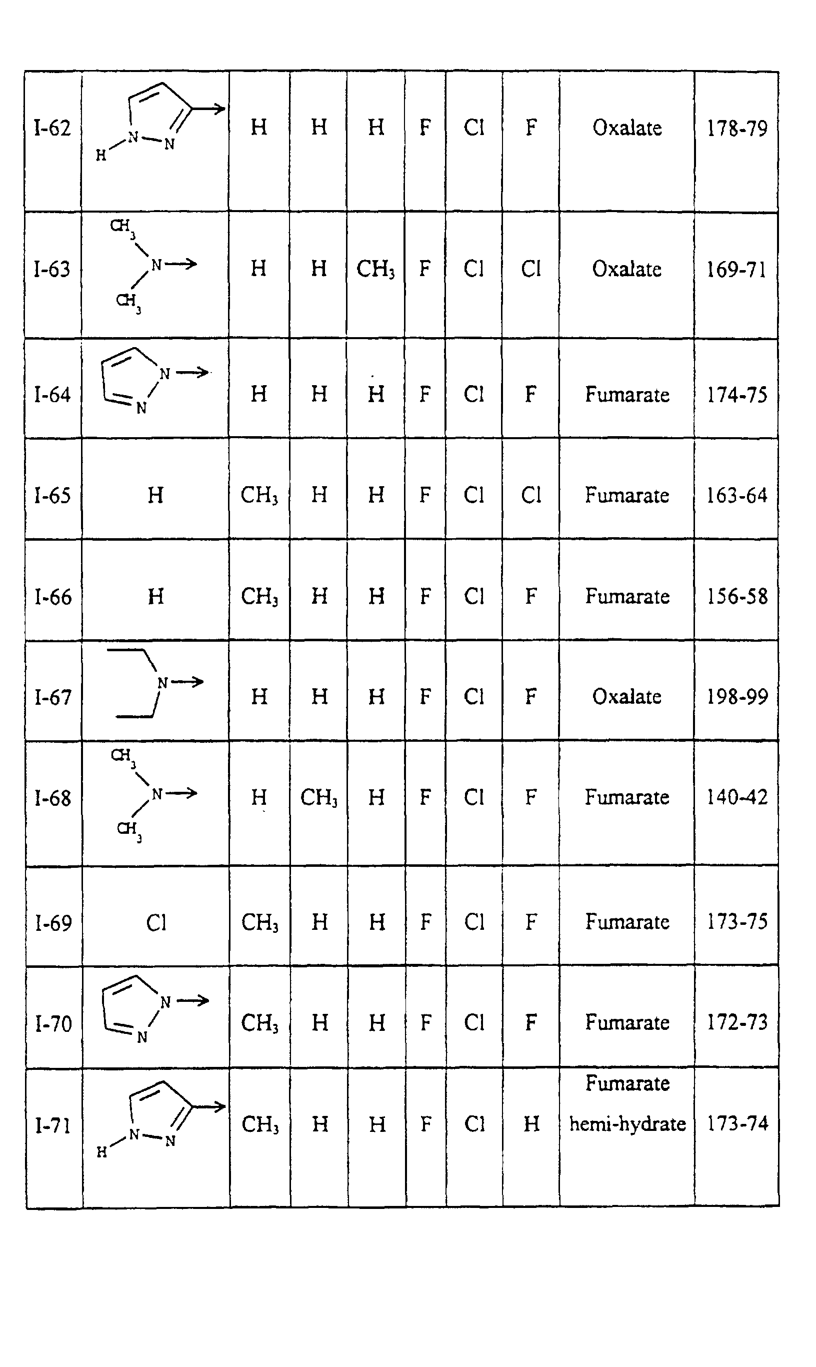

### ETUDE PHARMACOLOGIQUE DES COMPOSES DE L'INVENTION

### 1- Mesure de l'affinité des composés de l'invention pour les récepteurs 5-HT1A

### PROTOCOLE

L'affinité *in vitro* des composés de l'invention pour les récepteurs 5-HT1A a été déterminée par la mesure du déplacement de la (³H)8-OH-DPAT (TRK 850; 160-240 Ci/mmole).

L'étude de la liaison au récepteur 5-HT_{1A} est réalisée comme décrit par Sleight et Peroutka (Naunyn-Schmiedeberg's Arch. Pharmaco. 1991, 343, 106-16). Pour ces expérimentations, des cortex cérébraux de rat sont utilisés. Après décongélation du cerveau dans du tampon Tris-HCI 50 mmoles, pH = 7.40 à 25°C, le cortex cérébral est prélevé et homogénéisé dans 20 volumes de tampon maintenu à 4°C. L'homogénat est centrifugé à 39000 g pendant 10 minutes, le culot de centrifugation est mis en suspension dans le même volume de tampon et centrifugé à nouveau. Après une nouvelle mise en suspension dans les mêmes conditions, l'homogénat est incubé pendant 10 minutes à 37°C puis centrifugé à nouveau. Le culot final est mis en suspension dans du tampon de réaction froid Tris-HCI 50 mmoles, pH = 7.40 à 25°C contenant 10 mmoles de pargyline, 4 mmoles de CaCl₂ et 0.10% d'acide ascorbique. La concentration finale de tissu dans le milieu d'incubation est de 10 mg/tube.

Les tubes de réaction contiennent 0.10 ml de (³H)8-OH-DPAT (0.20 mmole en final), 0.10 ml de produit à tester 6-7 concentrations et 0.80 ml de tissu. La liaison non spécifique est définie en utilisant 10 mmoles de 5-HT. Les tubes de réaction sont incubés à 23°C pendant 30 minutes puis leur contenu est rapidement filtré sous vide sur filtres Whatman GF/B, les tubes sont rincés avec 2 fois 5 ml de tampon Tris-HCI 50 mmoles, pH = 7.4 à 25°C. La radioactivité recueillie sur le filtre est analysée en scintillation liquide en ajoutant 4ml de liquide scintillant (Emulsifier Safe, Packard). Toutes les expériences sont réalisées en triple.

### 2- Mesure de l'affinité des composés de l'invention pour les récepteurs D₂.

### PROTOCOLE

L'affinité *in vitro* des composés de l'invention pour les récepteurs dopaminergiques D₂, a été déterminée par la mesure du déplacement du (³H) YM-09151-2 (NET-1004 70-87 Ci/mmole). L'étude de la liaison au récepteur D₂ est réalisée comme décrit par Niznik (Naunyn-Schmiedeberg's Arch. Pharmacol. Methods, 1985, 329, 333-38). Pour ces expérimentations, on utilise le striatum de rat. Après décongélation du cerveau dans du tampon Tris-HCl 50 mmoles, pH = 7.40 à 25°C, le striatum est prélevé et homogénéisé dans 40 volumes de tampon maintenu à 4°C. l'homogénat est centrifugé à 20000 g pendant 10 minutes, le culot de centrifugation est mis en suspension dans le même volume de tampon et centrifugé à nouveau. Le culot final est mis en suspension dans du tampon de réaction froid Tris-HCl 50 mmoles, pH = 7.40 à 25°C contenant 120 mmoles de NaCl et 5 mmoles de KCl. La concentration finale de tissu dans le milieu d'incubation est de 2 mg/tube. Les tubes de réaction contiennent 0.20 ml de [³H]YM-09151-2 (0.05 mmole en final), 0.20 ml de produit à tester 6-7 concentrations et 1.60 ml de tissu. La liaison non spécifique est définie en utilisant 1 mmole de (+)-Butaclamol. Les tubes de réaction sont incubés à 23°C pendant 60 minutes puis leur contenu est rapidement filtré sous vide sur filtres Whatman GF/B, les tubes sont rincés 2 fois avec 5 ml de tampon Tris-HCl 50 mmoles, pH = 7.40 à 25°C. La radioactivité recueillie sur le filtre est analysée en scintillation liquide en ajoutant 4 ml de liquide scintillant (Emulsifier Safe, Packard). Toutes les expériences sont réalisées en triple.

### RESULTATS

Les constantes d'inhibition (Ki) des produits de l'invention sont estimées à partir des expérimentations de déplacement en utilisant le programme de régression non-linéaire RADLIG version 4 de EBDA (Equilibrium Binbing Data Analysis) (Biosoft, Cambridge, UK, Mc Pherson, 1985). Les constantes de dissociation des ligands radioactifs utilisées dans les calculs sont de 0.31 mmole pour (³H)8-OH-DPAT et de 0.036 mmole pour (³H)YM-09151-2. Les valeurs de pKi (-logKi) sont données sous forme de moyenne ± SEM d'au moins 3 expérimentations. Le tableau 2 donne, à titre l'exemple, les pKi (D2) ainsi que la sélectivité 5-HT1A / D2 pour plusieurs composés de l'invention, par rapport à la Buspirone et à la 8-OH-DPAT choisis comme produits de références.

**Tableau 2**

| Composé N° | pKi 5-HT_{1A} | pKi D₂ | Sélectivité 5-HT_{1A} / D₂ | LLR p.o (60 minutes) ED₅₀ mg/Kg |
|---|---|---|---|---|
| I-55 | 10.12 | 5.89 | 16982 | 0.08 |
| I-13 | 9.92 | 6.56 | 2291 | 0.31 |
| I-56 | 9.86 | 5.99 | 7414 | 0.08 |
| I-58 | 9.73 | 6.63 | 1259 | 0.31 |
| I-59 | 9.69 | 5.99 | 5011 | 0.31 |
| I-11 | 9.67 | 6.40 | 1862 | 0.31 |
| I-14 | 9.61 | 6.48 | 1349 | 0.31 |
| I-32 | 9.55 | 5.65 | 7942 | 0.31 |
| I-61 | 9.50 | 6.22 | 1906 | 0.08 |
| I-62 | 9.34 | 5.12 | 16594 | 0.31 |
| I-65 | 9.24 | < 5 | > 10000 | 0.31 |
| 8-OH-DPAT | 8.85 | 6.26 | 389 | 5 |
| Buspirone | 7.65 | 7.49 | 1.5 | 20 |

Les composés de formule générale (I) sont donc des ligands très puissants des récepteurs 5-HT_{1A} et se révèlent très sélectifs vis à vis des récepteurs D₂.

### 3- Evaluation de l'activité agoniste des récepteurs 5-HT_{1A} des composés de l'invention in vivo.

### PROTOCOLE

On utilise des rats mâles Sprague Dawley (ICO: OFAD [TOPS], Iffa Credo, France) pesant 160-180 g à leur arrivée et 180-200 g au début des tests. Les animaux sont placés en quarantaine de 4 à 8 jours avec un accès libre à la nourriture standardisée de laboratoire avant leur utilisation dans les expérimentations. Les animaux sont hébergés individuellement dans des cages en plastique sur portoir, (28 cm x 21 cm x 18 cm) avec un sol grillagé (RC Iffa Credo), 24 heures avant les tests. Grâce à un distributeur automatique, de l'eau filtrée à 0.22 µm, est disponible à volonté. La zone de quarantaine et le laboratoire d'expérimentation sont climatisés (température: 22 +- 1°C; degré hygrométrique: 55 +- 5 %) et éclairés de 7 heure à 19 heure. Tous les rats sont traités suivant l'éthique des animaux de laboratoire (Guide for the Care and Use of Laboratory Animals, U.S. Department of Agriculture. Public Health Service. National Institutes of Health publication N° 85-23, Revised 1985), et le protocole (n° 15) est réalisé en accord avec les recommandations du comité d'éthique local des animaux de recherche.

Les méthodes utilisées sont essentiellement identiques à celles décrites précédemment (Drug. Dev. Res. 1992, 26, 21-48; Eur. J. Pharmacol. 1995, 281, 219-28).

On observe le comportement de l'animal pendant une période de 10 minutes chacune, centrée à t60 minutes, après administration par voie orale. Quatre animaux sont observés individuellement durant la période de 10 minutes (de t55 à t65); les 4 rats sont observés à tour de rôle, toutes les 15 secondes, durée de l'observation 10 secondes par animal. Durant chacune de ces périodes d'observation, on note la présence (1) ou l'absence (0) de la rétraction de la lèvre inférieure (LLR) de l'animal. On considère qu'il y a rétraction de la lèvre inférieure si l'animal présente des signes ininterrompus durant au moins 3 secondes. Ce cycle est répété 10 fois durant une période de 10 minutes, ainsi, la fréquence d'un comportement peut varier de 0 à 10 pour chaque période d'observation. Chaque jour, deux animaux de chaque groupe reçoivent la même dose du même produit.

Les produits sont dissous dans de l'eau distillée ou sont mis en suspension dans une solution aqueuse de Tween 80 (2 gouttes / 10 ml d'eau distillée). Les produits sont administrés dans un volume de 10 ml / kg et les doses sont exprimées en poids de base. L'ordre d'administration des produits et des doses est randomisé.

### RESULTATS

Le tableau 2 donne, à titre d'exemple, les doses actives (ED₅₀) pour certains dérivés de l'invention par rapport à la Buspirone et à la 8-OH-DPAT.
Les résultats des essais montrent donc que certains composés de formule générale (I) possèdent une activité agoniste au niveau des récepteurs 5-HT_{1A}, aprés administration orale chez le rat, très supérieure à celle des produits de référence.

## Revendications

1. Dérivés de la pyridin-2-yl-méthylamine de formule (I): 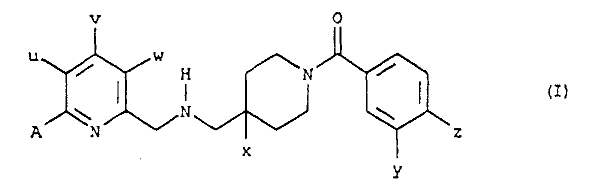 dans laquelle :
**u** représente un atome d'hydrogène ou un radical méthyl avec la réserve que lorsque **u** est un radical méthyl alors **v** et **w** représentent un atome d'hydrogène;
**v** représente un atome d'hydrogène ou un atome de chlore ou un radical méthyl avec la réserve que lorsque **v** représente un atome de chlore ou un radical méthyl alors **u** et **w** représentent un atome d'hydrogène;
**w** représente un atome d'hydrogène ou un atome de fluor ou un radical méthyl avec la réserve que lorsque **w** représente un atome de fluor ou un radical méthyl alors **u** et **v** représentent un atome d'hydrogène;
**x** représente un atome d'hydrogène ou un atome de fluor;
**y** représente un atome de chlore ou un radical méthyl;
**z** représente un atome d'hydrogène ou un atome de fluor ou un atome de chlore ou un radical méthyl;
**A** représente:
- un atome d'hydrogène ou un atome de fluor ou un atome de chlore;
- un radical alkyl en C₁-C₅, i.e. un reste d'hydrocarbures aliphatique saturé à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthyl-propyl, 2-méthyl-butyl ou 3-méthyl-butyl, 1-éthylpropyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons choisi parmi
furan-2-yl, (O.CH:CH.CH:C-),
furan-3-yl, (CH:CH.O.CH:C-),
1H-pyrrol-2-yl, (NH.CH:CH.CH:C-),
1H-pyrrol-3-yl, (CH:CH.NH.CH:C-),
1-méthyl-pyrrol-2-yl, (N(CH₃).CH:CH.CH:C-),
1-méthyl-pyrrol-3-yl, (CH:CH.N(CH₃).CH:C-),
thiophen-2-yl, (S.CH:CH.CH:C-),
thiophen-3-yl, (CH:CH.S.CH:C-),
pyrazol-1-yl, (N:CH.CH:CH.N-),
1H-pyrazol-3-yl, (CH:CH.NH.N:C-),
1H-pyrazol-4-yl, (CH:N.NH.CH:C-),
1-méthyl-pyrazol-3-yl, (CH:CH.N(CH₃).N:C-),
imidazol-1-yl (CH:N.CH:CH.N-),
1H-imidazol-2-yl, (NH.CH:CH.N:C-),
1H-imidazol-4-yl, (N:CH.NH.CH:C-),
oxazol-2-yl, (O.CH:CH.N:C-),
oxazol-4-yl, (N:CH.O.CH:C-),
oxazol-5-yl, (O.CH:N.CH:C-),
isoxazol-5-yl (O.N:CH.CH:C-),
isoxazol-4-yl, (CH:N.O.CH:C-),
isoxazol-3-yl, (CH:CH.O.N:C-),
thiazol-2-yl, (S.CH:CH.N:C-),
thiazol-4-yl, (N:CH.S.CH:C-),
thiazol-5-yl, (S.CH:N.CH:C-),
isothiazol-5-yl, (S.N:CH.CH:C-),
isothiazol-4-yl, (CH:N.S.CH:C-),
isothiazol-3-yl, (CH:CH.S.N:C-),
[1,2,4]triazol-1-yl, (CH:N.CH:N.N-),
1H-[1,2,4]triazol-3-yl, (N:CH.NH.N:C-),
[1,2,4]oxadiazol-3-yl, (N:CH.O.N:C-),
[1,2,4]oxadiazol-5-yl, (O.N:CH.N:C-),
5-méthyl-[1,2,4]oxadiazol-3-yl, (N:C(CH₃).O.N:C-) et
1H-tétrazol-5-yl, (NH.N:N.N:C-) ;
- un groupe alkoxy (R₁O-) ou alkylthio (R₁S-) dans lequel le radical R₁ représente:
· un radical alkyl en C₁-C₅ tel que défini précédemment,
· un radical monofluorométhyl ou trifluorométhyl,
· un radical cyclopropyl ou cyclobutyl ou cyclopentyl;
- un groupe amino de type II 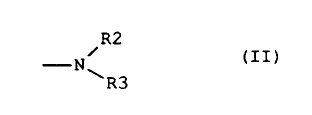 dans lequel R₂ et R₃, identiques ou différents représentent l'hydrogène, ou un radical alkyl en C₁-C₅ tel que défini précédemment ou un radical cyclopropyl ou cyclobutyl ou un radical trifluorométhyl;
- un groupe amino cyclique saturé de type III 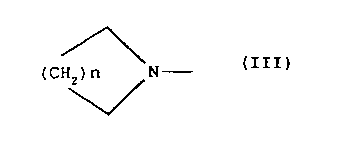 dans lequel **n** peut prendre les valeurs entières 1 ou 2;
- un groupe alkoxycarbonyl de préférence un groupe méthoxycarbonyl (CH₃OCO-) ou un groupe éthoxycarbonyl (CH₃CH₂OCO-).
ainsi que les sels d'addition des composés de formule générale (I) avec des acides minéraux ou les acides organiques pharmaceutiquement acceptables.

2. Composés de formule générale (I), selon la revendication 1, **caractérisés par le fait qu'**ils sont choisis parmi:
(3,4-Dichloro-phényl)-(4-{[(6-pyrazol-1-yl-pyridin-2-ylméthyl)-amino]-methyl}-pipéridin-1-yl)-méthanone
(3-Chloro-4-fluoro-phényl)-(4-{[(6-pyrazol-1-yl-pyridin-2-ylméthyl)-amino]-méthyl}-pipéridin-1-yl)-méthanone
(4-Chloro-3-méthyl-phényl)-(4-{[(6-pyrazol-1-yl-pyridin-2-ylméthyl)-amino]-méthyl}-pipéridin-1-yl)-méthanone
(3-Chloro-phényl)-(4-{[(6-pyrazol-1-yl-pyridin-2-ylméthyl)-amino]-méthyl}-pipéridin-1-yl)-méthanone
(4- {[(6-Pyrazol-1-yl-pyridin-2-ylméthyl)-amino]-méthyl}-pipéridin-1-yl)-*m*-tolyl-méthanone
(3,4-Dichloro-phényl)-(4-fluoro-4-{[(6-pyrazol-1-yl-pyridin-2-ylméthyl)-amino]-méthyl} -pipéridin-1-yl)-méthanone
(3,4-Dichloro-phényl)-(4-{ [(6-imidazol-1-yl-pyridin-2-ylméthyl)-amino]-méthyl}-pipéridin-1-yl)-méthanone
(3,4-Dichloro-phényl)-(4- {[(6-[1,2,4,]triazol-1-yl-pyridin-2-ylméthyl)-amino]-méthyl}-pipéridin-1-yl)-méthanone
(3,4-Dichloro-phényl)-(4-{[(6-pyrrol-1-yl-pyridin-2-ylméthyl)-amino]-méthyl}-pipéridin-1-yl)-méthanone
(3,4-Dichloro-phényl)-(4-{[(6-méthylamino-pyridin-2-ylméthyl)-amino]-méthyl}-pipéridin-1-yl)-méthanone
(3,4-Dichloro-phényl)-(4-fluoro-4-{[(6-méthylamino-pyridin-2-ylméthyl)-amino]-méthyl}-pipéridin-1-yl)-méthanone
(3,4-Dichloro-phényl}-(4-{[(6-diméthylamino-pyridin-2-ylméthyl)-amino]-méthyl}-pipéridin-1-yl)-méthanone
(3-Chloro-4-fluoro-phényl)-(4-{[(6-diméthylamino-pyridin-2-ylméthyl)-amino]-méthyl}-4-fluoro-pipéridin-1-yl)-méthanone
(3,4-Dichloro-phényl)-(4-{[(6-diméthylamino-pyridin-2-ylméthyl}-amino]-méthyl}-4-fluoro-pipéridin-1-yl)-méthanone
(3,4-Dichloro-phényl)-[4-({[6-(éthyl-méthyl-amino)-pyridin-2-ylméthyl]-amino}-méthyl)-4-fluoro-pipéridin-1-yl]-méthanone
(3,4-Dichloro-phényl)-[4-({[6-(méthyl-propyl-amino)-pyridin-2-ylméthyl]-amino}-méthyl)-pipéridin-1-yl]-méthanone
(4-{[(6-Azétidin-1-yl-pyridin-2-ylméthyl)-amino]-méthyl}-pipéridin-1-yl}-(3,4-dichloro-phényl)-méthanone
(4-{[(6-Azétidin-1-yl-pyridin-2-ylméthyl)-amino]-méthyl}-4-fluoro-pipéridin-1-yl)-(3,4-dichloro-phényl)-méthanone
(4-{[(6-Pyrrolidin-1-yl-pyridin-2-ylméthyl)-amino]-méthyl}-pipéridin-1-yl)-(3,4-dichloro-phényl)-méthanone
(4-{[(6-Chloro-pyridin-2-ylméthyl)-amino]-méthyl}-pipéridin-1-yl)-(3,4-dichloro-phényl)-méthanone
(3,4-Dichloro-phényl)-[4-({[6-(1*H*-pyrazol-3-yl-)-pyridin-2-ylméthyl]-amino}-méthyl)-pipéridin-1-yl]-méthanone
(3,4-Dichloro-phényl)-[4-fluoro-4-({[6-(1*H*-pyrazol-3-yl-)-pyridin-2-ylméthyl]-amino}-méthyl)-pipéridin-1-yl]-méthanone
(3,4-Dichloro-phényl)-[4-fluoro-4-( {[6-(1-méthyl-pyrazol-3-yl)-pyridin-2-ylméthyl]-amino}-méthyl)-pipéridin-1-yl]-méthanone
(3,4-Dichloro-phényl)-[4-({[6-(1*H*-imidazol-2-yl)-pyridin-2-ylméthyl]-amino}-méthyl)-pipéridin-1-yl]-méthanone
(3,4-Dichloro-phényl)-(4- {[(6-thiazol-2-yl-pyridin-2-ylméthyl)-amino]-methyl}-pipéridin-1-yl)-méthanone
(3,4-Dichloro-phényl)-(4-fluoro-4-{[(6-thiazol-2-yl-pyridin-2-ylméthyl)-amino]-méthyl}-pipéridin-1-yl)-méthanone
(3,4-Dichloro-phényl)-[4-( {[6-(1*H*-pyrrol-2-yl)-pyridin-2-ylméthyl]-amino} -méthyl)-pipéridin-1-yl]-méthanone
(3,4-Dichloro-phényl}-(4-{[(6-thiophen-2-yl-pyridin-2-ylméthyl)-amino]-méthyl}-pipéridin-1-yl)-méthanone
(3,4-Dichloro-phényl)-(4-fluoro-4-{[(6-thiophen-2-yl-pyridin-2-ylméthyl)-amino]-méthyl }-pipéridin-1-yl)-méthanone
(3,4-Dichloro-phényl)-(4-{[(6-furan-2-yl-pyridin-2-ylméthyl)-amino]-méthyl}-pipéridin-1-yl)-méthanone
(3,4-Dichloro-phényl)-(4-fluoro-4-{[(6-furan-2-yl-pyridin-2-ylméthyl)-amino]-méthy}-pipéridin-1-yl)-méthanone
(3-Chloro-4-fluoro-phényl)-(4-fluoro-4-{[(6-furan-2-yl-pyridin-2-ylméthyl)-amino]-méthyl}-pipéridin-1-yl)-méthanone
(3,4-Dichloro-phényl)-(4-{[(6-oxazol-5-yl-pyridin-2-ylméthyl)-amino]-méthyl}-pipéridin-1-yl)-méthanone
(3,4-Dichloro-phényl)-(4-fluoro-4-{[(6-oxazol-5-yl-pyridin-2-ylméthyl)-amino]-méthyl}-pipéridin-1-yl)-méthanone
(3,4-Dichloro-phényl)-(4-fluoro-4-{[(6-furan-3-yl-pyridin-2-ylméthyl)-amino]-méthyl}-pipéridin-1-yl)-méthanone
(3,4-Dichloro-phényl)-[4-({[6-(5-méthyl-[1,2,4]oxadiazol-3-yl)-pyridin-2-ylméthyl]-amino }-méthyl)-pipéridin-1-yl]-méthanone
(3,4-Dichloro-phényl)-(4-{[(6-méthyl-pyridin-2-ylméthyl)-amino]-méthyl}-pipéridin-1-yl)-méthanone
(3,4-Dichloro-phényl)-(4-{[(6-isopropyl-pyridin-2-ylméthyl)-amino]-méthyl}-pipéridin-1-yl)-méthanone
(3,4-Dichloro-phényl)-(4-{[(6-cyclopropyl-pyridin-2-ylméthyl)-amino]-méthyl}-4-fluoro-pipéridin-1-yl)-méthanone
(3,4-Dichloro-phényl)-(4-fluoro-4-{[(6-fluorométhyl-pyridin-2-ylméthyl)-amino]-méthyl} -pipéridin-1-yl)-méthanone
(3,4-Dichloro-phényl)-(4-{[(6-difluorométhyl-pyridin-2-ylméthyl)-amino]-méthyl}-pipéridin-1-yl)-méthanone
(3,4-Dichloro-phényl)-(4-{[(6-difluorométhyl-pyridin-2-ylméthyl)-amino]-méthyl}-4-fluoro-pipéridin-1-yl)-méthanone
(3,4-Dichloro-phényl)-[4-fluoro-4-({[6-(1-fluoro-éthyl)-pyridin-2-ylméthyl]-amino}-méthyl)-pipéridin-1-yl]-méthanone
6-({[1-(3,4-Dichloro-benzoyl)-pipéridin-4-ylméthyl]-amino}-méthyl)-pyridine-2-carboxylic acid méthyl ester
6-({[1-(3,4-Dichloro-benzoyl)-pipéridin-4-ylméthyl]-amino}-méthyl)-pyridine-2-carboxylic acid éthyl ester
(3,4-Dichloro-phényl)-(4-{[(6-méthoxy-pyridin-2-ylméthyl)-amino]-méthyl}-pipéridin-1-yl)-méthanone
(3,4-Dichloro-phényl)-(4-fluoro-4-{[(6-méthoxy-pyridin-2-ylméthyl)-amino]-méthyl}-pipéridin-1-yl)-méthanone
(3,4-Dichloro-phényl)-(4-fluoro-4{[(6-isopropyloxy-pyridin-2-ylméthyl)-amino)-méthyl}-pipéridin-1-yl)-méthanone
(4-{[(6-Cyclopentyloxy-pyridin-2-ylméthyl)-amino]-méthyl}-pipéridin-1-yl)-(3,4-dichloro-phényl)-méthanone
(3,4-Dichloro-phényl)-(4-{[(6-méthylsulfanyl-pyridin-2-ylméthyl)-amino]-méthyl}-pipéridin-1-yl)-méthanone
(3,4-Dichloro-phényl)-(4-{[(6-fluoro-pyridin-2-ylméthyl)-amino]-méthyl}-pipéridin-1-yl)-méthanone
(3,4-Dichloro-phényl)-(4-fluoro-4-{[(6-fluoro-pyridin-2-ylméthyl)-amino]-méthyl}-pipéridin- 1-yl)-méthanone
(3,4-Dichloro-phényl)-(4-fluoro-{[(3-fluoro-pyridin-2-ylméthyl)-amino]-méthyl}-pipéridin-1-yl)-méthanone
(4-{[(4-Chloro-pyridin-2-ylméthyl)-amino]-méthyl}-pipéridin-1-yl)-(3,4-dichloro-phényl)-méthanone
(3-Chloro-4-fluoro-phényl)-(4-fluoro-4-{[(5-méthyl-6-furan-2-yl-pyridin-2-ylméthyl)-amino]-méthyl)-pipéridin-1-yl)-méthanone
(3-Chloro-phényl)-[4-fluoro-4-({[5-méthyl-6-(1*H*-pyrazol-3-yl)-2-yl-pyridin-2-ylméthyl]-amino}-méthyl)-pipéridin-1-yl]-méthanone
(3-Chloro-4-fluoro-phényl)-(4-fluoro-4-{[(5-méthyl-6-méthylamino-pyridin-2-ylméthyl)-amino]-méthyl}-pipéridin-1-yl)-méthanone
(4-{[(6-Azétidin-1-yl-pyridin-2-ylméthyl)-amino]-méthyl}-4-fluoro-pipéridin-1-yl)-(3-chloro-4-fluoro-phényl)-méthanone
(3-Chloro-4-fluoro-phényl)-(4-fluoro-4-{[(6-oxazol-5-yl-pyridin-2-ylméthyl)-amino]-méthyl)-pipéridin-1-yl)-méthanone
(3-Chloro-4-fluoro-phényl)-(4-fluoro-4-{[(6-éthylamino-pyridin-2-ylméthyl)-amino]-méthyl}-pipéridin-1-yl)-méthanone
(3-Chloro-4-fluoro-phényl)-(4-fluoro-4-{[(6-méthylamino-pyridin-2-ylméthyl)-amino]-méthyl}-pipéridin-1-yl)-méthanone
(3-Chloro-4-méthyl-phényl)-(4-fluoro-4-{[(6-diméthylamino-pyridin-2-ylméthyl)-amino]-méthyl}-pipéridin-1-yl)-méthanone
(3-Chloro-4-fluoro-phényl)-(4-fluoro-4{[(5-méthyl-6-diméthylamino-pyridin-2-ylméthyl)-amino]-méthyl}-pipéridin-1-yl)-méthanone
(3-Chloro-4-fluoro-phényl)-[4-fluoro-4-({[6-(1*H*-pyrazol-3-yl)-pyridin-2-ylméthyl]-amino}-méthyl)-pypéridin-1-yl]-méthanone
(3,4-Dichloro-phényl)-(4-fluoro-4-{[(3-méthyl-6-diméthylamino-pyridin-2-ylméthyl)-amino]-méthyl}-pipéridin-1-yl)-méthanone
(3-Chloro-4-fluoro-phényl)-(4-fluoro-4- {[(6-pyrazol-1-yl-pyridin-2-ylméthyl)-amino]-méthyl }-pipéridin-1-yl)-méthanone
{3,4-Dichloro-phényl)-(4-fluoro-4-{[(5-méthyl-pyridin-2-ylméthyl)-amino]-méthyl}-pipéridin-1-yl)-méthanone
(3-Chloro-4-fluoro-phényl)-(4-fluoro-4-{[(5-méthyl-pyridin-2-ylméthyl)-amino]-méthyl}-pipéridin-1-yl)-méthanone
(3-Chloro-4-fluoro-phényl)-(4-fluoro-4-{[(6-diéthylamino-pyridin-2-ylméthyl)-amino]-méthyl}-pipéridin-1-yl)-méthanone
(3-Chloro-4-fluoro-méthyl)-(4-fluoro-4-{[(5-méthyl-6-chloro-pyridin-2-ylméthyl)-amino]-méthyl}-pipéridin-1-yl)-méthanone
(3-Chloro-4-fluoro-phényl)-(4-fluoro-4-{[(4-méthyl-6-diméthylamino-pyridin-2-ylméthyl)-amino]-méthyl}-pipéridin-1-yl)-méthanone
(3-Chloro-4-fluoro-phényl)-(4-fluoro-4-{[(5-méthyl-6-pyrazol-1-yl-pyridin-2-ylméthyl)-amino]-méthyl}-pipéridin-1-yl)-méthanone
(3-Chloro-phényl)(4-fluoro-4-{[(6-diméthylamino-pyridin-2-ylméthyl)-amino]-méthyl}-pipéridin-1-yl)-méthanone

3. Procédé de préparation des composés de formule générale (**Ia**) selon les revendications 1 et 2, **caractérisé en ce que** l'on fait réagir un aldéhyde de formule générale (**IV**) avec une amine de formule générale (**V**) en milieu réducteur: 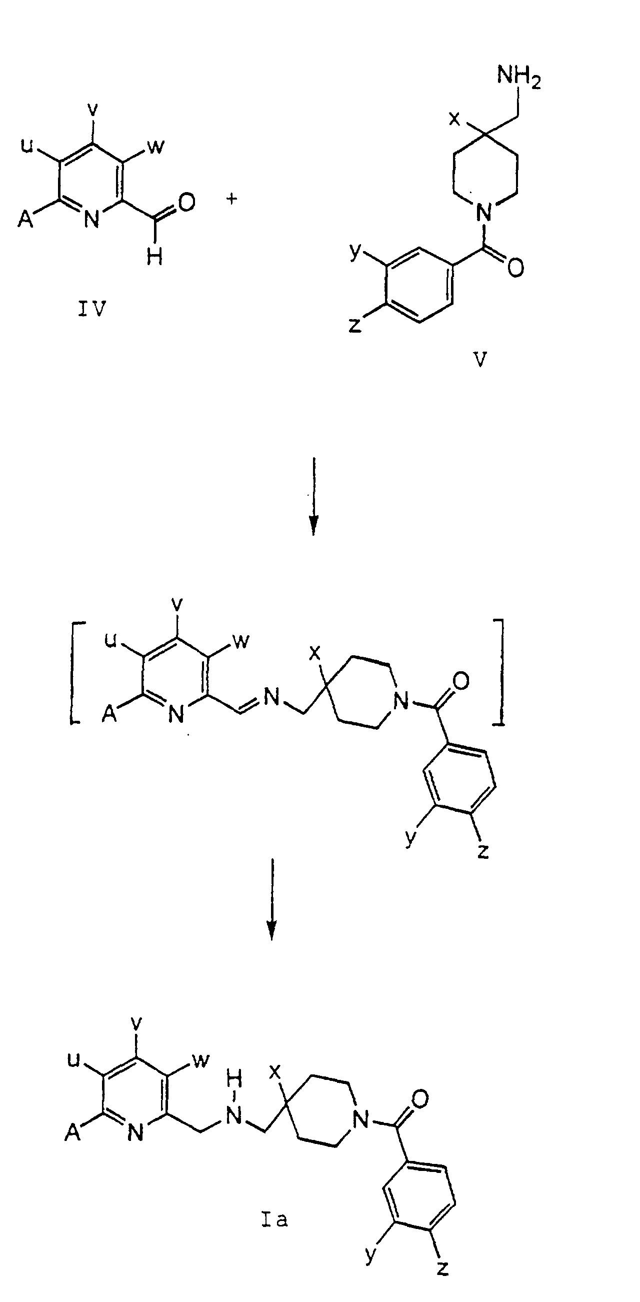 où : **A, u, v, w, x, y et z** sont définis comme précédemment.

4. Procédé de préparation des composés de formule générale (Ia) selon les revendications 1 et 2, **caractérisé en ce que** l'on fait réagir un aldéhyde de formule générale (IV) avec un dérivé de type azido de formule générale (VI) en présence d'une aryle ou alkylphosphine en milieu réducteur : 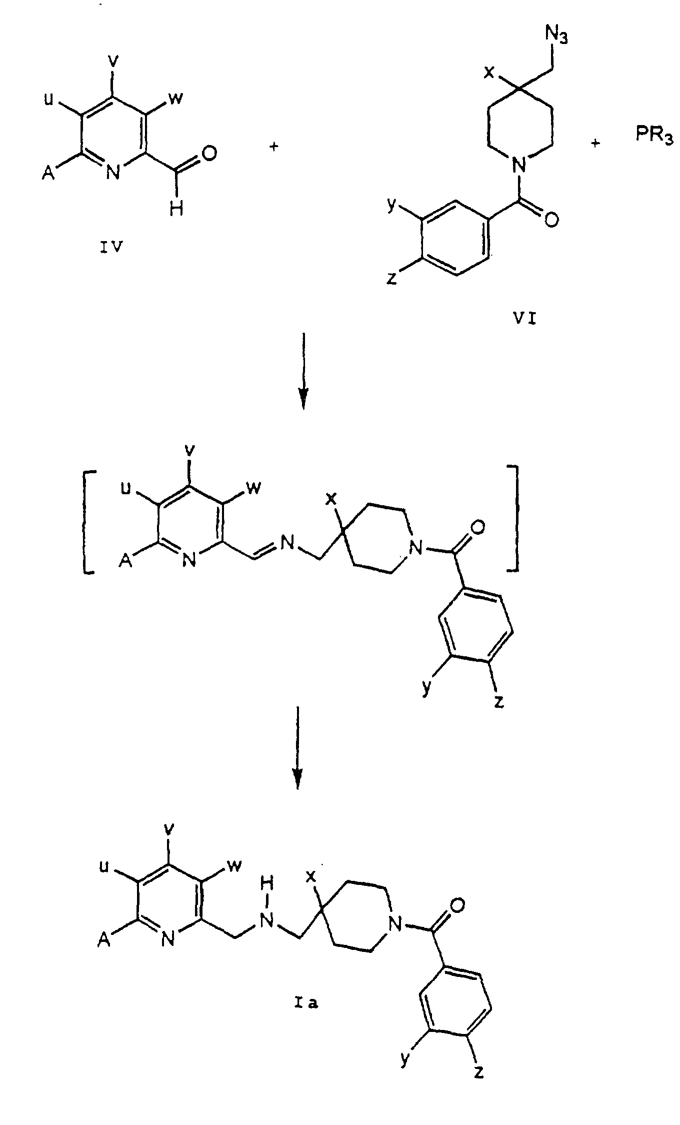 où : **A, u, v, w, x, y et z** sont définis comme précédemment.

5. Procédé de préparation de composé (Ib), cas particulier des composés de formule (Ia) où **x** est un atome d'hydrogène, selon les revendications 1 et 2, **caractérisé en ce que** l'on fait réagir un aldéhyde de formule (IV) successivement avec la pipéridin-4-yl-méthylamine, un chlorure d'acide puis un agent réducteur selon une technique "one-pot": 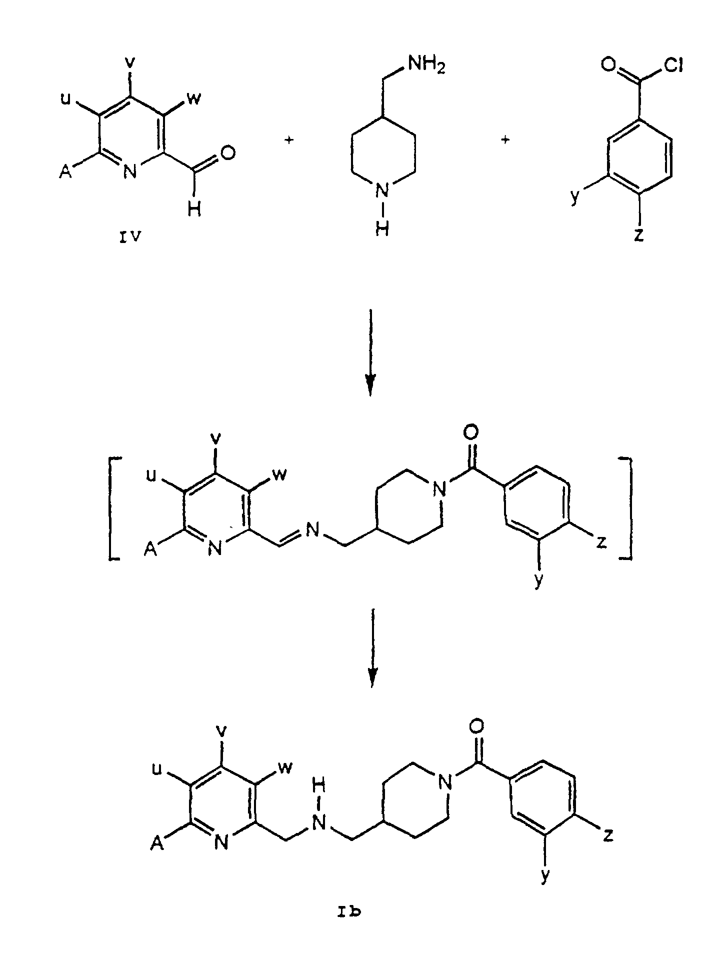 où: **A, u, v, w, x, y et z** sont tels que définis précédemment.

6. Procédé de préparation de composé (Ic), cas particulier des composés de formule (Ia), selon les revendications 1 et 2, **caractérisé en ce que** l'on fait réagir un réactif du type (HA) ou (A⁻Na⁺⁾ sur un composé de formule (Ia: A = F ou Cl) : 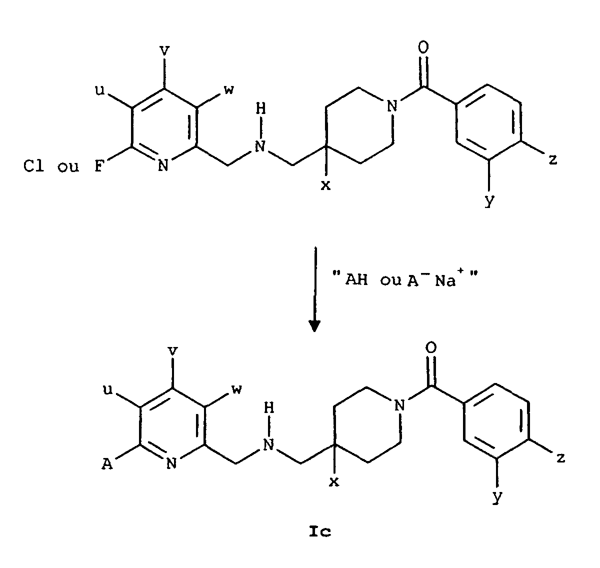 où:
· **u, v, w, x, y et z** sont tels que définis précédemment,
· **A** est choisi parmi un groupe amino de type II ou un groupe amino cyclique saturé de type III ou un radical pyrrol-1-yl, pyrazol-1-yl, imidazol-1-yl ou [1,2,4]triazol-1-yl.

7. Intermédiaires de synthèse de formule (V) utilisés pour la préparation des composés de formule générale (I) : 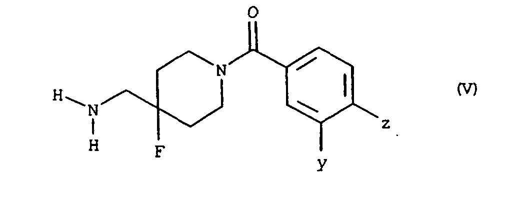 où **y** et **z** sont tels que définis à la revendication 1.

8. Intermédiaires de synthèse de formule (VI) utilisés pour la préparation des composés de formule générale (I): 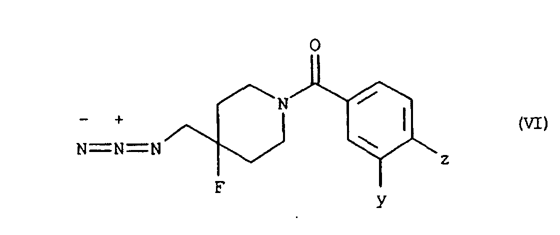 où **y** et **z** sont tels que définis à la revendication 1.

9. Dérivés selon la revendication 1 ou 2 à titre de médicament.

10. Médicament selon la revendication 9 pour le traitement de la dépression.

11. Médicament selon la revendication 9 pour le traitement de l'anxiété.

12. Médicament selon la revendication 9 pour le traitement des troubles obsessionnels compulsifs.

13. Médicament selon la revendication 9 pour le traitement des crises de panique.

14. Médicament selon la revendication 9 pour le traitement de la perception de la douleur.

15. Médicament selon la revendication 9 pour le traitement de l'agressivité, de l'abus d'alcool, des troubles du sommeil.

16. Médicament selon la revendication 9 pour le traitement des désordres vasculaires en particulier des désordres cérébrovasculaires, de la migraine et du vomissement.

17. Composition pharmaceutique **caractérisée en ce qu'**elle contient à titre de principe actif au moins un composé selon l'une des revendications 1 et 2, ou un de ses sels d'addition à un acide pharmaceutiquement acceptable, en combinaison avec un ou plusieurs excipients ou véhicules pharmaceutiquement acceptables.

## Patentansprüche

1. Derivate von Pyridin-2-ylmethylamin der Formel (I): 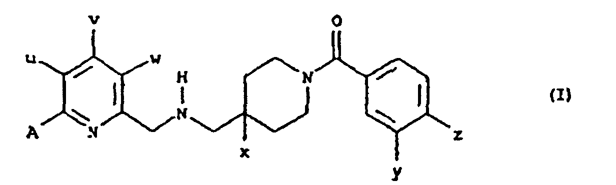 in welcher:
**u** für ein Wasserstoffatom oder einen Methylrest steht mit der Maßgabe, dass, wenn **u** ein Methylrest ist, dann **v** und **w** für ein Wasserstoffatom stehen;
**v** für ein Wasserstoffatom oder ein Chloratom oder einen Methylrest steht mit der Maßgabe, dass, wenn **v** für ein Chloratom oder einen Methylrest steht, dann **u** und **w** für ein Wasserstoffatom stehen;
**w** für ein Wasserstoffatom oder ein Fluoratom oder einen Methylrest steht mit der Maßgabe, dass, wenn **w** für ein Fluoratom oder einen Methylrest steht, dann **u** und **v** für ein Wasserstoffatom stehen;
**x** für ein Wasserstoffatom oder ein Fluoratom steht;
**y** für ein Chloratom oder einen Methylrest steht;
**z** für ein Wasserstoffatom oder ein Fluoratom oder ein Chloratom oder einen Methylrest steht;
**A** steht für:
- ein Wasserstoffatom oder ein Fluoratom oder ein Chloratom;
- einen C₁-C₅-Alkylrest, d.h. einen gesättigten aliphatischen gerad- oder verzweigtkettigen Kohlenwasserstoffrest, der 1 bis 5 Kohlenstoffatome enthält, wie Methyl, Ethyl, Propyl, Butyl, Pentyl, Isopropyl, 1-Methylethyl, 1-Methylpropyl, 1-Methylbutyl, 2-Methylpropyl, 2-Methylbutyl oder 3-Methylbutyl, 1-Ethylpropyl, 2-Ethylpropyl;
- einen Fluoralkylrest, wie Monofluormethyl (-CH₂F) oder Difluormethyl (-CHF₂) oder Trifluormethyl (-CF₃) oder 1-Fluor-1-ethyl (CHFCH₃) oder 1,1-Difluor-1-ethyl (-CF₂CH₃) ;
- einen Cyclopropyl- oder Cyclobutyl- oder Cyclopentylrest;
- eine heterocyclische aromatische Gruppe mit 5 Kettengliedern, ausgewählt unter
Furan-2-yl, (O.CH:CH.CH:C-),
Furan-3-yl, (CH:CH.O.CH:C-),
1H-Pyrrol-2-yl, (NH.CH:CH.CH:C-),
1H-Pyrrol-3-yl, (CH:CH.NH.CH:C-),
1-Methylpyrrol-2-yl, (N(CH₃) .CH:CH.CH:C-),
1-Methylpyrrol-3-yl, (CH:CH.N(CH₃).CH:C-),
Thiophen-2-yl, (S.CH:CH.CH:C-),
Thiophen-3-yl, (CH:CH.S.CH:C-),
Pyrazol-1-yl, (N:CH.CH:CH.N-),
1H-Pyrazol-3-yl, (CH:CH.NH.N:C-),
1H-Pyrazol-4-yl, (CH:N.NH.CH:C-),
1-Methylpyrazol-3-yl, (CH:CH.N(CH₃).N:C-),
Imidazol-1-yl, (CH:N.CH:CH.N-),
1H-Imidazol-2-yl, (NH.CH:CH.N:C-),
1H-Imidazol-4-yl, (N:CH.NH.CH:C-),
Oxazol-2-yl, (O.CH:CH.N:C-),
Oxazol-4-yl, (N:CH.O.CH:C-),
Oxazol-5-yl, (O.CH:N.CH:C-),
Isoxazol-5-yl (O.N:CH.CH:C-),
Isoxazol-4-yl, (CH:N.O.CH:C-),
Isoxazol-3-yl, (CH:CH.O.N:C-),
Thiazol-2-yl, (S.CH:CH.N:C-),
Thiazol-4-yl, (N:CH.S.CH:C-),
Thiazol-5-yl, (S.CH:N.CH:C-),
Isothiazol-5-yl, (S.N:CH.CH:C-),
Isothiazol-4-yl, (CH:N.S.CH:C-),
Isothiazol-3-yl, (CH:CH.S.N:C-),
[1,2,4]-Triazol-1-yl, (CH:N.CH:N.N-),
lH-[1,2,4]-Triazol-3-yl, (N:CH.NH.N:C-),
[1,2,4]-Oxadiazol-3-yl, (N:CH.O.N:C-),
[1,2,4]-Oxadiazol-5-yl, (O.N:CH.N:C-),
5-Methyl-[1,2,4]-oxadiazol-3-yl, (N:C(CH₃).O.N:C-) und
1H-Tetrazol-5-yl, (NH.N:N.N:C-);
- eine Alkoxygruppe (R₁O-) oder Alkylthiogruppe (R₁S-),
in welcher der Rest R₁ für
- einen C₁-C₅-Alkylrest, wie zuvor definiert,
- einen Monofluormethyl- oder Trifluormethylrest,
- einen Cyclopropyl- oder Cyclobutyl- oder Cyclopentylrest steht;
- eine Aminogruppe vom Typ II 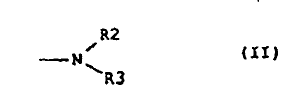 in welcher R₂ und R₃, die gleich oder verschieden sind, für Wasserstoff oder einen C₁-C₅-Alkylrest, wie zuvor definiert, oder einen Cyclopropyl- oder Cyclobutylrest oder einen Trifluormethylrest stehen;
- eine gesättigte cyclische Aminogruppe vom Typ III 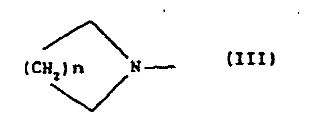 in welcher **n** die ganzen Zahlen 1 oder 2 annehmen kann;
- eine Alkoxycarbonylgruppe, vorzugsweise eine Methoxycarbonylgruppe (CH₃OCO-) oder eine Ethoxycarbonylgruppe (CH₃CH₂OCO-),
wie auch die Additionssalze der Verbindungen der allgemeinen Formel (I) mit anorganischen Säuren oder den organischen Säuren, die pharmazeutisch annehmbar sind.

2. Verbindungen der allgemeinen Formel (I) nach Anspruch 1, **dadurch gekennzeichnet, dass** sie ausgewählt werden unter:
(3,4-Dichlorphenyl)-(4-{[(6-pyrazol-1-ylpyridin-2-ylmethyl)-amino]methyl}piperidin-l-yl)methanon
(3-Chlor-4-fluorphenyl)-(4-{[(6-pyrazol-1-ylpyridin-2-ylmethyl)amino]methyl}piperidin-l-yl)methanon
(4-Chlor-3-methylphenyl)-(4-{[(6-pyrazol-1-ylpyridin-2-ylmethyl)amino}methyl}piperidin-1-yl)methanon
(3-Chlorphenyl)-(4-{[(6-pyrazol-1-ylpyridin-2-ylmethyl)amino]-methyl}piperidin-1-yl)methanon
(4-{[(6-Pyrazol-1-ylpyridin-2-ylmethyl)amino]methyl}piperidin-1-yl)-m-tolyl-methanon
(3,4-Dichlorphenyl)-4-fluor-4-{[(6-pyrazol-1-ylpyridin-2-ylmethyl)amino]methyl}piperidin-1-yl)methanon
(3,4-Dichlorphenyl)-(4-{[(6-imidazol-1-ylpyridin-2-ylmethyl)-amino]methyl}piperidin-1-yl)methanon
(3,4-Dichlorphenyl)-(4-{[(6-[1,2,4]-triazol-1-ylpyridin-2-ylmethyl)amino]methyl}piperidin-1-yl)methanon
(3,4-Dichlorphenyl)-(4-{[(6-pyrrol-1-ylpyridin-2-ylmethyl)-amino]methyl}piperidin-1-yl)methanon
(3,4-Dichlorphenyl)-(4-{[(6-methylaminopyridin-2-ylmethyl)-amino]methyl}piperidin-1-yl)methanon
(3,4-Dichlorphenyl)-(4-fluor-4-{[(6-methylaminopyridin-2-ylmethyl)amino]methyl}piperidin-1-yl)methanon
(3,4-Dichlorphenyl)-(4-{[(6-dimethylaminopyridin-2-ylmethyl)-amino]methyl}piperidin-1-yl)methanon
(3-Chlor-4-fluorphenyl)-(4-{[(6-dimethylaminopyridin-2-ylmethyl)amino]methyl}-4-fluorpiperidin-1-yl)methanon
(3,4-Dichlorphenyl)-(4-{[(6-dimethylaminopyridin-2-ylmethyl)amino]methyl}-4-fluorpiperidin-1-yl)methanon
(3,4-Dichlorphenyl)-[4-({[6-(ethylmethylamino)-pyridin-2-ylmethyl]amino}methyl)-4-fluorpiperidin-1-yl]methanon
(3,4-Dichlorphenyl)-[4-({[6-(methylpropylamino)-pyridin-2-ylmethyl]amino}methyl)piperidin-1-yl]methanon
(4-{[(6-Azetidin-1-ylpyridin-2-ylmethyl)amino]methyl}piperidin-1-yl)-(3,4-dichlorphenyl)methanon
(4-([(6-Azetidin-1-ylpyridin-2-ylmethyl)amino]methyl}-4-fluorpiperidin-1-yl)-(3,4-dichlorphenyl)methanon
(4-([(6-Pyrrolidin-1-ylpyridin-2-ylmethyl)amino]methyl}-piperidin-1-yl)-(3,4-dichlorphenyl)methanon
(4-{[(6-Chlorpyridin-2-ylmethyl)amino]methyl}piperidin-1-yl)-(3,4-dichlorphenyl)methanon
(3,4-Dichlorphenyl)-[4-({(6-(1*H*-pyrazol-3-yl)pyridin-2-ylmethyl]amino}methyl)piperidin-1-yl]methanon
(3,4-Dichlorphenyl)-[4-fluor-4-({[6-(1*H*-pyrazol-3-yl)pyridin-2-ylmethyl]amino}methyl)piperidin-1-yl]methanon
(3,4-Dichlorphenyl)-[4-fluor-4-({[6-(1-methylpyrazol-3-yl)-pyridin-2-ylmethyl]amino}methyl)piperidin-1-yl]methanon
(3,4-Dichlorphenyl)-[4-({[6- (1*H*-imidazol-2-yl)pyridin-2-ylmethyl]amino}methyl)piperidin-1-yl]methanon
(3,4-Dichlorphenyl)-(4-{[(6-thiazol-2-ylpyridin-2-ylmethyl)-amino]methyl}piperidin-1-yl)methanon
(3,4-Dichlorphenyl)-(4-fluor-4-{[(6-thiazol-2-ylpyridin-2-ylmethyl)amino]methyl}piperidin-1-yl)methanon
(3,4-Dichlorphenyl)-[4-({(6-(1*H*-pyrrol-2-yl)pyridin-2-ylmethyl]amino}methyl)piperidin-1-yl]methanon
(3,4-Dichlorphenyl)-(4-{[(6-thiophen-2-ylpyridin-2-ylmethyl)-amino]methyl}piperidin-1-yl)methanon
(3,4-Dichlorphenyl)-(4-fluor-4-{[(6-thiophen-2-ylpyridin-2-ylmethyl)amino]methyl}piperidin-1-yl)methanon
(3,4-Dichlorphenyl)-(4-{[(6-furan-2-ylpyridin-2-ylmethyl)-amino]methyl}piperidin-1-yl)methanon
(3,4-Dichlorphenyl)-(4-fluor-4-{[(6-furan-2-ylpyridin-2-ylmethyl)amino]methyl}piperidin-1-yl)methanon
(3-Chlor-4-fluorphenyl)-(4-fluor-4-{[(6-furan-2-ylpyridin-2-ylmethyl)amino]methyl}piperidin-1-yl)methanon
(3,4-Dichlorphenyl)-(4-{[(6-oxazol-5-ylpyridin-2-ylmethyl)-amino]methyl}piperidin-1-yl)methanon
(3,4-Dichlorphenyl)-(4-fluor-4-{[(6-oxazol-5-ylpyridin-2-ylmethyl)amino]methyl}piperidin-1-yl)methanon
(3,4-Dichlorphenyl)-(4-fluor-4-{[(6-furan-3-ylpyridin-2-ylmethyl)amino]methyl}piperidin-1-yl)methanon
(3,4-Dichlorphenyl)-[4-({[6-(5-methyl-[1,2,4]-oxadiazol-3-yl)pyridin-2-ylmethyl]amino}methyl)piperidin-1-yl]methanon
(3,4-Dichlorphenyl)-(4-{[(6-methylpyridin-2-ylmethyl)amino]-methyl}piperidin-1-yl)methanon
(3,4-Dichlorphenyl)-(4-{[(6-isopropylpyridin-2-ylmethyl)-amino]methyl}piperidin-1-yl)methanon
(3,4-Dichlorphenyl)-(4-{[(6-cyclopropylpyridin-2-ylmethyl)-amino]methyl}-4-fluorpiperidin-1-yl)methanon
(3,4-Dichlorphenyl)-(4-fluor-4-{[(6-fluormethylpyridin-2-ylmethyl)amino]methyl}piperidin-1-yl)methanon
(3,4-Dichlorphenyl)-(4-{[(6-difluormethylpyridin-2-ylmethyl)-amino]methyl}piperidin-1-yl)methanon
(3,4-Dichlorphenyl)-(4-{[(6-difluormethylpyridin-2-ylmethyl)-amino]methyl}-4-fluorpiperidin-1-yl)methanon
(3,4-Dichlorphenyl)-[4-fluor-4-({[6-(1-fluorethyl)pyridin-2-ylmethyl]amino}methyl)piperidin-1-yl]methanon
6-({[1-(3,4-Dichlorbenzoyl)piperidin-4-ylmethyl]amino}methyl)-pyridin-2-carbonsäuremethylester
6-({[1-(3,4-Dichlorbenzoyl)piperidin-4-ylmethyl]amino}methyl)-pyridin-2-carbonsäureethylester
(3,4-Dichlorphenyl)-(4-{[(6-methoxypyridin-2-ylmethyl)amino]-methyl)piperidin-1-yl)methanon
(3,4-Dichlorphenyl)-(4-fluor-4-{[(6-methoxypyridin-2-ylmethyl)amino]methyl}piperidin-1-yl)methanon
(3,4-Dichlorphenyl)-(4-fluor-4-{[(6-isopropyloxypyridin-2-ylmethyl)amino]methyl)piperidin-1-yl)methanon
(4-{[(6-Cyclopentyloxypyridin-2-ylmethyl)amino]methyl}piperidin-1-yl)-(3,4-dichlorphenyl)methanon
(3,4-Dichlorphenyl)-(4-{[(6-methylsulfanylpyridin-2-ylmethyl)-amino]methyl}piperidin-1-yl)methanon
(3,4-Dichlorphenyl)-(4-{[(6-fluorpyridin-2-ylmethyl)amino]-methyl}piperidin-1-yl)methanon
(3,4-Dichlorphenyl)-(4-fluor-4-{[(6-fluorpyridin-2-ylmethyl)-amino]methyl}piperidin-1-yl)methanon
(3,4-Dichlorphenyl)-(4-fluor-{[(3-fluorpyridin-2-ylmethyl)-amino]methyl}piperidin-1-yl)methanon
(4-{[(4-Chlorpyridin-2-ylmethyl)amino]methyl)piperidin-1-yl)-(3, 4-dichlorphenyl)methanon
(3-Chlor-4-fluorphenyl)-(4-fluor-4-{[(5-methyl-6-furan-2-ylpyridin-2-ylmethyl)amino]methyl}piperidin-1-yl)methanon
(3-Chlorphenyl)-[4-fluor-4-({[5-methyl-6-(1*H*-pyrazol-3-yl)-2-ylpyridin-2-ylmethyl]amino}methyl)piperidin-1-yl]methanon
(3-Chlor-4-fluorphenyl)-(4-fluor-4-{[(5-methyl-6-methylaminopyridin-2-ylmethyl)amino]methyl}piperidin-1-yl)methanon
(4-{[(6-Azetidin-1-ylpyridin-2-ylmethyl)amino]methyl}-4-fluorpiperidin-1-yl)-(3-chlor-4-fluorphenyl)methanon
(3-Chlor-4-fluorphenyl)-(4-fluor-4-{[(6-oxazol-5-ylpyridin-2-ylmethyl)amino]methyl}piperidin-1-yl)methanon
(3-Chlor-4-fluorphenyl)-(4-fluor-4-{[(6-ethylaminopyridin-2-ylmethyl)amino]methyl}piperidin-1-yl)methanon
(3-Chlor-4-fluorphenyl)-(4-fluor-4-{[(6-methylaminopyridin-2-ylmethyl)amino]methyl}piperidin-1-yl)methanon
(3-Chlor-4-methylphenyl)-(4-fluor-4-{[(6-dimethylaminopyridin-2-ylmethyl)amino]methyl}piperidin-1-yl)methanon
(3-Chlor-4-fluorphenyl)-(4-fluor-4-{[(5-methyl-6-dimethylaminopyridin-2-ylmethyl)amino]methyl}piperidin-1-yl)methanon
(3-Chlor-4-fluorphenyl)-[4-fluor-4-({[6-(1*H*-pyrazol-3-yl)pyridin-2-ylmethyl]amino}methyl)piperidin-1-yl]methanon
(3,4-Dichlorphenyl)-(4-fluor-4-{[(3-methyl-6-dimethylaminopyridin-2-ylmethyl)amino]methyl}piperidin-1-yl)methanon
(3-Chlor-4-fluorphenyl)-(4-fluor-4-{[(6-pyrazol-l-ylpyridin-2-ylmethyl)amino]methyl}piperidin-1-yl)methanon
(3,4-Dichlorphenyl)-(4-fluor-4-([(5-methylpyridin-2-ylmethyl)-amino]methyl}piperidin-l-yl)methanon
(3-Chlor-4-fluorphenyl)-(4-fluor-4-{[(5-methylpyridin-2-ylmethyl)amino]methyl)piperidin-1-yl)methanon
(3-Chlor-4-fluorphenyl)-(4-fluor-4-{[(6-diethylaminopyridin-2-ylmethyl)amino]methyl}piperidin-1-yl)methanon
(3-Chlor-4-fluormethyl)-(4-fluor-4-{[(5-methyl-6-chlorpyridin-2-ylmethyl)amino]methyl}piperidin-1-yl)methanon
(3-Chlor-4-fluorphenyl)-(4-fluor-4-{[(4-methyl-6-dimethylaminopyridin-2-ylmethyl)amino]methyl}piperidin-1-yl)methanon
(3-Chlor-4-fluorphenyl)-(4-fluor-4-{[(5-methyl-6-pyrazol-1-ylpyridin-2-ylmethyl)amino]methyl}piperidin-1-yl)methanon
(3-Chlorphenyl)-(4-fluor-4-{[(6-dimethylaminopyridin-2-ylmethyl)amino]methyl}piperidin-1-yl)methanon

3. Verfahren zur Herstellung der Verbindungen der allgemeinen Formel **(Ia)** nach den Ansprüchen 1 und 2, **dadurch gekennzeichnet, dass** man ein Aldehyd der allgemeinen Formel **(IV)** mit einem Amin der allgemeinen Formel (**V**) in reduzierendem Milieu umsetzt: 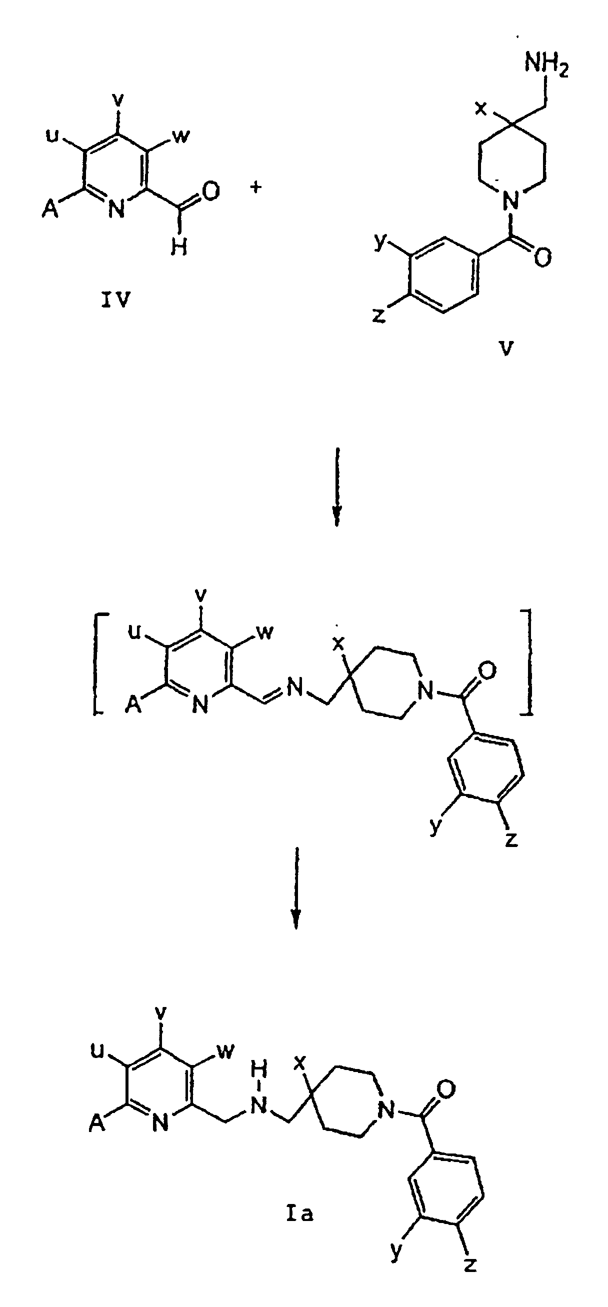 worin: **A, u, v, w, x, y** und **z** wie zuvor definiert sind.

4. Verfahren zur Herstellung der Verbindungen der allgemeinen Formel (**Ia**) nach den Ansprüchen 1 und 2, **dadurch gekennzeichnet, dass** man ein Aldehyd der allgemeinen Formel (**IV**) mit einem Derivat vom Azido-Typ mit der allgemeinen Formel (**VI**) in Gegenwart eines Aryl- oder Alkylphosphins in reduzierendem Milieu umsetzt: 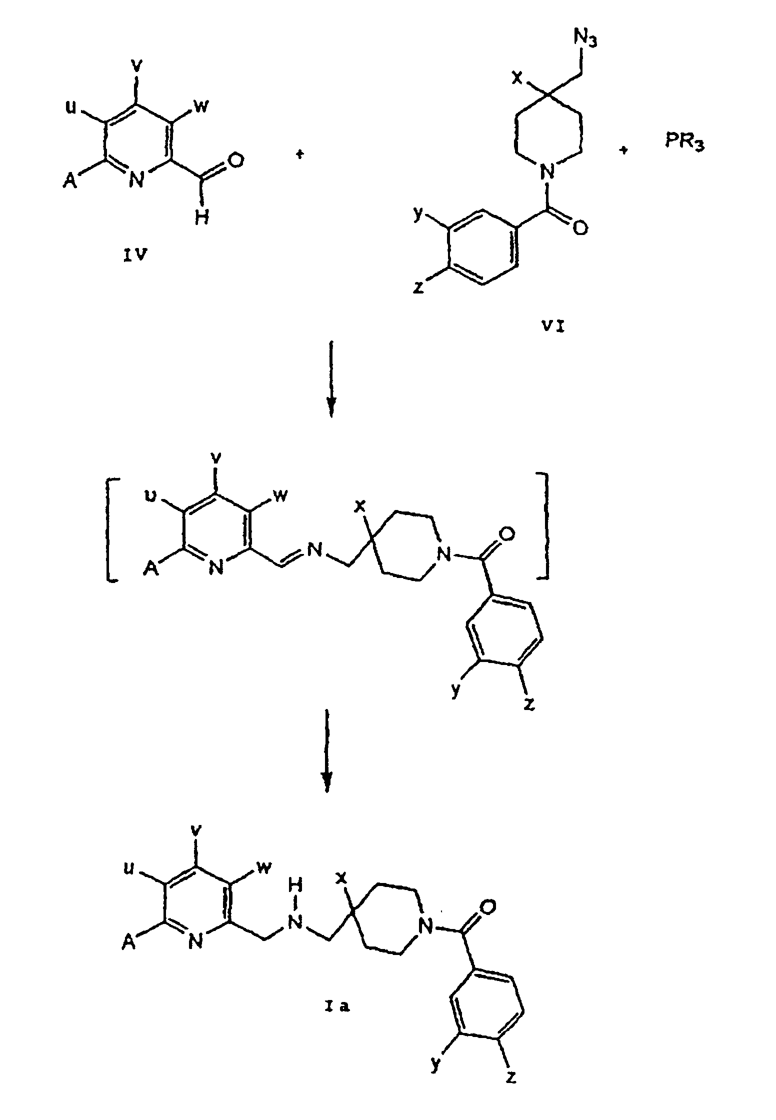 worin: **A, u, v, w, x, y** und **z** wie zuvor definiert sind.

5. Verfahren zur Herstellung von Verbindung (**Ib**), einem besonderen Fall der Verbindungen der Formel (**Ia**), worin **x** ein Wasserstoffatom ist, nach den Ansprüchen 1 und 2, **dadurch gekennzeichnet, dass** man ein Aldehyd der Formel (**IV**) nacheinander mit Piperidin-4-ylmethylamin, einem Säurechlorid, dann einem Reduktionsmittel gemäß einer "Eintopf"-Technik umsetzt: 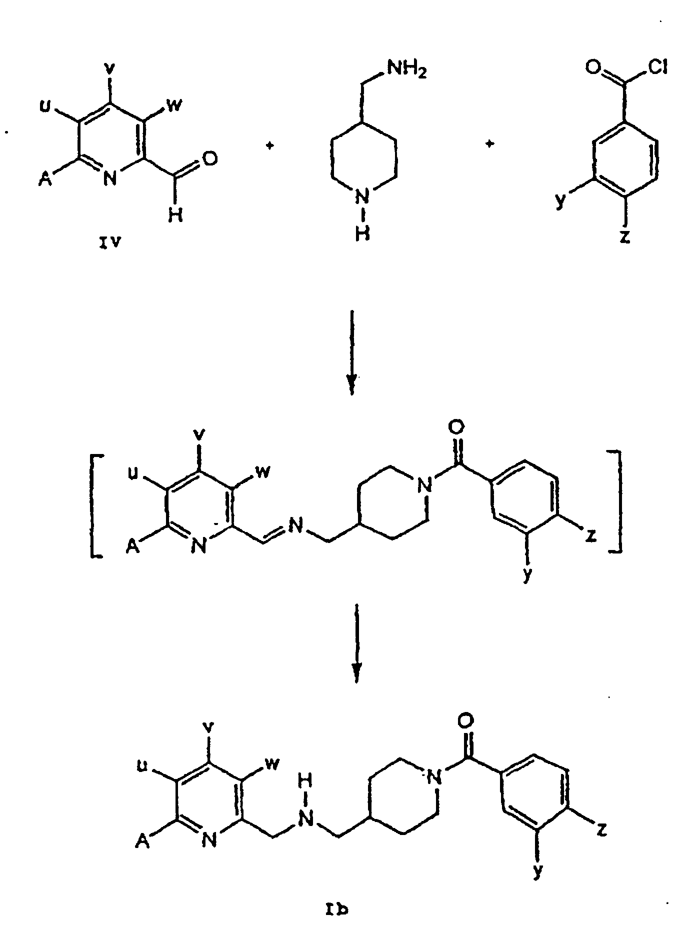 worin: **A, u, v, w, x, y** und **z** wie zuvor definiert sind.

6. Verfahren zur Herstellung von Verbindung (**Ic**), einem besonderen Fall der Verbindungen der Formel (**Ia**), nach den Ansprüchen 1 und 2, **dadurch gekennzeichnet, dass** man ein Reagens vom Typ (HA) oder (A⁻Na⁺) auf eine Verbindung der Formel (**Ia**: A = F oder Cl) einwirken lässt: 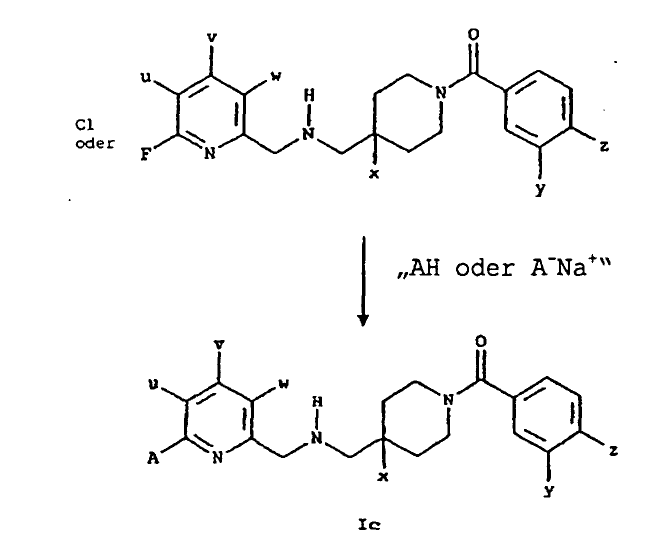 worin:
- **u, v, w, x, y** und **z** wie zuvor definiert sind.
- **A** unter einer Aminogruppe vom Typ II oder einer gesättigten cyclischen Aminogruppe vom Typ III oder einem Pyrrol-1-yl-, Pyrazol-1-yl-, Imidazol-1-yl- oder [1,2,4]-Triazol-1-ylrest ausgewählt wird.

7. Synthesezwischenprodukte der Formel (**V**), die für die Herstellung der Verbindungen der allgemeinen Formel (**I**) eingesetzt werden: 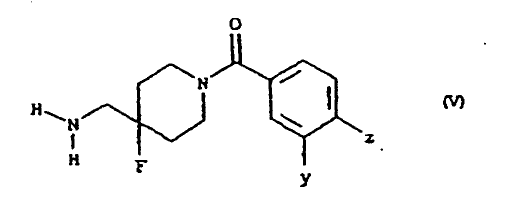 worin **y** und **z** wie in Anspruch 1 definiert sind.

8. Synthesezwischenprodukte der Formel (**VI**), die für die Herstellung der Verbindungen der allgemeinen Formel (**I**) eingesetzt werden: 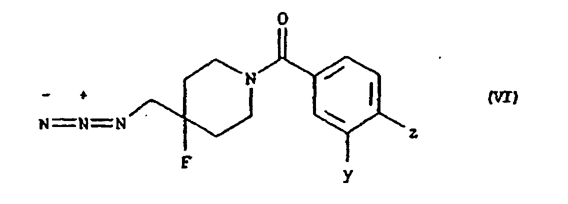 worin **y** und **z** wie in Anspruch 1 definiert sind.

9. Derivate nach Anspruch 1 oder 2 als Arzneimittel.

10. Arzneimittel nach Anspruch 9 für die Behandlung von Depressionen.

11. Arzneimittel nach Anspruch 9 für die Behandlung von Angst.

12. Arzneimittel nach Anspruch 9 für die Behandlung von obsessiv-kompulsiven Störungen oder Leiden.

13. Arzneimittel nach Anspruch 9 für die Behandlung von Panikattacken.

14. Arzneimittel nach Anspruch 9 für die Behandlung von Schmerzwahrnehmungen.

15. Arzneimittel nach Anspruch 9 für die Behandlung von Aggressivität, Alkoholmissbrauch, Schlafstörungen.

16. Arzneimittel nach Anspruch 9 für die Behandlung von Gefäßerkrankungen oder -störungen, insbesondere zerebrovaskulären Erkrankungen oder Störungen, Migräne und Emesis.

17. Pharmazeutische Zusammensetzung, **dadurch gekennzeichnet, dass** sie als Wirkstoff wenigstens eine Verbindung gemäß einem der Ansprüche 1 und 2 oder eines von deren Additionssalzen mit einer pharmazeutisch annehmbaren Säure in Kombination mit einer bzw. einem oder mehreren Trägersubstanzen oder Vehikeln, die pharmazeutisch annehmbar sind, enthält.

## Claims

1. Pyridin-2-yl-methylamine derivatives of formula (I): 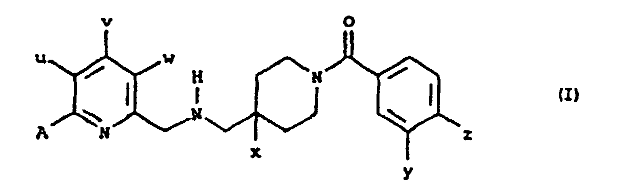 in which:
**u** represents a hydrogen atom or a methyl radical with the proviso that when **u** is a methyl radical then **v** and **w** represent a hydrogen atom;
**v** represents a hydrogen atom or a chlorine atom or a methyl radical with the proviso that when **v** represents a chlorine atom or a methyl radical then **u** and **w** represent a hydrogen atom;
**w** represents a hydrogen atom or a fluorine atom or a methyl radical with the proviso that when **w** represents a fluorine atom or a methyl radical then **u** and **v** represent a hydrogen atom;
**x** represents a hydrogen atom or a fluorine atom;
**y** represents a chlorine atom or a methyl radical;
**z** represents a hydrogen atom or a fluorine atom or a chlorine atom or a methyl radical;
**A** represents:
- a hydrogen atom or a fluorine atom or a chlorine atom;
- a C₁-C₅ alkyl radical, i.e. a straight or branched chain saturated aliphatic hydrocarbon radical containing 1 to 5 carbon atoms such as methyl, ethyl, propyl, butyl, pentyl, isopropyl, 1-methyl-ethyl, 1-methyl-propyl, 1-methyl-butyl, 2-methyl-propyl, 2-methyl-butyl or 3-methyl-butyl, 1-ethyl-propyl, 2-ethyl-propyl;
- a fluoroalkyl radical such as monofluoromethyl (-CH₂F) or difluoromethyl (-CHF₂) or trifluoromethyl (-CF₃) or 1-fluoro-1-ethyl (-CHFCH₃) or 1,1-difluoro-1-ethyl (-CF₂CH₃);
- a cyclopropyl or cyclobutyl or cyclopentyl radical;
- a 5-membered aromatic heterocyclic group chosen from:
furan-2-yl, (O.CH:CH.CH:C-),
furan-3-yl, (CH:CH.O.CH:C-),
1H-pyrrol-2-yl, (NH.CH:CH.CH:C-),
1H-pyrrol-3-yl, (CH:CH.NH.CH:C-),
1-methyl-pyrrol-2-yl, (N(CH₃).CH:CH.CH:C-),
1-methyl-pyrrol-3-yl, (CH:CH.N(CH₃).CH:C-),
thiophen-2-yl, (S.CH:CH.CH:C-),
thiophen-3-yl, (CH:CH.S.CH:C-),
pyrazol-1-yl, (N:CH.CH:CH.N-),
1H-pyrazol-3-yl, (CH:CH.NH.N:C-),
1H-pyrazol-4-yl, (CH:N.NH.CH:C-),
1-methyl-pyrazol-3-yl, (CH:CH.N(CH₃).N:C-),
imidazol-1-yl, (CH:N.CH:CH.N-),
1H-imidazol-2-yl, (NH.CH:CH.N:C-),
1H-imidazol-4-yl, (N:CH.NH.CH:C-),
oxazol-2-yl, (O.CH:CH.N:C-),
oxazol-4-yl, (N:CH.O.CH:C-),
oxazol-5-yl, (O.CH:N.CH:C-),
isoxazol-5-yl, (O.N:CH.CH:C-),
isoxazol-4-yl, (CH:N.O.CH:C-),
isoxazol-3-yl, (CH:CH.O.N:C-),
thiazol-2-yl, (S.CH:CH.N:C-,
thiazol-4-yl, (N:CH.S.CH:C-),
thiazol-5-yl, (S.CH:N.CH:C-),
isothiazol-5-yl, (S.N:CH.CH:C-),
isothiazol-4-yl, (CH:N.S.CH:C-),
isothiazol-3-yl, (CH:CH.S.N:C-),
[1,2,4]triazol-1-yl, (CH:N.CH:N.N-),
1H-[1,2,4]triazol-3-yl, (N:CH.NH.N:C-),
[1,2,4]oxadiazol-3-yl, (N:CH.O.N:C-),
[1,2,4]oxadiazol-5-yl, (O.N:CH.N:C-),
5-methyl-[1,2,4]oxadiazol-3-yl, (N:C(CH₃).O.N:C-) and
1H-tetrazol-5-yl, (NH.N:N.N:C-);
- an alkoxy (R₁O-) or alkylthio (R₁S-) group in which the R₁ radical represents:
• a C₁-C₅ alkyl radical as defined above,
• a monofluoromethyl or trifluoromethyl radical,
• a cyclopropyl or cyclobutyl or cyclopentyl radical;
- a type II amino group 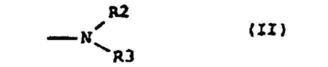 in which R₂ and R₃, which are identical or different, represent hydrogen, or a C₁-C₅ alkyl radical as defined above or a cyclopropyl or cyclobutyl radical or a trifluoromethyl radical;
- a type III saturated cyclic amino group 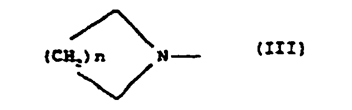 in which **n** may take the integers 1 or 2;
- an alkoxycarbonyl group, preferably a methoxycarbonyl group (CH₃OCO-) or an ethoxycarbonyl group (CH₃CH₂OCO-);
as well as the addition salts of the compounds of general formula (I) with pharmaceutically acceptable inorganic acids or organic acids.

2. Compounds of general formula (I), according to Claim 1, **characterized in that** they are selected from:
(3,4-Dichlorophenyl)-(4-{[(6-pyrazol-l-yl-pyridin-2-ylmethyl)amino]methyl}piperidin-l-yl)methanone
(3-Chloro-4-fluorophenyl)-(4-{[(6-pyrazol-l-yl-pyridin-2-ylmethyl)amino]methyl}piperidin-1-yl)methanone
(4-Chloro-3-methylphenyl)-(4-{[(6-pyrazol-l-yl-pyridin-2-ylmethyl)amino]methyl}piperidin-1-yl)methanone
(3-Chlorophenyl)-(4-{[(6-pyrazol-l-yl-pyridin-2-ylmethyl)amino]methyl}piperidin-1-yl)methanone
(4-{[(6-Pyrazol-l-yl-pyridin-2-ylmethyl)amino]methyl}-piperidin-1-yl)-*m*-tolylmethanone
(3,4-Dichlorophenyl)-(4-fluoro-4-{[(6-pyrazol-l-yl-pyridin-2-ylmethyl)amino]methyl}piperidin-1-yl)methanone
(3,4-Dichlorophenyl)-(4-{[(6-imidazol-l-yl-pyridin-2-ylmethyl)amino]methyl}piperidin-1-yl)methanone
(3,4-Dichlorophenyl)-(4-{[(6-[1,2,4]triazol-1-yl-pyridin-2-ylmethyl)amino]methyl}piperidin-1-yl)methanone
(3,4-Dichlorophenyl)-(4-{[(6-pyrrol-l-yl-pyridin-2-ylmethyl)amino]methyl)piperidin-1-yl)methanone
(3,4-Dichlorophenyl)-(4-{[(6-methylaminopyridin-2-ylmethyl)amino]methyl}piperidin-1-yl)methanone
(3,4-Dichlorophenyl)-(4-fluoro-4-{[(6-methylaminopyridin-2-ylmethyl)amino]methyl}piperidin-1-yl)methanone
(3,4-Dichlorophenyl)-(4-{[(6-dimethylaminopyridin-2-ylmethyl)amino]methyl}piperidin-1-yl)methanone
(3-Chloro-4-fluorophenyl)-(4-{[(6-dimethylaminopyridin-2-ylmethyl)amino]methyl}-4-fluoropiperidin-1-yl)methanone
(3,4-Dichlorophenyl)-(4-{[(6-dimethylaminopyridin-2-ylmethyl)amino]methyl)-4-fluoropiperidin-l-yl)methanone
(3,4-Dichlorophenyl)-[4-({[6-ethylmethylamino)pyridin-2-ylmethyl]amino}methyl)-4-fluoropiperidin-l-yl)methanone
(3,4-Dichlorophenyl)-[4-({[6-(methylpropylamino)-pyridin-2-ylmethyl]amino}methyl)piperidin-1-yl)-methanone
(4-{[(6-Azetidin-1-yl-pyridin-2-ylmethyl)amino]-methyl}piperidin-1-yl)-(3,4-dichlorophenyl)methanone
(4-{[(6-Azetidin-1-yl-pyridin-2-ylmethyl)amino]methyl}-4-fluoropiperidin-1-yl)-(3,4-dichlorophenyl)methanone
(4-{[(6-Pyrrolidin-1-ylpyridin-2-ylmethyl)amino]-methyl)piperidin-1-yl)-(3,4-dichlorophenyl)methanone
(4-{[(6-Chloropyridin-2-ylmethyl)amino]methyl}-piperidin-1-yl)-(3,4-dichlorophenyl)methanone
(3,4-Dichlorophenyl)-[4-({[6-(1*H*-pyrazol-3-yl)pyridin-2-ylmethyl]amino}methyl)piperidin-1-yl]methanone
(3,4-Dichlorophenyl)-[4-[fluoro-4-({[6-(1*H*-pyrazol-3-yl)pyridin-2-ylmethyl]amino}methyl)piperidin-1-yl]methanone
(3,4-Dichlorophenyl)-[4-fluoro-4-({[6-(1-methylpyrazol-3-yl)pyridin-2-ylmethyl]amino}methyl)piperidin-1-yl]methanone
(3,4-Dichlorophenyl)-[4-({[6-(1*H*-imidazol-2-yl)pyridin-2-ylmethyl]amino}methyl)piperidin-1-yl]methanone
(3,4-Dichlorophenyl)-(4-{[(6-thiazol-2-ylpyridin-2-ylmethyl)amino]methyl)piperidin-1-yl)methanone
(3,4-Dichlorophenyl)-(4-fluoro-4-{[(6-thiazol-2-yl-pyridin-2-ylmethyl)amino]methyl)piperidin-1-yl)methanone
(3,4-Dichlorophenyl)-[4-({[6-(l*H*-pyrrol-2-yl)pyridin-2-ylmethyl]amino)methyl)piperidin-1-yl]methanone
(3,4-Dichlorophenyl)-(4-{[(6-thiophen-2-yl-pyridin-2-ylmethyl)amino]methyl}piperidin-1-yl)methanone
(3,4-Dichlorophenyl)-(4-fluoro-4-{[(6-thiophen-2-yl-pyridin-2-ylmethyl)amino]methyl)piperidin-1-yl]methanone
(3,4-Dichlorophenyl)-(4-{[(6-furan-2-yl-pyridin-2-ylmethyl)amino]methyl}piperidin-1-yl)methanone
(3,4-Dichlorophenyl)-(4-fluoro-4-{[(6-furan-2-yl-pyridin-2-ylmethyl)amino]methyl}piperidin-1-yl)methanone
(3-Chloro-4-fluorophenyl)-(4-fluoro-4-{[(6-furan-2-yl-pyridin-2-ylmethyl)amino]methyl}piperidin-1-yl]methanone
(3,4-Dichlorophenyl)-(4-{[(6-oxazol-5-yl-pyridin-2-ylmethyl)amino]methyl}piperidin-1-yl)methanone
(3,4-Dichlorophenyl)-(4-fluoro-4-{[(6-oxazol-5-yl-pyridin-2-ylmethyl)amino]methyl}piperidin-1-yl)methanone
(3,4-Dichlorophenyl)-(4-fluoro-4-{[(6-furan-3-yl-pyridin-2-ylmethyl)amino]methyl}piperidin-1-yl]methanone
(3,4-Dichlorophenyl)-[4-({[6-(5-methyl-[1,2,4]oxadiazol-3-yl)pyridin-2-ylmethyl]amino}methyl)-piperidin-1-yl)methanone
(3,4-Dichlorophenyl)-(4-{[(6-methylpyridin-2-ylmethyl)-amino]methyl}piperidin-1-yl)methanone
(3,4-Dichlorophenyl)-(4-{[(6-isopropylpyridin-2-ylmethyl)amino]methyl}piperidin-1-yl)methanone
(3,4-Dichlorophenyl)-(4-{[(6-cyclopropylpyridin-2-ylmethyl)amino]methyl}-4-fluoropiperidin-1-yl)methanone
(3,4-Dichlorophenyl)-(4-fluoro-4-{[(6-fluoromethyl-pyridin-2-ylmethyl)amino]methyl}piperidin-1-yl)-methanone
(3,4-Dichlorophenyl)-(4-{[(6-difluoromethylpyridin-2-ylmethyl)amino]methyl}piperidin-1-yl]methanone
(3,4-Dichlorophenyl)-(4-{[(6-difluoromethylpyridin-2-ylmethyl)amino]methyl}-4-fluoropiperidin-1-yl)methanone
(3,4-Dichlorophenyl)-[4-fluoro-4-({[6-(1-fluoroethyl)pyridin-2-ylmethyl]amino}methyl)piperidin-1-yl)methanone
6-({[1-(3,4-Dichlorobenzoyl)piperidin-4-ylmethyl]-amino}methyl)pyridine-2-carboxylic acid methyl ester
6-({[1-(3,4-Dichlorobenzoyl)piperidin-4-ylmethyl}amino}methyl)pyridine-2-carboxylic acid ethyl ester
(3,4-Dichlorophenyl)-(4-{[(6-methoxypyridin-2-ylmethyl}amino]methyl}piperidin-1-yl)methanone
(3,4-Dichlorophenyl)-(4-fluoro-4-{[(6-methoxypyridin-2-ylmethyl)amino]methyl}piperidin-1-yl)methanone
(3,4-Dichlorophenyl)-(4-fluoro-4{[(6-isopropyloxypyridin-2-ylmethyl)amino)methyl}piperidin-1-yl)methanone
(4-{[(6-Cyclopentyloxypyridin-2-ylmethyl)amino]methyl}-piperidin-l-yl)-(3,4-dichlorophenyl)methanone
(3,4-Dichlorophenyl)-(4-{[(6-methylsulfanylpyridin-2-ylmethyl)amino]methyl}piperidin-1-yl)methanone
(3,4-Dichlorophenyl)-(4-{[(6-fluoropyridin-2-ylmethyl)amino]methyl}piperidin-1-yl)methanone
(3,4-Dichlorophenyl)-(4-fluoro-4-{[(6-fluoropyridin-2-ylmethyl)amino]methyl}piperidin-1-yl)methanone
(3,4-Dichlorophenyl)-(4-fluoro-{[(3-fluoropyridin-2-ylmethyl)amino]methyl}piperidin-1-yl)methanone
(4-{[(4-Chloropyridin-2-ylmethyl)amino]methyl}-piperidin-1-yl)-(3,4-dichlorophenyl)methanone
(3-Chloro-4-fluorophenyl)-(4-fluoro-4-{[(5-methyl-6-furan-2-yl-pyridin-2-ylmethyl)amino]methyl}piperidin-1-yl)methanone
(3-Chlorophenyl)-[4-fluoro-4-({[5-methyl-6-(1*H*-pyrazol-3-yl)-2-yl-pyridin-2-ylmethyl]amino}methyl)piperidin-1-yl)methanone
(3-Chloro-4-fluorophenyl)-(4-fluoro-4-{[(5-methyl-6-methylaminopyridin-2-ylmethyl)amino]methyl}piperidin-1-yl)methanone
(4-{[(6-Azetidin-1-ylpyridin-2-ylmethyl)amino]methyl}-4-fluoropiperidin-1-yl)-(3-chloro-4-fluorophenyl)-methanone
(3-Chloro-4-fluorophenyl)-(4-fluoro-4-{[(6-oxazol-5-yl-pyridin-2-ylmethyl)amino]methyl}piperidin-1-yl)methanone
(3-Chloro-4-fluorophenyl)-(4-fluoro-4-([(6-ethylaminopyridin-2-ylmethyl)amino]methyl}piperidin-1-yl)methanone
(3-Chloro-4-fluorophenyl)-(4-fluoro-4-{[(6-methylaminopyridin-2-ylmethyl)amino]methyl)piperidin-1-yl)methanone
(3-Chloro-4-methylphenyl)-(4-fluoro-4-{[(6-dimethylaminopyridin-2-ylmethyl)amino]methyl}piperidin-1-yl)methanone
(3-Chloro-4-fluorophenyl)-(4-fluoro-4-{[(5-methyl-6-dimethylaminopyridin-2-ylmethyl)amino]methyl}piperidin-1-yl)methanone
(3-Chloro-4-fluorophenyl)-[4-fluoro-4-({[6-(1*H*-pyrazol-3-yl)pyridin-2-ylmethyl]amino}methyl)piperidin-1-yl]methanone
(3,4-Dichlorophenyl)-(4-fluoro-4-{[(3-methyl-6-dimethylaminopyridin-2-ylmethyl)amino]methyl}piperidin-1-yl)methanone
(3-Chloro-4-fluorophenyl)-(4-fluoro-4-{[(6-pyrazol-1-yl-pyridin-2-ylmethyl)amino]methyl}piperidin-1-yl)methanone
(3,4-Dichlorophenyl)-(4-fluoro-4-{[(5-methylpyridin-2-ylmethyl)amino}methyl}piperidin-1-yl)methanone
(3-Chloro-4-fluorophenyl)-(4-fluoro-4-{[(5-methylpyridin-2-ylmethyl) amino]methyl}piperidin-1-yl)methanone
(3-Chloro-4-fluorophenyl)-(4-fluoro-4-{[(6-diethylaminopyridin-2-ylmethyl)amino]methyl)piperidin-1-yl)methanone
(3-Chloro-4-fluoromethyl)-(4-fluoro-4-{[(5-methyl-6-chloropyridin-2-ylmethyl)amino]methyl}piperidin-1-yl)methanone
(3-Chloro-4-fluorophenyl)-(4-fluoro-4-{[(4-methyl-6-dimethylaminopyridin-2-ylmethyl)amino]methyl}piperidin-1-yl)methanone
(3-Chloro-4-fluorophenyl)-(4-fluoro-4-{[(5-methyl-6-pyrazol-1-yl-pyridin-2-ylmethyl)amino]methyl}piperidin-1-yl)methanone
(3-Chlorophenyl)(4-fluoro-4-{[(6-dimethylaminopyridin-2-ylmethyl)amino]methyl}piperidin-1-yl)methanone

3. Process for preparing the compounds of general formula (Ia) according to Claims 1 and 2, **characterized in that** an aldehyde of general formula (IV) is reacted with an amine of general formula (V) in a reducing medium: 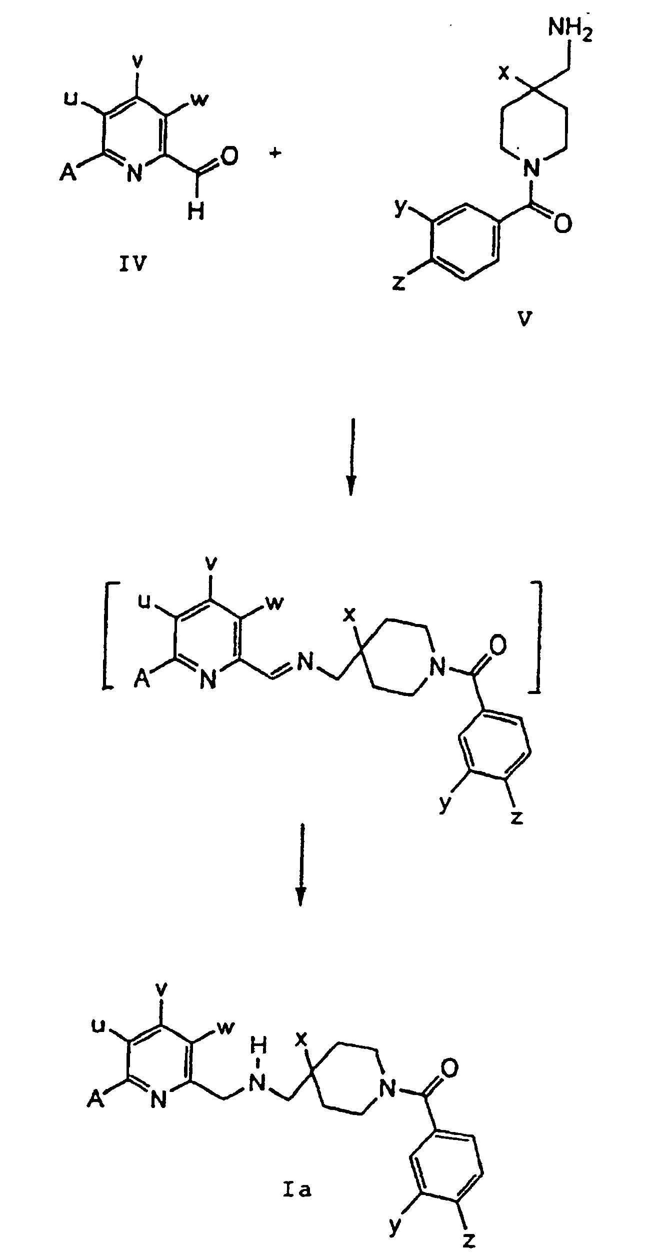 where: **A, u, v, w, x, y and z** are as defined above.

4. Process for preparing the compounds of general formula (Ia) according to Claims 1 and 2, **characterized in that** an aldehyde of general formula (IV) is reacted with an azido-type derivative of general formula (VI) in the presence of an aryl or alkylphosphine in a reducing medium: 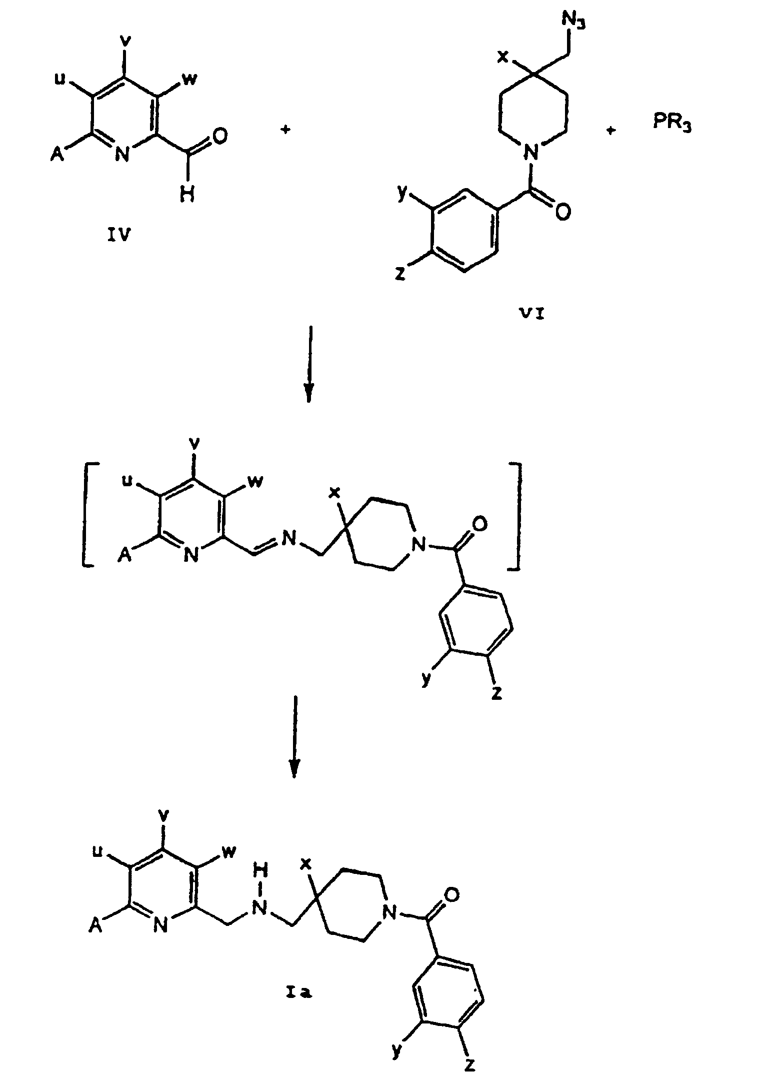 where: **A, u, v, w, x, y and z** are as defined above.

5. Process for preparing compound (Ib), specific case of the compounds of formula (Ia) where **x** is a hydrogen atom, according to Claims 1 and 2, **characterized in that** an aldehyde of formula (IV) is reacted successively with piperidin-4-yl-methylamine, an acid chloride and then a reducing agent according to a "one-pot" technique: 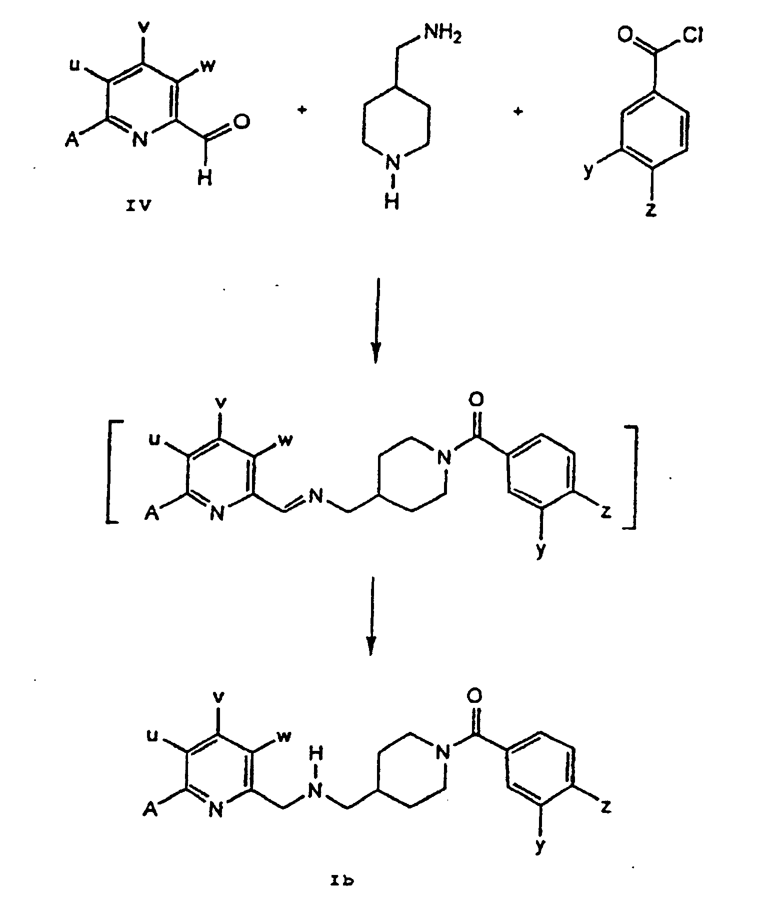 where: **A, u, v, w, x, y and z** are as defined above.

6. Process for preparing compound (Ic), specific case of the compounds of formula (Ia), according to Claims 1 and 2, **characterized in that** a reagent of the (HA) or (A⁻Na⁺) type is reacted with a compound of formula (Ia: A = F or Cl): 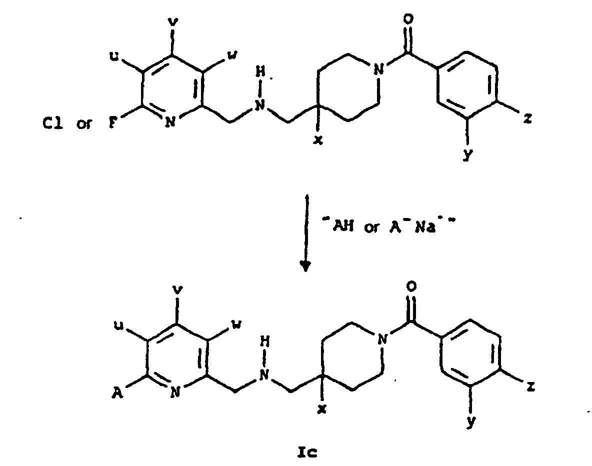 where:
• **u, v, w, x, y and z** are as defined above,
• **A** is selected from a type II amino group or a type III saturated cyclic amino group or a pyrrol-1-yl, pyrazol-1-yl, imidazol-1-yl or [1,2,4]triazol-1-yl radical.

7. Synthetic intermediates of formula (V) used for the preparation of the compounds of general formula (I): 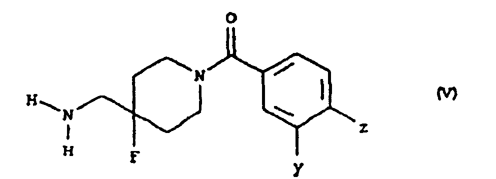 where **y and z** are as in Claim 1.

8. Synthetic intermediates of formula (VI) used for the preparation of the compounds of general formula (I): 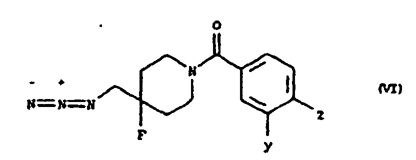 where **y and z** are as defined in Claim 1.

9. Derivatives according to Claim 1 or 2, as a medicine.

10. Medicine according to Claim 9, for the treatment of depression.

11. Medicine according to Claim 9, for the treatment of anxiety.

12. Medicine according to Claim 9, for the treatment of compulsive-obsessive disorders.

13. Medicine according to Claim 9, for the treatment of panic attacks.

14. Medicine according to Claim 9, for the treatment of perception of pain.

15. Medicine according to Claim 9, for the treatment of aggression, alcohol abuse, sleeping disorders.

16. Medicine according to Claim 9, for the treatment of vascular disorders, in particular cerebrovascular disorders, migraine and vomiting.

17. Pharmaceutical composition, **characterized in that** it contains, as active ingredient, at least one compound according to either of Claims 1 and 2, or one of its addition salts with a pharmaceutically acceptable acid, in combination with one or more pharmaceutically acceptable excipients or vehicles.
